# FASCICULE DE BREVET EUROPEEN

(11) **EP 2 262 804 B1**
(45) Date de publication et mention de la délivrance du brevet: **03.12.2014**
(21) Numéro de dépôt: 09754042.1
(22) Date de dépôt: 20.03.2009
(51) Int. Cl.: C07D 471/04, C07D 213/28, C07D 233/56, A61K 31/437, A61P 25/00, A61P 19/00, A61P 35/00

(54) **DÉRIVÉS POLYSUBSTITUES DE 6-HETEROARYLE-IMIDAZO[1,2-A]PYRIDINES, LEUR PRÉPARATION ET LEUR APPLICATION EN THÉRAPEUTIQUE**
POLYSUBSTITUIERTE 6-HETEROARYL-IMIDAZO-[L,2-A-]PYRIDIN-DERIVATE SOWIE DEREN HERSTELLUNG UND THERAPEUTISCHE ANWENDUNG
POLYSUBSTITUTED DERIVATIVES OF 6-HETEROARYL-IMIDAZO[L,2- A] PYRIDINES, AND PREPARATION AND THERAPEUTIC USE THEREOF

(30) Priorité: 21.03.2008 FR 0801585
(43) Date de publication de la demande: 22.12.2010
(73) Titulaire: SANOFI, 75008 Paris (FR)
(72) Inventeur: DE PERETTI, Danielle, F-75013 Paris (FR); EVANNO, Yannick, F-75013 Paris (FR); MACHNIK, David, F-75013 Paris (FR); RAKOTOARISOA, Nathalie, F-75013 Paris (FR)
(74) Mandataire: Le Coupanec, Pascale A.M.P.
(86) Numéro de dépôt international: PCT/FR2009/000298
(87) Numéro de publication internationale: WO 2009/144392

(56) Documents cités:
- WO-A-2008/034974
- FR-A- 2 903 105
- FR-A- 2 903 107
- DIMAURO E F ET AL: "Microwave-Assisted Preparation of Fused Bicyclic Heteroaryl Boronates: Application in One-Pot Suzuki Couplings" JOURNAL OF ORGANIC CHEMISTRY, AMERICAN CHEMICAL SOCIETY, EASTON.; US, vol. 71, no. 10, 1 janvier 2006 (2006-01-01), pages 3959-3962, XP002475376 ISSN: 0022-3263

## Description

La présente invention se rapporte à des dérivés polysubstitués de 6-hétéroaryl-imidazo[1,2-a]pyridine, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

Les demandes WO2008/034974, FR2903105 et FR2903107 de la société déposante décrivent respectivement des dérivés de 2-aryl-6-phényl-imidazo[1,2-α]pyridines, des dérivés de 2-benzoyl-imidazo[1,2-α]pyridines et des dérivés d'imidazo[1,2-α]pyridine-2-carboxamides, utiles pour la prévention ou le traitement de maladies impliquant les récepteurs nucléaires Nurr-1.

L'article Dimauro et al. (J. Org. Chem., 2006, 71(10), 3959-62) décrit la préparation de boronates d'hétéroaryles bicycliques fusionnés ainsi que des intermédiaires de synthèse tel que notamment le 2-phénylimidazol[1,2-α]pyridine 6-boronate.

La présente invention décrit les composés de formule (I) : 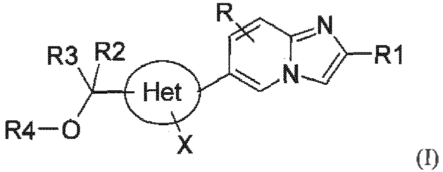 dans laquelle :
R₁ représente :
   un groupe phényle ou naphthyle, un groupe hétéroaryle ou un groupe hétérocyclique, ces groupes pouvant éventuellement être substitués par un ou plusieurs atomes ou groupes choisis indépendamment les uns des autres parmi les atomes ou groupes suivants : halogène, (C₁-C₁₀)alkyle, halo(C₁-C₁₀)alkyle, (C₁-C₁₀)alcoxy, halo(C₁-C₁₀)alcoxy, (C₁-C₁₀)thioalkyle, - S(O)(C₁-C₁₀)alkyle, -S(O)₂(C₁-C₁₀-*a*lkyle), hydroxyle, oxo, cyano, nitro, hydroxy(C₁-C₁₀)alkylène, NRaRb(C₁-C₁₀)alkylène, (C₁-C₁₀)alcoxy(C₁-C₁₀)alkylène-oxy, NRaRb, CONRaRb, SO₂NRaRb NRcCORd, OC(O)NRaRb, OCO(C₁-C₁₀)alkyle, NRcC(O)ORe, NRcSO₂Re, aryl(C₁-C₁₀)alkylène, aryle ou hétéroaryle monocyclique, l'aryle ou l'hétéroaryle monocyclique étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, oxo, nitro, cyano ou OCO(C₁-C₁₀)alkyle et R1 est lié à l'imidazo[1,2-a]pyridine par un carbone aromatique ;
Het représente un groupe hétéroaryle monocyclique comportant de 5 à 6 atomes dont de 1 à 3 hétéroatomes choisis parmi N, O et S ;
X représente de 1 à 3 substituants identiques ou différents l'un de l'autre choisis parmi un hydrogène, un halogène, (C₁-C₁₀)alkyle, (C₁-C₁₀)alcoxy, NRaRb, nitro, cyano, le (C₁-C₁₀)alkyle pouvant être éventuellement substitué par un ou plusieurs groupes choisis parmi un halogène, (C₁-C₁₀)alcoxy, (C₁-C₁₀)haloalcoxy, NRaRb ou hydroxyle ;
R représente en position 3, 5, 7 ou 8 de l'imidazo[1,2-*a*]pyridine de 1 à 4 substituants identiques ou différents l'un de l'autre choisi parmi un hydrogène, un halogène, (C₁-C₁₀)alkyle, halo(C₁-C₁₀)alkyle, (C₁-C₁₀)alcoxy ;
R₂ et R₃ représentent, indépendamment l'un de l'autre,
   un atome d'hydrogène,
   un groupe (C₁-C₁₀)alkyle, éventuellement substitué par un groupe Rf ;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy,
   halo(C₁-C₁₀)alcoxy, NRaRb, hydroxyle, nitro ou cyano ;
R₄ représente :
   un atome d'hydrogène,
   un groupe (C₁-C₁₀)alkyle, éventuellement substitué par up groupe Rf ;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano, (C₁-C₁₀)alkyl(CO)-, CONRaRb, NRcCORd, OC(O)NRaRb, OCO(C₁-C₁₀)alkyle, NRcC(O)ORe ou aryle, l'aryle étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
Ra et Rb représentent, indépendamment l'un de l'autre, un atome d'hydrogène ou un groupe (C₁-C₁₀)alkyle, aryl(C₁-C₁₀)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₁₀)alkyle, aryle ou aryl(C₁-C₁₀)alkylène ;
Rc et Rd représentent, indépendamment l'un de l'autre, un atome d'hydrogène ou un groupe (C₁-C₁₀)alkyle, aryl(C₁-C₁₀)alkylène, aryle ;
ou Rc et Rd forment ensemble un groupe (C₂-C₅)alkylène ;
Re représente un groupe (C₁-C₁₀)alkyle, aryl(C₁-C₁₀)alkylène, aryle ;
ou Rc et Re forment ensemble un groupe (C₂-C₅)alkylène ;
Rf représente un atome d'halogène, un groupe (C₁-C₁₀)alcoxy, halo(C₁-C₁₀)alcoxy, hydroxyle, cyano, NRaRb, C(O)NRaRb, NRcCORd, OC(O)NRaRb, OCO(C₁-C₁₀)alkyle, NRcCOORe, SO₂NRaRb NRcSO₂Re, aryl(C₁-C₁₀)alkylène, aryle, l'aryle étant éventuellement substitué par un ou plusieurs substituants choisi parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano ou OCO(C₁-C₁₀)alkyle;
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention. Dans le cadre de la présente invention, on entend par :
- un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl, cyclopropylméthyl etc ;

- un groupe alkylène : un groupe alkyle divalent
- un groupe alcoxy : un radical -O-*a*lkyle où le groupe alkyle est tel que précédemment défini ;
- un groupe haloalkyle : un groupe alkyle substitué par un ou plusieurs atomes d'halogène identiques ou différents. A titre d'exemples, on peut citer les groupes CF₃, CH₂CF₃, CHF₂, CCl₃.
- un groupe haloalcoxy : un radical -O-*a*lkyle où le groupe alkyle est tel que précédemment défini et substitué par un ou plusieurs atomes d'halogène identiques ou différents. A titre d'exemples, on peut citer les groupes OCF₃, OCHF₂, OCCl₃.
- un groupe thioalkyle : un radical S-*a*lkyle où le groupe alkyle est tel que précédemment défini ;
- un groupe aryle : un groupe aromatique mono ou bicyclique comportant de 6 à 10 atomes. A titre d'exemples de groupe aryle, on peut citer les groupes phényle et naphthyle
- un groupe hétéroaryle : un groupe aromatique mono ou bicyclique comportant de 5 à 10 atomes dont de 1 à 4 hétéroatomes choisis parmi N, O et S. A titre d'exemples de groupes hétéroaryles, on peut citer : pyrrole, furane, thiophène, pyrazole, imidazole, triazole, tetrazole, oxazole, isoxazole, oxadiazole, thiazole, isothiazole, thiadiazole, pyridine, pyrimidine, pyrazine, pyridazine, triazine, thiéno-thiophène, furo-furane, thiéno-furane, furo-pyrrole, thiéno-pyrrole, pyrrolo-pyrrole, pyrrolo-isoxazole, furo-isoxazole, thiéno-isoxazole, isoxazolo-isoxazole, pyrrolo-oxazole, furo-oxazole, thiéno-oxazole, oxazolo-isoxazole, oxazolo-oxazole, pyrrolo-isothiazole, furo-isothiazole, thiéno-isothiazole, isothiazolo-isoxazole, isothiazolo-oxazole, isothiazolo-isothiazole, pyrrolo-thiazole, furo-thiazole, thiéno-thiazole, thiazolo-oxazole, thiazolo-isoxazole, thiazoio-isothiazole, thiazolo-thiazole, pyrrolo-pyrazole, furo-pyrazole, thiéno-pyrazole, pyrazolo-isoxazole, pyrazolo-oxazole, pyrazolo-isothiazole, pyrazolo-thiazole, pyrazolo-pyrazole, pyrrolo-imidazole, furo-imidazole, thiéno-imidazole, imidazo-isoxazole, imidazo-oxazole, imidazo-isothiazole, imidazo-thiazole, imidazo-pyrazole, imidazo-imidazole, pyrrolo-oxadiazole, furo-oxadiazole, thiéno-oxadiazole, pyrazolo-oxadiazole, imidazo-oxadiazole, furo-thiadiazole, thiéno-thiadiazole, pyrrolo-thiadiazole, imidazo-thiadiazole, pyrazolo-thiadiazole, thiéno-triazole, pyrrolo-triazole, furo-triazole, oxazolo-triazole, isoxazolo-triazole, thiazolo-triazole, isothiazolo-triazole, pyrazolo-triazole, imidazo-triazole, indole, isoindole, benzimidazole, indazole, indolizine, benzofurane, isobenzofurane, benzothiophène, benzo[c]thiophène, pyrrolopyridine, imidazopyridine, pyrazolopyridine, triazolopy-ridine, tétrazolopyridine, pyrrolopyrimidine, imidazopyrimidine, pyrazolopyrimidine, triazolopyrimidine, pyrrolopyrazine, imidazopyrazine, pyrazolopyrazine, triazolopyrazine, pyrrolopyridazine, imidazopyridazine, pyrazolopyridazine, triazolopyridazine, pyrrolotriazine, imidazotriazine, pyrazolotriazine, furopyridine, furopyrimidine, furopyrazine, furopyridazine, furotriazine, oxazolopyridine, oxazolopyrimidine, oxazolopyrazine, oxazolopyridazine, isoxazolopyridine, isoxazolopyrimidine, isoxazolopyrazine, isoxazolopyridazine, oxadiazolopyridine, benzoxazole, benzisoxazole, benzoxadiazole, thiénopyridine, thiénopyrimidine, thiénopyrazine, thiénopyridazine, thiénotriazine, thiazolopyridine, thiazolopyrimidine, thiazolopyrazine, thiazolopyridazine, isothiazolopyridine, isothiazolopyrimidine, isothiazolopyrazine, isothiazolopyridazine, thiadiazolopyridine, benzothiazole, benzoisothiazole, benzothiadiazole, benzotriazole, quinoléine, isoquinoléine, cinnoline, phthalazine, quinoxaline, quinazoline, naphthyridine, benzotriazine, pyridopyrimidine, pyridopyrazine, pyridopyridazine, pyridotriazine, pyrimidopyrimidine, pyrimidopyrazine, pyrimidopyridazine, pyrazinopyrazine, pyrazinopyridazine, pyridazinopyridazine.
- un groupe hétérocyclique : un groupe bicyclique de 9 à 10 atomes comportant de 1 à 4 hétéroatomes choisis parmi N, O et S, dont un cycle est aromatique et l'autre cycle est saturé ou partiellement saturé, chacun des cycles ne comportant au maximum que 2 hétéroatomes. A titre d'exemples de groupes bicycliques, on peut citer : benzodioxole, benzoxathiole, benzopyrane, benzothiopyrane, benzoxazine, benzothiazine, benzodioxine, benzothioxine, dioxolo-pyridine, oxathiolo-pyridine, pyrano-pyridine, thiopyrano-pyridine, oxazino-pyridine, thiazino-pyridine, dioxino-pyridine, thioxino-pyridine, dioxolo-pyrimidine, oxathiolo-pyrimidine, pyrano-pyrimidine, thiopyrano-pyrimidine, oxazino-pyrimidine, thiazino-pyrimidine, dioxino-pyrimidine, thioxino-pyrimidine, dioxolo-pyrazine, oxathiolo-pyrazine, pyrano-pyrazine, thiopyrano-pyrazine, oxazino-pyrazine, thiazino-pyrazine, dioxino-pyrazine, thioxino-pyrazine, dioxolo-pyridazine, oxathiolo-pyridazine, pyrano-pyridazine, thiopyrano-pyridazine, oxazino-pyridazine, thiazino-pyridazine, dioxino-pyridazine, thioxino-pyridazine, indole, isoindole, benzimidazole, indazole, indolizine, benzofuran, isobenzofuran, benzothiophene, benzo[c]thiophene pyrrolopyridine, imidazopyridine, pyrazolopyridine, pyrrolopyrimidine, imidazopyrimidine, pyrazolopyrimidine, pyrrolopyrazine, imidazopyrazine, pyrazolopyrazine, pyrrolopyridazine, imidazopyridazine, pyrazolopyridazine, furopyridine, furopyrimidine, furopyrazine,furopyridazine, oxazolopyridine, oxazolopyrimidine, oxazolopyrazine, oxazolopyridazine, isoxazolopyridine, isoxazolopyrimidine, isoxazolopyrazine, isoxazolopyridazine, benzoxazole, benzisoxazole, benzoxadiazole, thienopyridine, thienopyrimidine, thienopyrazine, thienopyridazine, thiazolopyridine, thiazolopyrimidine, thiazolopyrazine, thiazolopyridazine, isothiazolopyridine, isothiazolopyrimidine, isothiazolopyrazine, isothiazolopyridazine, benzothiazole, benzoisothiazole, quinoléine, isoquinoléine, cinnoline, phthalazine, quinoxaline, quinazoline, naphthyridine, pyridopyrimidine, pyridopyrazine, pyridopyridazine, pyrimidopyrimidine, pyrixnidopyrazine, pyrimidopyridazine, pyrazinopyrazine, pyrazinopyridazine, pyridazinopyridazine, l'un des cycles de ces groupes bicycliques étant sous forme saturée ou partiellement saturée, par exemple dihydrobenzofurane, tétrahydroquinoléine, dihydrobenzoxazole, benzodioxole.
- les atomes de soufre et d'azote peuvent être à l'état oxydé (N-oxide, sulfoxide, sulfone).
Parmi les composés de formule (I) figurent les composés (I) objets de l'invention pour lesquels,
Het représente un groupe hétéroaryle monocyclique comportant de 5 à 6 atomes dont de 1 à 3 hétéroatomes choisis parmi N, O et S ;
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, (C₁-C₁₀)alkyle, (C₁-C₁₀)alkoxy, CONRaRb, NRaRb , (C₁-C₁₀)thioalkyle, -S(O)₂(C₁-C₁₀-alkyle), halo(C₁-C₁₀)alkyle, hydroxy(C₁-C₁₀)alkylène, NRaRb(C₁-C₁₀)alkylène, NRcCORd, SO₂NRaRb Cyano, nitro ;
Ra et Rb représentent, indépendamment l'un de l'autre, un atome d'hydrogène ou un groupe (C₁-C₁₀)alkyle ;
X représente de 1 à 3 substituants identiques ou différents l'un de l'autre choisis parmi un hydrogène, un halogène, les groupes (C₁-C₁₀)alkyle ;
R représente en position 3 de l'imidazo[1,2-a]pyridine 1 substituant choisi parmi un hydrogène, (C₁-C₁₀)alkyle ;
R₂ et R₃ représentent, indépendamment l'un de l'autre, un atome d'hydrogène ou un groupe (C₁-C₁₀)alkyle ;
R₄ représente : un atome d'hydrogène ou un groupe (C₁-C₁₀)alkyle ;
à l'état de base ou de sel d'addition à un acide.

Parmi les composés de formule (I), un premier groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, (C₁-C₁₀)alkyle, (C₁-C₁₀)alkoxy, CONRaRb, NRaRb , (C₁-C₁₀)thioalkyle, -S(O)₂(C₁-C₁₀-alkyle), halo(C₁-C₁₀)alkyle, hydroxy(C₁-C₁₀)alkylène, NRaRb(C₁-C₁₀)alkylène, NRcCORd, SO₂NRaRb, Cyano, nitro ;
Ra et Rb représentent, indépendamment l'un de l'autre, un atome d'hydrogène ou un groupe (C₁-C₁₀)alkyle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₁₀)alkyle, aryle ou aryl(C₁-C₁₀)alkylène ;
Rc et Rd représentent, indépendamment l'un de l'autre, un atome d'hydrogène ou un groupe (C₁-C₁₀)alkyle ;
les autre substituants étant tels que définis précédemment.

Parmi les composés de formule (I), un deuxième groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, (C₁-C₁₀)alkyle, (C₁-C₁₀)alkoxy, CONRaRb, NRaRb, (C₁-C₁₀)thioalkyle, -S(O)₂(C₁-C₁₀-alkyle), halo(C₁-C₁₀)alkyle, hydroxy(C₁-C₁₀)alkylène, NRaRb(C₁-C₁₀)alkylène, NRcCORd, SO₂NRaRb cyano, nitro ;
Ra et Rb représentent, indépendamment l'un de l'autre, un atome d'hydrogène ou un groupe (C₁-C₁₀)alkyle;
Rc et Rd représentent, indépendamment l'un de l'autre, un atome d'hydrogène ou un groupe (C₁-C₁₀)alkyle ;
les autre substituants étant tels que définis précédemment.

Parmi les composés de formule (I), un troisème groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, méthyle, méthoxy, hydroxyméthyle, CON(CH₃)₂, morpholinyle, pyrrolidinyl-éthyle, NHCO-CH(CH₃)₂, CH₂N(CH₃)₂, NH₂, CONHCH(CH3)₂, pyrrolidinyle, méthylsulfonyle, trifluorométhyle, méthylthio, cyano, nitro, -NHCO(CH₃), CONH(CH₃), CONHC(CH₃)₃ -SO₂N(CH₃)₂, isopentyl ;
les autre substituants étant tels que définis précédemment.

Parmi les composés de formule (I), un quatrième groupe de composés est constitué des composés pour lesquels :
Het représente un groupe furyle, thiényle, pyridinyle, thiazolyle, pyrazolyle, imidazolyle ;
les autre substituants étant tels que définis précédemment.

Parmi les composés de formule (I), un cinquième groupe de composés est constitué des composés pour lesquels :
X représente de 1 à 3 substituants identiques ou différents l'un de l'autre choisis parmi parmi un hydrogène, un halogène, les groupes (C₁-C₁₀)alkyle ;
les autre substituants étant tels que définis précédemment.

Parmi les composés de formule (I), un sixième groupe de composés est constitué des composés pour lesquels :
X représente de 1 à 3 substituants identiques ou différents l'un de l'autre choisis parmi un hydrogène, un fluor, un groupe méthyle ;
les autre substituants étant tels que définis précédemment.

Parmi les composés de formule (I), un septième groupe de composés est constitué des composés pour lesquels :
R représente en position 3, 5, 7 ou 8 de l'imidazo[1,2-*a*]pyridine de 1 à 4 substituants identiques ou différents l'un de l'autre choisi parmi un hydrogène, (C₁-C₁₀)alkyle ;
les autre substituants étant tels que définis précédemment.

Parmi les composés de formule (I), un huitième groupe de composés est constitué des composés pour lesquels :
R représente en position 3, 5, 7 ou 8 de l'imidazo[1,2-*a*]pyridine de 1 à 4 atomes d'hydrogène, ou 1 groupe méthyle ;
les autre substituants étant tels que définis précédemment.

Parmi les composés de formule (I) objets de l'invention, un neuvième groupe de composés est constitué des composés pour lesquels :
R représente en position 3, de l'imidazo[1,2-*a*]pyridine 1 atome d'hydrogène, ou 1 groupe méthyle ;
les autre substituants étant tels que définis précédemment.

Parmi les composés de formule (I), un dixième groupe de composés est constitué des composés pour lesquels :
R₂ et R₃ représentent, indépendamment l'un de l'autre, un atome d'hydrogène ou un groupe (C₁-C₁₀)alkyle ;
les autre substituants étant tels que définis précédemment.

Parmi les composés de formule (I), un onzième groupe de composés est constitué des composés pour lesquels :
R₂ et R₃ représentent, indépendamment l'un de l'autre, un atome d'hydrogène ou un groupe méthyle ;
les autre substituants étant tels que définis précédemment.

Parmi les composés de formule (I), un douzième groupe de composés est constitué des composés pour lesquels :
R₄ représente : un atome d'hydrogène ou un groupe (C₁-C₁₀)alkyle ;
les autre substituants étant tels que définis précédemment.

Parmi les composés de formule (I), un treizième groupe de composés est constitué des composés pour lesquels :
R₄ représente : un atome d'hydrogène ou un groupe méthyle ;
les autre substituants étant tels que définis précédemment.

Parmi les composés de formule (I), un quatorzième groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, (C₁-C₁₀)alkyle, (C₁-C₁₀)alkoxy, - CONRaRb, -NRaRb, (C₁-C₁₀)thioalkyle, -S(O)₂(C₁-C₁₀-alkyle), halo(C₁-C₁₀)alkyle, hydroxy(C₁-C₁₀)alkylène, NRaRb(C₁-C₁₀)alkylène, NRcCORd, SO₂NRaRb cyano, nitro ;
Ra et Rb représentent, indépendamment l'un de l'autre, un atome d'hydrogène ou un groupe (C₁-C₁₀)alkyle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₁₀)alkyle, aryle ou aryl(C₁-C₁₀)alkylène ;
Rc et Rd représentent, indépendamment l'un de l'autre, un atome d'hydrogène ou un groupe (C₁-C₁₀)alkyle ;
Het représente un groupe furyle, thiényle, pyridinyle, thiazolyle, pyrazolyle, imimdazolyle ; X représente de 1 à 3 substituants identiques ou différents l'un de l'autre choisis parmi parmi un hydrogène, un halogène, les groupes (C₁-C₁₀)alkyle ;
R représente en position 3, 5, 7 ou 8 de l'imidazo[1,2-*a*]pyridine de 1 à 4 substituants identiques ou différents l'un de l'autre choisi parmi un hydrogène, un halogène, (C₁-C₁₀)alkyle ;
R₂ et R₃ représentent, indépendamment l'un de l'autre, un atome d'hydrogène ou un groupe (C₁-C₁₀)alkyle ;
R₄ représente : un atome d'hydrogène ou un groupe (C₁-C₁₀)alkyle ;
à l'état de base ou de sel d'addition à un acide.

Parmi les composés de formule (I), un quinzième groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, (C₁-C₁₀)alkyle, (C₁-C₁₀)alkoxy, CONRaRb, NRaRb, (C₁-C₁₀)thioalkyle, -S(O)₂(C₁-C₁₀-*a*lkyle), halo(C₁-C₁₀)alkyle, hydroxy(C₁-C₁₀)alkylène, NRaRb(C₁-C₁₀)alkylène, NRcCORd, SO₂NRaRb, cyano, nitro ;
Ra et Rb représentent, indépendamment l'un de l'autre, un atome d'hydrogène ou un groupe (C₁-C₁₀)alkyle ;
Rc et Rd représentent, indépendamment l'un de l'autre, un atome d'hydrogène ou un groupe (C₁-C₁₀)alkyle ;
Het représente un groupe furyle, thiényle, pyridinyle, thiazolyle, pyrazolyle, imimdazolyle ; X représente de 1 à 3 substituants identiques ou différents l'un de l'autre choisis parmi parmi un hydrogène, un halogène, les groupes (C₁-C₁₀)alkyle ;
R représente en position 3, 5, 7 ou 8 de l'imidazo[1,2-*a*]pyridine de 1 à 4 substituants identiques ou différents l'un de l'autre choisi parmi un hydrogène, un halogène, (C₁-C₁₀)alkyle;
R₂ et R₃ représentent, indépendamment l'un de l'autre, un atome d'hydrogène ou un groupe (C₁-C₁₀)alkyle;
R₄ représente : un atome d'hydrogène ou un groupe (C₁-C₁₀)alkyle
à l'état de base ou de sel d'addition à un acide.

Parmi les composés de formule (I) objets de l'invention, un seizième groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, méthyle, méthoxy, hydroxyméthyle, -CON(CH₃)₂, morpholinyle, pyrrolidinyl-éthyle, -NHCO-CH(CH₃)₂, - CH₂N(CH₃)₂ , -NH₂, -CONHCH(CH3)₂, pyrrolidinyle, méthylsulfonyle, trifluorométhyle, méthylthio, cyano, nitro, -NHCO(CH₃), CONH(CH₃), CONHC(CH₃)₃ -SO₂N(CH₃)₂, isopentyl ;
Het représente un groupe furyle, thiényle, pyridinyle, thiazolyle, pyrazolyle, imidazolyle ; X représente de 1 à 3 substituants identiques ou différents l'un de l'autre choisis parmi un hydrogène, un fluor, un groupe méthyle ;
R représente en position 3 de l'imidazo[1,2-*a*]pyridine 1 atome d'hydrogène ou groupe méthyle ;
R₂ et R₃ représentent, indépendamment l'un de l'autre, un atome d'hydrogène ou un groupe méthyle ;
R₄ représente : un atome d'hydrogène ou un groupe méthyle ;
à l'état de base ou de sel d'addition à un acide.

Parmi les composés de formule (I), un dix-septième groupe de composés est constitué des composés pour lesquels :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, méthyle, méthoxy, hydroxyméthyle, -CON(CH₃)₂, morpholinyle, pyrrolidinyl-éthyle, -NHCO-CH(CH₃)₂, - CH₂N(CH₃)₂ , -NH₂, -CONHCH(CH3)₂, pyrrolidinyle, méthylsulfonyle, trifluorométhyle, méthylthio, cyano, nitro, -NHCO(CH₃), CONH(CH₃), CONHC(CH₃)₃ -SO₂N(CH₃)₂, isopentyl ;
Het représente un groupe furyle, thiényle, pyridinyle, thiazolyle, pyrazolyle, imidazolyle ; X représente de 1 à 3 substituants identiques ou différents l'un de l'autre choisis parmi un hydrogène, un fluor, un groupe méthyle ;
R représente en position 3, 5, 7 ou 8 de l'imidazo[1,2-*a*]pyridine de 1 à 4 atomes d'hydrogène, ou 1 groupe méthyle ;
R₂ et R₃ représentent, indépendamment l'un de l'autre, un atome d'hydrogène ou un groupe méthyle ;
R₄ représente : un atome d'hydrogène ou un groupe méthyle
à l'état de base ou de sel d'addition à un acide.

Parmi les composés de formule (I) objets de l'invention, un dix-huitième groupe de composés est constitué des composés pour lesquels
R₁ représente un groupe phényle, furyle ou quinoléinyle, ces groupes pouvant éventuellement être substitués par un ou plusieurs atomes ou groupes choisis indépendamment les uns des autres parmi halogène, (C₁-C₁₀)alkyle ;
Het représente un groupe furyle, un groupe thiényle, un groupe pyridinyle ou un groupe thiazolyle ;
X représente un hydrogène ;
R est un hydrogène ;
R₂ et R₃ représentent, indépendamment l'un de l'autre, un atome d'hydrogène ;
R₄ représente un atome d'hydrogène,
à l'état de base ou de sel d'addition à un acide.

Parmi les composés de formule (I) objets de l'invention, on peut notamment citer les composés suivants :
- {5-[2-(4-Chlorophényl)imidazo[1,2-*a*]pyridin-6-yl]furan-2-yl}méthanol ;
- {4-[2-(4-Chlorophényl)imidazo[1,2-*a*]pyridin-6-yl]thièn-2-yl}méthanol ;
- {5-[2-(4-Chlorophényl)imidazo[1,2-*a*]pyridin-6-yl]thièn-2-yl}méthanol ;
- {2-[2-(4-Chlorophényl)imidazo[1,2-*a*]pyridin-6-yl]pyridin-4-yl}méthanol ;
- {5-[2-(4-Chlorophényl)imidazo[1,2-*a*]pyridin-6-yl]pyridin-3-yl}méthanol ;
- {4-[2-(4-Chlorophényl)imidazo[1,2-*a*]pyridin-6-yl]furan-2-yl}méthanol ;
- {6-[2-(4-Chlorophényl)imidazo[1,2-*a*]pyridin-6-yl]pyridin-2-yl}méthanol ;
- {5-[2-(4-Chlorophényl)imidazo[1,2-*a*]pyridin-6-yl]thièn-3-yl}méthanol ;
- {2-[2-(4-Chlorophényl)imidazo[1,2-*a*]pyridin-6-yl]thiazol-5-yl}méthanol ;
- {2-[2-(4-Chlorophényl)imidazo[1,2-*a*]pyridin-6-yl]thiazol-4-yl}méthanol ;
- {4-[2-(4-Chlorophenyl)imidazo[1,2-*a*]pyridin-6-yl]thiazol-2-yl}méthanol ;
- [2-(2-Furan-3-ylimidazo[1,2-*a*]pyridin-6-yl)pyridin-4-yl]méthanol et son chlorhydrate ;
- [5-(2-Quinoléin-3-ylimidazo[1,2-*a*]pyridin-6-yl)furan-2-yl]méthanol et son chlorhydrate ;
- [4-(2-*p*Tolylimidazo[1,2-a]pyridin-6-yl)thién-2-yl]méthanol ;
- [2-(2-Quinoléin-3-ylimidazo[1,2-*a*]pyridin-6-yl)pyridin-4-yl]méthanol et son chlorhydrate ;
- [4-(2-Quinoléin-3-ylimidazo[1,2-*a*]pyridin-6-yl)thièn-2-yl]méthanol et son chlorhydrate ;
- {4-[2-(1*H*-Indol-5-yl)imidazo[1,2-*a*]pyridin-6-yl]thién-2-yl}méthanol ;
- {5-[2-(1*H*-Indol-5-yl)imidazo[1,2-*a*]pyridin-6-yl]furan-2-yl}méthanol et son chlorhydrate ;
- [4-(2-Phénylimidazo[1,2-*a*]pyridin-6-yl)thién-2-yl]méthanol et son chlorhydrate ;
- {4-[2-(1*H*-Pyrrolo[2,3-i]pyridin-5-yl)imidazo[1,2-*a*]pyridin-6-yl]thién-2-yl}méthanol et son chlorhydrate ;
- {4-[2-(3-Méthoxyphényl)imidazo[1,2-*a*]pyridin-6-yl]thién-2-yl}méthanol et son chlorhydrate ;
- {2-[2-(1*H*-Pyrrolo[2,3-b]pyridin-5-yl)imidazo[1,2-*a*]pyridin-6-yl]pyridin-4-yl}méthanol et son chlorhydrate ;
- [2-(2-Phénylimidazo[1,2-*a*]pyridin-6-yl)pyridin-4-yl]méthanol ;
- [5-(2-pTolylimidazo[1,2-*a*]pyridin-6-yl)furan-2-yl]méthanol ;
- [5-(2-Phénylimidazo[1,2-*a*]pyridin-6-yl)furan-2-yl]méthanol ;
- {5-[2-(3-Méthoxyphényl)imidazo[1,2-*a*]pyridin-6-yl]furan-2-yl}méthanol ;
- {5-[2-(2,4-Difluorophényl)imidazo[1,2-*a*]pyridin-6-yl]furan-2-yl}méthanol ;
- {5-[2-(4-Chlorophényl)imidazo[1,2-*a*]pyridin-6-yl]furan-3-yl}méthanol ;
- 2-(4-Chlorophényl)-6-(4-méthoxyméthyl-furan-2-yl)imidazo[1,2-*a*]pyridine ;
- {5-[2-(4-Hydroxyméthyl-phényl)imidazo[1,2-*a*]pyridin-6-yl]furan-2-yl}méthanol ;
- 4-[6-(5-Hydroxyméthyl-furan-2-yl)imidazo[1,2-*a*]pyridin-2-yl]-*N,N-*diméthylbenzamide ;
- {5-[2-(1*H*-Indol-6-yl)imidazo[1,2-*a*]pyridin-6-yl]furan-2-yl}méthanol ;
- {5-[2-(2-Méthoxypyridin-3-yl)imidazo[1,2-*a*]pyridin-6-yl]furan-2-yl}méthanol ;
- [5-(2-Quinoléin-5-ylimidazo[1,2-*a*]pyridin-6-yl)furan-2-yl]méthanol ;
- {5-[2-(4-Morpholin-4-ylphényl)imidazo[1,2-*a*]pyridin-6-yl]furan-2-yl}méthanol ;
- [5-(2-Isoquinoléin-5-ylimidazo[1,2-*a*]pyridin-6-yl)furan-2-yl]méthanol ;
- (5-{2-[4-(1-Pyrrolidin-1-yléthyl)phényl]imidazo[1,2-*a*]pyridin-6-yl}furan-2-yl)méthanol ;
- 4-[6-(5-Hydroxyméthyl-thién-3-yl)imidazo[1,2-*a*]pyridin-2-yl]-*N,N-*diméthylbenzamide ;
- {4-[2-(2-Méthoxypyridin-3-yl)imidazo[1,2-*a*]pyridin-6-yl]thién-2-yl}méthanol ;
- [4-(2-Isoquinolin-5-ylimidazo[1,2-*a*]pyridin-6-yl)thién-2-yl]méthanol ;
- N-{3-[6-(5-Hydroxyméthyl-thién-3-yl)imidazo[1,2-*a*]pyridin-2-yl]phényl}isobutyramide ;
- (4-{2-[4-(1-Pyrrolidin-1-yléthyl)phényl]imidazo[1,2-*a*]pyridin-6-yl}thién-2-yl)méthanol ;
- {4-[2-(4-Diméthylaminométhylphényl)imidazo[1,2-*a*]pyridin-6-yl]thién-2-yl}méthanol ;
- 2-Fluoro-4-[6-(5-hydroxyméthyl-thién-3-yl)imidazo[1,2-*a*]pyridin-2-yl]-*N,N-*diméthylbenzamide ;
- {4-[2-(3-aminophényl)imidazo[1,2-*a*]pyridin-6-yl]thién-2-yl}méthanol;
- *N*-tertButyl-5-[6-(5-hydroxyméthylthién-3-yl)imidazo[1,2-*a*]pyridin-2-yl]nicotinamide ;
- [5-(3-Méthyl-2-phénylimidazo[1,2-*a*]pyridin-6-yl)furan-2-yl]méthanol ;
- [5-(3-Méthyl-2-thién-2-ylimidazo[1,2-*a*]pyridin-6-yl)furan-2-yl]méthanol ;
- {5-[2-(3-Chlorophényl)-3-méthylimidazo[1,2-*a*]pyridin-6-yl]furan-2-yl}méthanol ;
- {5-[2-(4-Pyrrolidin-1-ylphényl)imidazo[1,2-*a*]pyridin-6-yl]furan-2-yl}méthanol ;
- 3-[6-(5-Hydroxyméthylfuran-2-yl)imidazo[1,2-*a*]pyridin-2-yl]benzonitrile ;
- [5-(2-Benzofuran-2-ylimidazo[1,2-*a*]pyridin-6-yl)furan-2-yl]méthanol ;
- [5-(2-Benzothiazol-2-ylimidazo[1,2-*a*]pyridin-6-yl)furan-2-yl]méthanol ;
- [5-(2-Benzo[b]thién-2-ylimidazo[1,2-*a*]pyridin-6-yl)furan-2-yl]méthanol ;
- {4-[2-(4-Nitrophényl)imidazo[1,2-*a*]pyridin-6-yl]thién-2-yl}méthanol
- [4-(2-Thién-3-ylimidazo[1,2-*a*]pyridin-6-yl)thién-2-yl]méthanol ;
- {4-[2-(4-Méthylthiophényl)imidazo[1,2-*a*]pyridin-6-yl]thién-2-yl}méthanol
- {4-[2-(5-Chlorothién-2-yl)imidazo[1,2-*a*]pyridin-5-yl]thién-2-yl}méthanol ;
- {4-[2-(4-Méthylsulfonyl-phényl)imidazo[1,2-*a*]pyridin-6-yl]thién-2-yl}méthanol ;
- [4-(2-Benzo[b]thién-2-ylimidazo[1,2-*a*]pyridin-6-yl)thién-2-yl]méthanol ;
- [4-(2-Benzo[b]thién-5-ylimidazo[1,2-*a*]pyridin-6-yl)thién-2-yl]méthanol ;
- [4-(3-Méthyl-2-phény]imidazo[1,2-*a*]pyridin-6-yl)thiazol-2-yl]métllanol ;
- [4-(3-Méthyl-2-thién-2-ylimidazo[1,2-*a*]pyridin-6-yl)thiazol-2-yl]méthanol
- {4-[2-(4-Méthoxyphényl)-3-méthylimidazo[1,2-*a*]pyridin-6-yl]thiazol-2-yl}méthanol ;
- {4-[2-(4-Pyrrolidin-1-yl-phényl)imidazo[1,2-*a*]pyridin-6-yl]thiazol-2-yl}méthanol ;
- {4-[2-(2-Fluorophényl)imidazo[1,2-*a*]pyridin-6-yl]thiazol-2-yl}méthanol ;
- {4-[2-(4-Méthylsulfonyl-phényl)imidazo[1,2-*a*]pyridin-6-yl]thiazol-2-yl}méthanol ;
- [4-(2-Benzofuran-2-ylimidazo[1,2-*a*]pyridin-6-yl)thiazol-2-yl]méthanol ;
- [4-(2-Benzo[b]thién-2-ylimidazo[1,2-*a*]pyridin-6-yl)thiazol-2-yl]méthanol ;
- [4-(2-Furan-2-ylimidazo[1,2-*a*]pyridin-6-yl)thiazol-2-yl]méthanol ;
- [4-(2-Benzo[b]thién-5-ylirnidazo[1,2-*a*]pyridin-6-yl)thiazol-2-yl]méthanol ;
- {4-[2-(4-Pyrrolidin-1-ylphényl)imidazo[1,2-*a*]pyridin-6-yl]thién-2-yl}méthanol ;
- 1-{4-[2-(4-Chlorophényl)imidazo[1,2-*a*]pyridin-6-yl]thién-2-yl}éthanol ;
- 2-{4-[2-(4-Chlorophényl)imidazo[1,2-*a*]pyridin-6-yl]thién-2-yl}propan-2-ol ;
- {5-[2-(4-Méthoxyphényl)-3-méthylimidazo[1,2-*a*]pyridin-6-yl]furan-2-yl}méthanol ;
- {5-[2-(4-Trifluorométhylphényl)imidazo[1,2-*a*]pyridin-6-yl]furan-2-yl-méthanol ;
- {5-[2-(4-Methoxyphényl)imidazo[1,2-*a*]pyridin-6-yl]furan-2-yl}méthanol ;
- {5-[2-(4-Fluorophényl)imidazo[1,2-*a*]pyridin-6-yl]furan-2-yl}méthanol ;
- {5-[2-(3,4-Difluorophényl)imidazo[1,2-*a*]pyridin-6-yl]furan-2-yl}méthanol ;
- [5-(2-Thién-3-ylimidazo[1,2-*a*]pyridin-6-yl)furan-2-yl]méthanol ;
- {5-[2-(5-Chlorothién-2-yl)imidazo[1,2-*a*]pyridin-6-yl]furan-2-yl}méthanol ;
- {5-[2-(2-Fluorophényl)imidazo[1,2-*a*]pyridin-6-yl]furan-2-yl}méthanol ;
- {5-[2-(3-Bromophényl)imidazo[1,2-*a*]pyridin-6-yl]furan-2-yl}méthanol ;
- {5-[2-(4-Chloro-3-méthylphényl)imidazo[1,2-*a*]pyridin-6-yl]furan-2-yl}méthanol ;
- {5-[2-(4-Méthylsulfonyl-phényl)imidazo[1,2-*a*]pyridin-6-yl]furan-2-yl}méthanol ;
- [5-(2-Thién-2-ylimidazo[1,2-*a*]pyridin-6-yl)furan-2-yl]méthanol ;
- [5-(2-Benzofuran-3-ylimidazo[1,2-*a*]pyridin-6-yl)furan-2-yl]méthanol ;
- {5-[2-(2,3-Dihydrobenzofuran-5-yl)imidazo[1,2-*a*]pyridin-6-yl]furan-2-yl}méthanol ;
- [5-(2-Furan-2-ylimidazo[1,2-*a*]pyridin-6-yl)furan-2-yl]méthanol ;
- [5-(2-Benzofuran-5-ylimidazo[1,2-*a*]pyridin-6-yl)furan-2-yl]méthanol ;
- [5-(2-Thiazol-2-ylimidazo[1,2-*a*]pyridin-6-yl)furan-2-yl]méthanol ;
- {5-[2-(4-Méthylthiophényl)imidazo[1,2-*a*]pyridin-6-yl]furan-2-yl}méthanol ;
- {4-[2-(4-Chlorophényl)-3-méthylimidazo[1,2-*a*]pyridin-6-yl]thién-2-yl}méthanol;
- [4-(3-Méthyl-2-thién-2-ylimidazo[1,2-*a*]pyridin-6-yl)thién-2-yl]méthanol ;
- {4-[2-(3-Chlorophényl)-3-méthylimidazo[1,2-*a*]pyridin-6-yl]thién-2-yl}méthanol ;
- {4-[2-(2,4-Difluorophényl)-3-methylimidazo[1,2-*a*]pyridin-6-yl]thién-2-yl}méthanol :
- {4-[2-(4-Fluorophényl)imidazo[1,2-*a*]pyridin-6-yl]thién-2-yl}méthanol ;
- {4-[2-(3,4-Difluorophényl)imidazo[1,2-*a*]pyridin-6-yl]thién-2-yl}méthanol ;
- 3-[6-(5-Hydroxyméthyl-thién-3-yl)imidazo[1,2-*a*]pyridin-2-yl]benzonitrile ;
- {4-[2-(2-Fluorophényl)imidazo[1,2-*a*]pyridin-6-yl]thién-2-yl}méthanol ;
- {4-[2-(3-Bromophényl)imidazo[1,2-*a*]pyridin-6-yl]thién-2-yl}méthanol ;
- [4-(2-Benzofuran-2-ylimidazo[1,2-*a*]pyridin-6-yl)thién-2-yl]méthanol ;
- [4-(2-Thién-2-ylimidazo[1,2-*a*]pyridin-6-yl)thién-2-yl]méthanol ;
- [4-(2-Benzofuran-3-ylimidazo[1,2-*a*]pyridin-6-yl)thién-2-yl]méthanol ;
- {4-[2-(1-Méthyl-1*H*-benzimidazol-2-yl)imidazo[1,2-*a*]pyridin-6-yl]thién-2-yl}méthanol ;
- {4-[2-(2,3-Dihydrobenzofuran-5-yl)imidazo[1,2-*a*]pyridin-C-yl]thién-2-yl}méthanol ;
- [4-(2-Furan-2-ylimidazo[1,2-*a*]pyridin-6-yl)thién-2-y]méthanol ;
- [4-(2-Benzofuran-5-ylimidazo[1,2-*a*]pyridin-6-yl)thién-2-yl]méthanol ;
- [4-(2-Thiazol-2-ylimidazo[1,2-*a*]pyridin-6-yl)thién-2-yl]méthanol ;
- {4-[2-(4-Chlorophényl)-3-méthylimidazo[1,2-*a*]pyridin-6-yl]thiazol-2-yl}méthanol ;
- {4-[2-(3-Chlorophényl)-3-méthylimidazo[1,2-*a*]pyridin-6-yl]thiazol-2-yl}méthanol ;
- {4-[2-(4-Trifluorométhylphényl)imidazo[1,2-*a*]pyridin-6-yl]thiazol-2-yl}méthanol ;
- {4-[2-(4-Fluorophényl)imidazo[1,2-*a*]pyridin-6-yl]thiazol-2-yl}méthanol ;
- {4-[2-(3,4-Difluorophényl)imidazo[1,2-*a*]pyridin-6-yl]thiazol-2-yl}méthanol ;
- [4-(2-Thién-3-ylimidazo[1,2-*a*]pyridin-6-yl)thiazol-2-yl]méthanol ;
- [4-(2-Pyridin-2-ylimidazo[1,2-*a*]pyridin-6-yl)thiazol-2-yl]méthanol ;
- {4-[2-(5-Chlorothién-2-yl)imidazo[1,2-*a*]pyridin-6-yl]thiazol-2-yl}méthanol ;
- {4-[2-(3-Bromophényl)imidazo[1,2-*a*]pyridin-6-yl]thiazol-2-yl}méthanol ;
- {4-[2-(4-Chloro-3-méthylphényl)imidazo[1,2-*a*]pyridin-6-yl]thiazol-2-yl}méthanol ;
- [4(2-Thién-2-ylimidazo[1,2-*a*]pyridin-6-yl)thiazol-2-yl]méthanol ;
- [4-(2-Benzofuran-3-ylimidazo[1,2-*a*]pyridin-6-yl)thiazol-2-yl]méthanol ;
- {4-[2-(2,3-Dihydrobenzofuran-5-yl)imidazo[1,2-*a*]pyridin-6-yl]thiazol-2-yl}méthanol ;
- [4-(2-Benzofuran-5-ylimidazo[1,2-*a*]pyridin-6-yl)thiazol-2-yl]méthanol ;
- [4-(2-Thiazol-2-ylimidazo[1,2-*a*]pyridin-6-yl)thiazol-2-yl]méthanol ;
- {5-[2-(3-Hydroxyméthylphényl)imidazo[1,2-*a*]pyridin-6-yl]furan-2-yl}méthanol ;
- N-{3-[6-(5-Hydroxyméthylfuran-2-yl)imidazo[1,2-*a*]pyridin-2-yl]phényl}*iso*butyramide ;
- 2-Fluoro-4-[6-(5-hydroxyméthyl-furan-2-yl)imidazo[1,2-*a*]pyridin-2-yl]-*N,N-*diméthylbenzamide ;
- 2-{4-[2-(*1H*-Indol-6-yl)imidazo[1,2-*a*]pyridin-6-yl]thién-2-ylpropan-2-ol
- N-{3-[6-(2-Hydroxyméthylthiazol-4-yl)imidazo[1,2-*a*]pyridin-2-yl]phényl}acétamide ;
- {4-[2-(4-Hydroxyméthylphényl)imidazo[1,2-*a*]pyridin-6-yl]thiazol-2-yl}méthanol ;
- {4-[2-(3-Hydroxyméthylphényl)imidazo[1,2-*a*]pyridin-6-yl]thiazol-2-yl}méthanol
- {4-[2-(1*H*-Indol-5-yl)imidazo[1,2-*a*]pyridin-6-yl]thiazol-2-yl}méthanol ;
- {4-[2-(4-Méthylthién-2-yl)imidazo[1,2-*a*]pyridin-6-yl]thiazol-2-yl}méthanol ;
- {4-[2-(1*H*-Indol-4-yl)imidazo[1,2-*a*]pyridin-6-yl]thiazol-2-yl}méthanol ;
- {4-[2-{2-Fluoropyridin-3-yl)imidazo[1,2-*a*]pyridin-6-yl]thiazol-2-yl}méthanol ;
- 3-[6-(2-Hydroxyméthylthiazol-4-yl)imidazo[1,2-*a*]pyridin-2-yl]-*N,N-*diméthylbenzamide ;
- {4-[2-(1H-Indol-6-yl)imidazo[1,2-*a*]pyridin-6-yl]thiazol-2-yl}méthanol ;
- {4-[2-(2-Méthoxypyridin-3-yl)imidazo[1,2-*a*]pyridin-6-yl]thiazol-2-yl}méthanol ;
- 4-[6-(2-Hydroxyméthyl-thiazol-4-yl)imidazo[1,2-*a*]pyridin-2-yl]-*N-*méthylbenzamide ;
- {4-[2-(4-Méthylthién-3-yl)imidazo[1,2-*a*]pyridin-6-yl]thiazol-2-yl}méthanol ;
- {4-[2-(1-Méthyl-*1H*-indol-5-yl)imidazo[1,2-*a*]pyridin-6-yl]thiazol-2-yl}méthanol ;
- [4-(2-Quinoléin-5-ylimidazo[1,2-*a*]pyridin-6-yl)-thiazol-2-yl]méthanol ;
- N-{4-[6-(2-Hydroxyméthylthiazol-4-yl)imidazo[1,2-*a*]pyridin-2-yl]phényl}acétamide ;
- {4-[2-(4-Morpholin-4-ylphényl)imidazo[1,2-*a*]pyridin-6-yl]thiazol-2-yl}méthanol ;
- [4-(2-Isoquinoléin-5-ylimidazo[1,2-*a*]pyridin-6-yl)thiazol-2-yl]méthanol;
- N-{3-[6-(2-Hydroxyméthyl-thiazol-4-yl)imidazo[1,2-*a*]pyridin-2-yl]phényl}*iso*butyramide ;
- 3-[6-(2-Hydroxyméthylthiazol-4-yl)imidazo[1,2-*a*]pyridin-2-yl]-*N,N-*diméthylbenzènesulfonamide ;
- {4-[2-(2,6-Difluoropyridin-4-yl)imidazo[1,2-*a*]pyridin-6-yl]thiazol-2-yl}méthanol ;
- (4-{2-[1-(3-Méthylbutyl)-1*H*-pyrazol-4-yl]imidazo[1,2-*a*]pyridin-6-yl}thiazol-2-yl)méthanol ;
- 2-{4-[2-(1*H*-Indol-6-yl)imidazo[1,2-*a*]pyridin-6-yl]-1*H*-pyrazol-3-yl}propan-2-ol ;
- 2-{5-Fluoro-2-[2-(1*H*-indol-6-yl)imidazo[1,2-*a*]pyridin-6-yl]pyridin-4-yl}propan-2-ol;
- 2-{2-(2-(1*H*-Indol-6-yl)imidazo[1,2-*a*]pyridin-6-yl]pyridin-4-yl}propan-2-ol ;
- 2-{2-[2-(1*H*-Indazol-3-yl)imidazo[1,2-*a*]pyridin-6-yl]pyridin-4-yl}propan-2-ol ;
- 2-{2-[2-(5-Méthylisoxazol-3-yl)imidazo[1,2-*a*]pyridin-6-yl]pyridin-4-yl}propan-2-ol ;
- {5-[2-(1*H*-Indol-6-yl)imidazo[1,2-*a*]pyridin-6-yl]-1-méthyl-*1H*-imidazol-2-yl}méthanol ;
- 2-{5-[2-(4-Chlorophényl)imidazo[1,2-*a*]pyridin-6-yl]furan-2-yl}propan-2-ol ;

Conformément à l'invention, on peut préparer les composés de formule générale (1) selon le procédé décrit dans le schéma I.

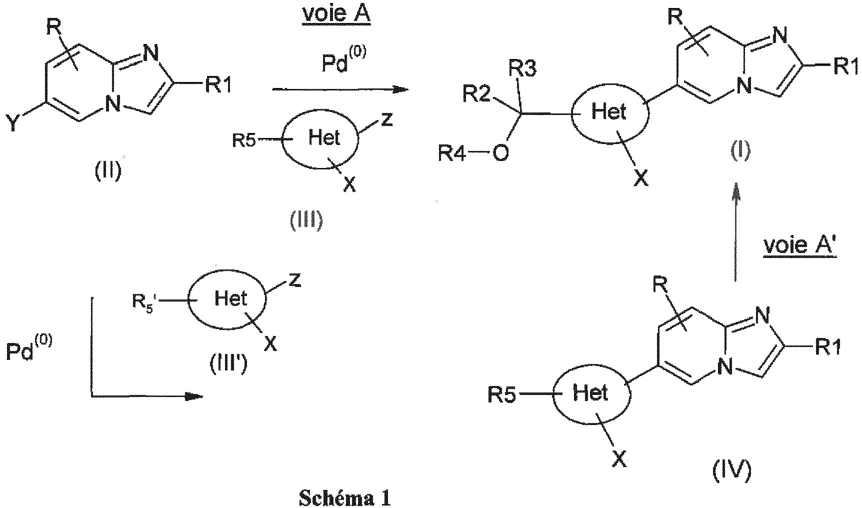

On peut préparer les composés de l'invention suivant le schéma 1 (voie A) par une réaction de couplage, catalysée par un métal tel que le palladium, entre une imidazopyridine de formule générale (II), dans laquelle R et R1 sont définis comme précédemment et Y représente un atome d'halogène ou un dérivé de bore, et un dérivé de formule générale (III) dans laquelle Het et X sont définis comme précédemment, Z représente un dérivé de bore ou d'étain si Y représente un atome d'halogène, ou bien un atome d'halogène si Y représente un dérivé de bore, et R5 représente le groupe 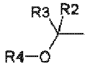 pour obtenir les composés de formule générale (I), par exemple, selon la méthode décrite par A. Gueiffier dans Helv. Chim. Acta 2001, 84, 3610-3615.

On peut aussi préparer les composés de l'invention suivant le schéma 1 (voie A') par une réaction de couplage, catalysée par un métal tel que le palladium, entre une imidazopyridine de formule générale (II), dans laquelle R et R1 sont définis comme précédemment et Y représente un atome d'halogène ou un dérivé de bore, et un dérivé de formule générale (III'), dans laquelle Het et X sont définis comme précédemment, Z représente un dérivé de bore ou d'étain si Y représente un atome d'halogène, ou bien un atome d'halogène si Y représente un dérivé de bore, et R5' représente un dérivé carbonylé R2CO dans lequel R2 est défini comme précédemment ou représente un groupe CO₂-alkyle pour obtenir les composés de formule générale (IV), par exemple, selon la méthode décrite par A. Gueiffier dans Helv. Chim. Acta 2001, 84, 3610-3615.

Ensuite, on peut transformer les composés de formule générale (IV) en composés de formule générale (I), dans lesquels R4 représente un atome d'hydrogène, par action d'un dérivé organométallique tel qu'un organomagnésien, par exemple R₃MgBr dans lequel R3 est défini comme précédemment ou par réduction du groupement carbonyle au moyen d'un hydrure métallique, par exemple le borohydrure de sodium ou un de ses dérivés, ou tout autre méthode connue de l'Homme du métier.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier. En particulier, les imidazopyridines de formule générale (II), dans laquelle Y représente un dérivé de bore, peuvent être obtenues, par exemple, selon la méthode décrite par E. DiMauro dans J. Org. Chem. 2006, 71, 3959*.*

Conformément à l'invention, on peut également préparer les composés de formule générale (I) selon le procédé décrit dans le schéma 2.

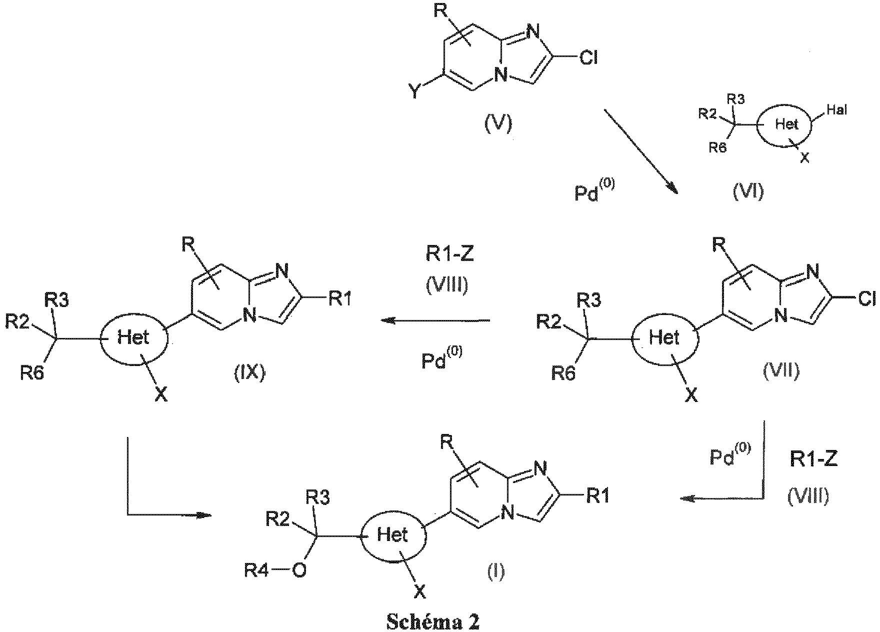

On peut préparer les composés de l'invention suivant le schéma 2 par une réaction de couplage, catalysée par un métal tel que le palladium, entre une imidazopyridine de formule générale (VII), dans laquelle Het, R, X, R2 et R3 sont définis comme précédemment et R6 représente le groupe OR4 et un dérivé de formule générale (VIII), dans laquelle R1 est défini comme précédemment et Z représente un dérivé de bore ou d'étain, pour obtenir les composés de formule générale (I), par exemple, selon la méthode décrite par S. Buchwald dans J.A.C.S. 2005, 127, 4685.

On peut aussi préparer les composés de l'invention suivant le schéma 2 par une réaction de couplage, catalysée par un métal tel que le palladium, entre une imidazopyridine de formule générale (VII), dans laquelle Het, R, X, R2 et R3 sont définis comme précédemment et R6 représente OPG dans lequel PG représente un groupement protecteur d'hydroxyle, tel que décrit par exemple par T. Greene dans « Protective Groups in Organic Synthesis » (Wiley Interscience*),* et un dérivé de formule générale (VIII) dans laquelle R1 est défini comme précédemment et Z représente un dérivé de bore ou d'étain, pour obtenir les composés de formule générale (IX), par exemple, selon la méthode décrite par S. Buchwald dans J.A.C.S. 2005, 127, 4685. Ensuite, on peut transformer les composés de formule générale (IX) en composés de formule générale (I), dans laquelle R4 représente un atome d'hydrogène, en réalisant une réaction de déprotection telle que décrite par exemple par T. Greene dans « Protective Groups in Organic Synthesis » (Wiley Interscience*),* ou par toute autre méthode connue de l'Homme du métier.

Les imidazopyridines de formule générale (VII) peuvent être obtenues par une réaction de couplage, catalysée par un métal tel que le palladium, entre une imidazopyridine de formule générale (V), dans laquelle R est défini tel que précédemment, Y représente un dérivé de bore, et un dérivé de formule générale (VI) dans laquelle Het, R2, R3 et X sont définis comme précédemment, R6 représente le groupement OR4 ou un groupement OPG dans lequel PG représente un groupement protecteur d'hydroxyle, et Hal représente un atome d'halogène différent du chlore, pour obtenir les composés de formule générale (VII), par exemple, selon la méthode décrite par A. Gueiffier dans Helv. Chim. Acta 2001, 84, 3610-3615.

Dans le schéma 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier. En particulier, les imidazopyridines chlorées de formule générale (V) peuvent être obtenues, par exemple, selon la méthode décrite par C. Townsend dans Syn. Commun. 1997, 27, 1763-1765.

Conformément à l'invention, on peut également préparer les composés de formule générale (I) dans laquelle R représente un atome d'hydrogène ou un groupe alkyle en position 3 du noyau imidazopyridine, selon le procédé décrit dans le schéma 3.

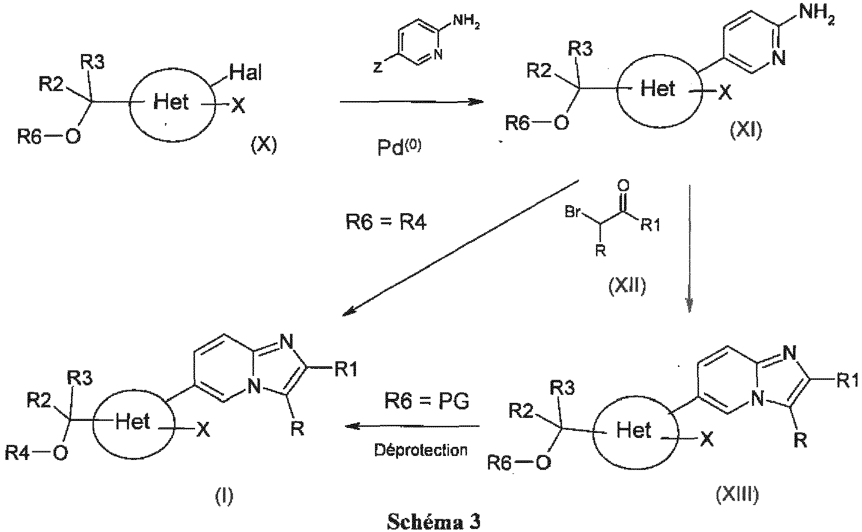

On peut préparer les composés de l'invention suivant le schéma 3 par une réaction de condensation entre une aminopyridine de formule générale (XI), dans laquelle R2, R3 et X sont définis comme précédemment, et R6 représente R4 ou un groupement protecteur de fonction hydroxyle PG, et une bromocétone de formule générale (XII), dans laquelle R1 est défini comme précédemment et R représente un atome d'hydrogène ou un groupe alkyle, pour obtenir, soit une imidazopyridine de formule générale (I), dans laquelle R, R1, R2, R3, R4 et X sont définis comme précédemment, soit une imidazopyridine de formule générale (XIII), dans laquelle R6 représente PG, par exemple selon la méthode décrite par M. Fisher dans J. Med. Chem 1972, 15, 982. On peut citer comme groupement protecteur de fonction hydroxyle, ceux décrits par exemple par T. Greene dans « Protective Groups in Organic Synthesis » (Wiley Interscience*),* par exemple un groupe terbutyldiméthylsilyle.

Par ailleurs, lorsque R6 représente un groupement protecteur de fonction hydroxyle PG, les composés de formule générale (XIII) sont soumis à une réaction de déprotection, comme décrit par exemple par T. Greene dans « Protective Groups in Organic Synthesis » (Wiley Interscience*),* pour obtenir les composés de formule générale (I), dans laquelle R est en position 3 du noyau imidazopyridine et R4 représente l'atome d'hydrogène.

Les composés de formule générale (XI) peuvent être obtenus par une réaction de couplage, catalysée par un métal tel que le palladium, entre un dérivé halogéné de formule générale (X) dans laquelle R2, R3, X et Hal sont définis comme précédemment, et R6 représente R4 ou un groupement protecteur de fonction hydroxyle PG et une 2-aminopyridine substituée par un groupement Z qui représente un dérivé de bore ou d'étain telle que, par exemple, la 5-(4,4,5,5-tétraméthyl-1,3,2-dioxaborolan-2-yl)pyridin-2-ylamine.

D'une façon générale, les intermédiaires ci-dessus peuvent être soumis, si désiré et si nécessaire, à toutes réactions de protection / déprotection connues de l'Homme de l'art avant et / ou après toutes réactions décrites dans les schémas ci-dessus.

Les produits de formule (I), peuvent être soumis, si désiré et si nécessaire, à toutes réactions connues de l'homme de l'art, dans un ordre quelconque, pour être transformés en d'autres produits de formule (I),

A titre d'exemples de réactions, on peut citer : les réactions d'estérification ou d'amidification de fonction acide, les réactions de carbamoylation, les réactions d'hydrolyse de fonction ester, les réactions de transformation de fonction hydroxyle en fonction alcoxyle, les réactions de couplage catalysées par un métal de transition, les réactions de protection des fonctions réactives, les réaction d'élimination des groupements protecteurs que peuvent porter les fonctions réactives protégées, les réactions de salification par un acide minéral ou organique ou par une base pour obtenir le sel correspondant, les réactions de dédoublement des formes racémiques en énantiomères, lesdits produits de formule (I) ainsi obtenus étant le cas échéant sous toutes les formes isomères possibles racémiques, énantiomères et diastéréoisomères.

L'invention, selon un autre de ses aspects, a également pour objet les composés de formules (II-1), (II-2), (II-3), (II-4), (II-5), (II-6), (II-7), (V-I), (VII-1), (VII-2), (VII-3), (VII-4), (X-1), (X-2) et (X-3)et (X4). Ces composés sont utiles comme intermédiaires de synthèse des composés de formule (I).

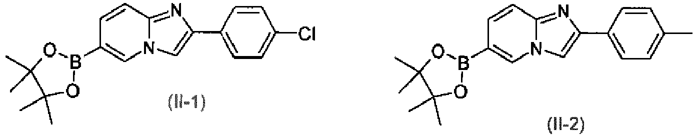

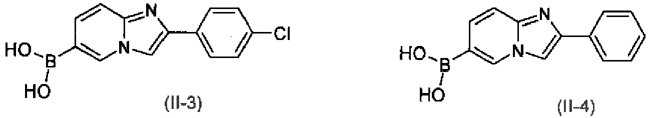

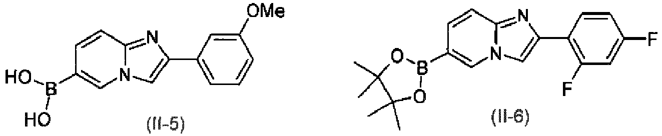

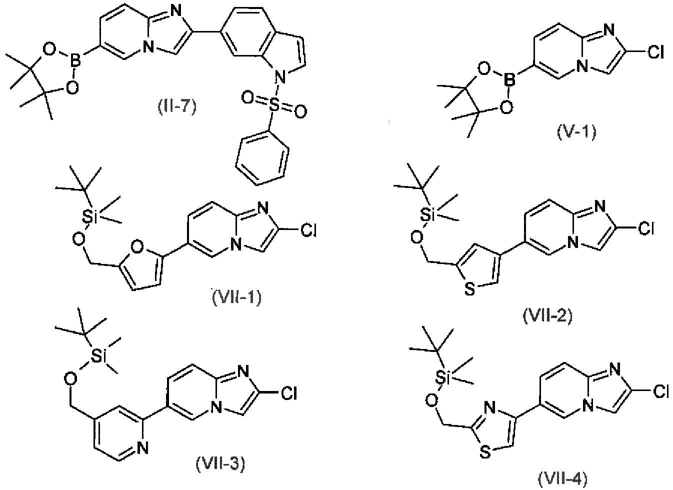

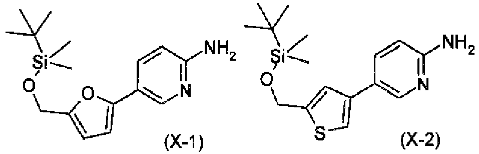

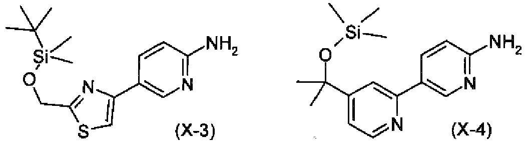

Les composés de formules (II-1), (II-2), (II-3), (II-4), (II-5), (II-6) et (II-7) peuvent être préparés en une étape (esters boroniques) ou deux étapes (acides boroniques), par exemple selon le procédé décrit dans les exemples N°2 et N° 6. Dans une première étape, on peut effectuer une condensation entre une aminopyridine substitué par un dérivé de bore telle que, par exemple, une 5-(4,4,5,5-tétraméthyl-1,3,2-dioxaborolan-2-yl)pyridin-2-ylamine et une alphabromocétone telle qu'une 2-bromo-1-(aryl)éthanone, par exemple dans un solvant tel que le n-propanol en présence d'une base telle que , par exemple, l'hydrogénocarbonate de sodium pour obtenir les esters boronates correspondants. Ensuite, dans une deuxième étape, on hydrolyse les esters boroniques en acides boroniques correspondants, par exemple dans un mélange d'acétone, d'eau et d'acide chlorhydrique.

Le composé de formule (V-1) peut être préparé, par exemple, selon le procédé décrit dans l'exemple N° 3. Dans une première étape, on peut effectuer une condensation entre une aminopyridine subsituée par un dérivé de bore telle que, par exemple, la 5-(4,4,5,5-tétraméthyl-1,3,2-dioxaborolan-2-yl)pyridin-2-ylamine et le 2-bromoacétate d'éthyle. Dans une deuxième étape, le composé est soumis à une réaction de cyclisation et de chloration en présence d'un agent chlorant tel que l'oxychlorure de phosphore qui conduit au composé (V-1).

Les composés de formule (VII-1), (VII-2), (VII-3) et (VII-4) peuvent être préparés par une réaction de couplage, catalysée par un métal, tel que le palladium, entre par exemple, le composé (V-1) et un hétérocycle halogéné, par exemple bromé, comportant une fonction alcool protégée, par exemple par un groupement tertbutyldiméthylsilanyle tel que décrit dans les exemples N° 3 et 4.

Les composés de formule (X-1), (X-2), (X-3) et (X-4) peuvent être préparés par condensation entre une aminopyridine substituée par un dérivé de bore telle que, par exemple, la 5-(4,4,5,5-tétraméthyl-1,3,2-dioxaborolan-2-yl)pyridin-2-ylamine et un hétérocycle halogéné, par exemple bromé, comportant une fonction alcool protégée, par exemple par un groupement tertbutyldiméthylsilanyle ou triméthylsilanyle, par exemple selon le procédé décrit dans les exemples N°5, 6 et 7.

Les composés de formules (II-1), (II-2), (II-3), (II-4), (II-5), (II-6), (V-1), (VII-1), (VII-2), (VII-3), (VII-4), (X-1), (X-2), (X-3) et (X-4) ont été préparés à l'état de poudre ou d'huile, à l'état de base ou de sel d'addition à un acide. Le tableau 1 rassemble quelques données physicochimiques de ces intermédiaires.

Dans ce tableau, la colonne « Sel/base », « - » représente un composé sous forme de base libre, alors que « HCl » représente un composé sous forme de chlorhydrate et le rapport entre parenthèses est le rapport (acide :base).

**Tableau 1**

| N° | R.M.N. ¹H (DMSO-d6, δ ppm) ; M+H ; PF | Sel / base |
|---|---|---|
| (II-1) | 1,35 (s, 12H) ; 7,35 (d, 1H) ; de 7,5 à 7,6 (m, 3H) ; 7,95 (d, 2H) ; 8,45 (d, 1H) ; 7,85 (s, 1H). M+H = 355. | - |
| (II-2) | 1,45 (s, 12H) ; 2,45 (s, 3H) ; 7,3 (d, 2H) ; de 7,5 à 7,7 (m, 2H) ; de 7,85 à 8 (m, 3H) ; 8,6 (s, 1H) ; M+H = 335 | - |
| (II-3) | De 7,6 à 7,75 (m, 2H) ; 7,95 (m, 1H) ; de 8,05 à 8,15 (m, 2H) ; 8,2 (m, 1H) ; 8,9 (s, 1H) ; 9,1 (s, 1H). M+H = 273. | HCl (1:1) |
| (II-4) | 7,55 (m, 1H) ; 7,6 (m, 2H) ; 8,0 (m, 1H) ; 8,1 (m, 2H) ; 8,25 (d, 1H) ; 8,9 (s, 1H) ; 9,1 (s, 1H). M+H = 275 | HCl (1:1) |
| (II-5) | 3,75 (s, 3H) ; 6,95 (d, 1H) ; de 7,3 à 7,65 (m, 3H) ; 7,8 (d, 1H) ; 8,05 (d, 1H) ; 8,75 (s, 1H) ; 8,9 (s, 1H). M+H = 325 | HCl (1:1) |
| (II-6) | 1,35 (s, 12H) ; 7,25 (t, 1H) ; de 7,35 à 7,45 (m, 2H) ; 7,6 (d, 1H) ; de 8,25 à 8,35 (m, 1H) ; 8,45 (s, 1H).; 8,9 (s, 1H) M+ =356. | - |
| (II-7) | 1,4 (s, 12H) ; 6,95 (d, 1H) ; de 7,55 à 7,85 (m, 7H) ; 7,95 (d, 1H) ; 8,05 (m, 2H) ; 8,55 (s, 1H) ; 8,75 (s, 1H) ; 9,05 (s, 1H). | HBr(1:1) |
| (V-1) | 1,35 (m, 12H) ; 7,4 (d, 1H) ; 7,5 (d, 1H) ; 8,1 (s, 1H) ; 8,85 (s, 1H) ; M+H = 279 : PF = 115 - 120°C | - |
| (VII-1) | 0,0 (s, 6H) ; 0,8 (s, 9H) ; 4,6 (s, 2H) ; 6,2 (d, 1H) ; 6,45 (d, 1H) ; 7,3 (d, 1H) ; de 7,4 à 7,45 (m,. 2H) ; 8,25 (s, 1H | - |
| (VII-2) | 0,0 (s, 6H) ; 0,8 (s, 9H) ; 4,8 (s, 2H); 7,3 (s, 1H) ; 7,45 (d, 1H) ; 7,6 (d, 1H) ; 7,75 (s, 1H) ; 7,9 (s, 1H) ; 8,8 (s, 1H). | - |
| (VII-3) | (CDCl₃, δ en ppm) : 0 (s, 6H) ; 0,85 (s, 9H) ; 4,65 (s, 2H) ; 7,1 (m, 1H) ; 7,45 à 7,55 (m, 3H) ; 7,65 (dd, 1H) ; 8,45 (d, 1H) ; 8,7 (s, 1H). M+H = 374 ; PF = 111 - 114°C | - |
| (VII-4) | 0,0 (s, 6H) ; 0,8 (s, 9H) ; 4,85 (s, 2H) ; 7,45 (d, 1H) ; 7,75 (d, 1H) ; 8,0 (m, 2H) ; 8,95 (s, 1H). | - |
| (X-1) | 0,0 (s, 6H) ; 0,8 (s, 9H) ; 4,55 (s, 2H); 6,05 (s, 2H) ; 6,3 (s,1H) ; 6,4 (d, 1H) ; 6,5 (s, 1H) ; 7,55 (d, 1H) ; 8,2 (s, 1H).M+H = 305 | - |
| (X-2) | 0,0 (s, 6H) ; 0,8 (s, 9H) ; 4,85 (s, 2H); 5,85 (s, 2H) ; 6,4 (d, 1H) ; 7,2 (s, 1H) ; 7,4 (s, 1H) ; 7,55 (d, 1H) ; 8,15 (s, 1H). | - |
| (X-3) | 0,0 (s, 6H) ; 0,8 (s, 9H) ; 4,85 (s, 2H); 5,95 (s, 2H) ; 6,35 (d, 1H) ; 7,6 (s, 1H); 7,75 (d, 1H); 8,35 (s, 1H). | - |
| (X-4) | (CDCl3, δ en ppm) : 0 (s, 9H) ; 1,4 (s , 6H) ; 4,4 (s, 2H) ; 6,45 (d, 1H) ; 7,05 (m, 1H) ; 7,55 (s, 1H) ; 7,95 (m, 1H) ; 8,4 (d, 1H) ; 8,5 (m; 1H). M+H = 302 | - |

Les exemples suivants décrivent la préparation de certains composés conformes à l'invention. Ces exemples ne sont pas limitatifs et ne font qu'illustrer la présente invention.

La nomenclature des composés a été établie à partir du logiciel Autonom.

### Exemple 1 : {5-[2-(4-Chlorophényl)imidazo[1,2-a]pyridin-6-yl]furan-2-yl}méthanol (composé 1 du tableau)

### 1.1 2-(4-Chlorophényl)-6-iodoimidazo[1,2-a]pyridine

On place dans un ballon 5,0 g de 2-amino-5-iodopyridine, 5,3 g de 2-bromo-1-(4-chlorophényl)éthanone et 2,67 g d'hydrogénocarbonate de sodium dans 150 ml de n-propanol. On chauffe à 80°C pendant 40h, laisse refroidir et ajoute 700 ml d'eau. Le précipité est recueilli par filtration, on le lave à l'eau et le sèche sous pression réduite. On obtient 5,51 g de composé.

RMN 1H (DMSO-d6, δ en ppm) : 7,45 (s, 2H) ; 7,55 (d, 2H) ; 8 (d, 2H) ; 8,35 (s, 1H) ; 8,95 (s, 1H). M+H = 355.

### 1.2 5-[2-(4-Chlorophényl)imidazo[1,2-a]pyridin-6-yl]furan-2-carbaldéhyde

300 mg de 2-(4-chlorophényl)-6-iodoimidazo[1,2-*a*]pyridine, 118 mg d'acide 5-formyl-2-furaneboronique, 89 mg de [1,1'-bis(diphénylphosphino)ferrocène] dichloropalladium, 280 mg de carbonate de potassium, 1,5 ml d'éthanol et 1 ml d'eau sont placés dans un tube à micro-ondes et dégazés à l'argon. Le tube est placé dans un appareil à micro-ondes et irradié à 90°C pendant 30 min. Après refroidissement, on élimine par filtration le catalyseur que l'on lave à l'acétate d'éthyle. La phase organique est séparée, séchée et le filtrat est concentré sous pression réduite. Le résidu est purifié par chromatographie sur gel de silice en éluant par un mélange dichlorométhane/methanol. On obtient 104 mg de composé.
RMN 1H (DMSO-d6, δ en ppm) : 7,30 (d, 1H) ; 7,54 (m, 2H) ; 7,71 ( d, 1H) ; de 7,74 à 7,79 (m, 2H) ; 8,01 (m, 2H) ; 8,55 (s, 1H) ; 9,16 (m, 1H) ; 9,65 (s, 1H). M+H = 323.

### 1.3 {5-[2-(4-Chlorophényl)imidazo[1,2-a]pyridin-6-yl]furan-2-yl}méthanol

A 104 mg de 5-[2-(4-chlorophényl)imidazo[1,2-*a*]pyridin-6-yl]furane-2-carbaldéhyde dissous dans 20 ml de méthanol, on ajoute par petites portions 122 mg de borohydrure de sodium. Le mélange est ensuite agité à température ambiante pendant 2 heures, puis le solvant est évaporé sous pression réduite. Le résidu est repris entre le dichlorométhane et l'eau. La phase organique est séparée, séchée sur sulfate de sodium et concentrée sous pression réduite. On obtient 39 mg de composé.
PF = 197 - 199°C. RMN 1H (DMSO-d6, δ en ppm) : 4,5 (d, 2H) ; 5,3 (t, 1H) ; 6,5 (m, 1H) ; 7,95 (m, 1H) ; 7,55 (m, 2H) ; de 7,60 à 7,70 (m, 2H) ; 8 (m, 2H) ; 8,5 (s, 1H) ; 8,85 (s, 1H). M+H = 325

### Exemple 2: {4-[2-(4-Chlorophényl)imidazo[1,2-a]pyridin-6-yl]thiazol-2-yl}méthanol (composé 11 du tableau)

### 2.1 2-(4-Chlorophényl)-6-(4,4,5,5-tétraméthyl-1,3,2-dioxaborolan-2-yl)imidazo[1,2-a]pyridine

On place dans un ballon 2,5 g de 5(4,4,5,5-tétraméthyl-1,3,2-dioxaborolan-2-yl)pyridin-2-ylamine, 2,65 g de 2-bromo-1-(4-chlorophényl)éthanone dans 76 ml de n-propanol. On ajoute 1,33 g d'hydrogénocarbonate de sodium. On chauffe à 80°C pendant 16h. On laisse refroidir et concentre le mélange réactionnel sous pression réduite. Le résidu est repris entre l'eau et l'acétate d'éthyle, la phase organique est ensuite décantée, séchée sur sulfate de magnésium, puis le solvant est évaporé sous pression réduite. On obtient 3,75g de composé.
RMN 1H (DMSO-d6, δ en ppm) : 1,35 (s, 12H) ; 7,35 (d, 1H) ; de 7,5 à 7,6 (m, 3H) ; 7,95 (d, 2H) ; 8,45 (d, 1H) ; 7,85 (s, 1H). M+H = 355.

### 2.2 Chlorhydrate (1:1) d'acide [2-(4-chlorophényl)imidazo[1,2-a]pyridin-6-yl]boronique

3,60 g de 2-(4-chlorophényl)-6-(4,4,5,5-tetraméthyl-1,3,2-dioxaborolan-2-yl)imidazo[1,2-*a*]pyridine sont dissous dans 112 ml d'acétone et 56 ml d'eau ; on y ajoute, goutte à goutte et sous agitation, 101 ml d'acide chlorhydrique 1N et agite à température ambiante pendant 24h. Le mélange réactionnel est ensuite concentré sous pression réduite. Le solide obtenu est trituré à l'éther diéthylique et recueilli par filtration, puis séché à l'étuve sous pression réduite à 60°C. On obtient 3,12 g de composé.
RMN 1H (DMSO-d6, δ en ppm) : de 7,6 à 7,75 (m, 2H) ; 7,95 (m, 1H) ; de 8,05 à 8,15 (m, 2H) ; 8,2 (m, 1H) ; 8,9 (s, 1H) ; 9,1 (s, 1H). M+H = 273.

### 2.3 {4-(2-(4-Chlorophényl)imidazo[1,2-a]pyridin-6-yl]thiazol-2-yl}méthanol

On place dans un ballon 28 ml de diméthoxyéthane et 14 ml d'une solution 2M de carbonate de sodium, on dégaze à l'argon pendant 10 min. On ajoute 768 mg d'acide [2-(4-chlorophényl)imidazo[1,2-*a*]pyridin-6-yl]boronique, 482 mg de (4-bromothiazol-2-yl)méthanol et 144 mg de tétrakis(triphénylphosphine)palladium. On laisse le mélange agiter 16 h à 80°C. On évapore ensuite le solvant sous pression réduite. Le résidu obtenu est repris à l'acétate d'éthyle. II se forme un précipité que l'on recueille par filtration et lave à l'acétate d'éthyle. Le composé est ensuite purifié par chromatographie sur gel de silice, en éluant avec un mélange dichlorométhane/méthanol. Le solide obtenu est trituré à l'éther diéthylique et recueilli par filtration, puis séché à l'étuve sous pression réduite à 50°C. On obtient 350 mg de composé.
PF = 190-192 °C. RMN 1H (DMSO-d6, δ en ppm) :4,85 (s, 2H) ; 6,2 (t, 1H), de 7,50 à 7,60 (m, 2H) ; 7,7 (d, 1H) ; 7,85 (d, 1H) ; de 7,95 à 8,05 (m, 2H) ; 8, 1 (s, 1H) ; 8,5 (s, 1H) ; 9,1 (s, 1H). M+H = 342.

### Exemple 3 : = Chlorhydrate (2:1) de [4-[2-quinolin-3-yl)imidazo[1,2-a]pyridin-6-yl]thién-2-yl]méthanol (composé 16 du tableau)

### 3.1 [4-(Bromothién-2-yl)méthyloxy]tert-butyldiméthylsilane

900 mg de (4-bromothién-2-yl)méthanol sont placés dans un ballon et dissous dans 45 ml de tétrahydrofurane. On y ajoute 440 mg d'1*H-*imidazole puis 910 mg de *tert-*butyl(chloro)diméthylsilane et on laisse agiter à température ambiante pendant 48 heures. Le mélange réactionnel est ensuite hydrolyse avec de l'eau et la phase organique, extraite à l'acétate d'éthyle, est séparée, séchée sur sulfate de magnésium et concentrée sous pression réduite. Le résidu obtenu est purifié par chromatographie sur gel de silice en éluant avec un mélange heptane/acétate d'éthyle. On obtient 1,2 g de composé.
Spectre RMN 1H (CDCl3, δ en ppm) : 0 (s, 6H) ; 0,85 (s, 9H) ; 4,7 (s, 2H) ; 6,7 (s, 1H) ; 7,0 (s, 1H). M+H = 308.

### 3.2 Bromhydrate de [2-imino-5-(4,4,5,5-tétraméthyl-1,3,2-dioxaborolan-2-yl)-2H-pyridin-1-yl]acétate d'éthyle

5,0 g de 5-(4,4,5,5-tétraméthyl-1,3,2-dioxaborolan-2-yl)pyridin-2-ylamine dans 7,6 ml de 2-bromoacétate d'éthyle sont placés dans un ballon et le mélange est agité à température ambiante pendant 20 h. Il se forme un précipité que l'on recueille par filtration, lave par de l'éther diéthylique puis par de l'éthanol et sèche à l'étuve sous pression réduite. On obtient 8,78 g de composé.
Spectre RMN 1H (DMSO-d6, δ en ppm) : 1,3 (m, 15H) ; de 4,1 à 4,25 (m, 2H) ; 5,2 (s, 2H) ; 7,1 (d, 1H) ; 8,0 (d, 1H) ; 8,3 (s, 1H) ; 9,0 (s, 1H). M+H = 388.

### 3.3 2-Chloro-6-(4,4,5,5-tétraméthyl-1,3,2-dioxaboroian-2-yl)imidazo[1,2-a]pyridine

Dans un ballon on place 8,78 g de bromhydrate de [2-imino-5-(4,4,5,5-tétraméthyl-1,3,2-dioxaborolan-2-yl)-1*H*-pyridin-1-yl]acétate d'éthyle dans 20 ml de POCl₃. Le mélange réactionnel est chauffé à 105°C pendant 16h, refroidi à température ambiante et concentré sous pression réduite. Le résidu est repris entre le dichlorométhane et l'eau à 0°C et on ajoute une solution aqueuse à 30% de NH₄OH jusqu'à pH basique. La phase organique est séparée, séchée sur sulfate de magnésium et concentrée sous pression réduite.On obtient 4,3 g de composé.
PF = 115 - 120°C. Spectre RMN 1H (DMSO-d6, δ en ppm) : 1,35 (m, 12H) ; 7,4 (d, 1H) ; 7,5 (d, 1H) ; 8,1 (s, 1H) ; 8,85 (s, 1H). M+H = 279.

### 3.4 6-[5-[(tert-Butyldiméthylsilanyl)oxyméthyl]thién-3-yl]-2-chloroimidazo[1,2-a]pyridine

1,0 g de [(4-bromothién-2-yl)méthyloxy]*tert*-butyldiméthylsilane dans 16 ml de tétrahydrofurane et 4 ml d'eau sont placés dans un ballon et dégazés sous courant d'argon pendant 10 mn. On y ajoute 1,0 g de 2-chloro-6-(4,4,5,5-tétraméthyl-1,3,2-dioxaborolan-2-yl)imidazo[1,2-*a*]pyridine, 210 mg de [1,1'-bis(diphénylphosphino)ferrocène]dichloropalladium et 3,2 g de carbonate de césium et on chauffe à 80°C pendant 16 heures. Après refroidissement, le mélange réactionnel est concentré sous pression réduite. Le résidu obtenu est purifié par chromatographie sur gel de silice en éluant avec un mélange heptane/acétate d'éthyle. On obtient 450 mg de composé.
PF = 100 - 104°C. Spectre RMN 1H (CDCl₃, δ en ppm) : 0 (s, 6H) ; 0,8 (s, 9H) ; 4,8 (s, 2H) ; 7,3 (s, 1H) ; 7,45 (d, 1H) ; 7,6 (d, 1H) ; 7,75 (s, 1H) ; 7,9 (s, 1H) ; 8,75 (s, 1H). M+H = 379.

### 3.5 3-{6-[S-((tert-Butyldiméthylsilanyl)oxyméthyl)thién-3-yl]imidazo[1,2-a]pyridin-2-yl}quinoléine

Dans un réacteur, on place 150 mg de 6-[5-[(*tert-*butyldiméthylsilanyl)oxyméthyl]thién-3-yl]-2-chloroimidazo[1,2-*a*]pyridine dans 2 ml de toluène et on dégaze à l'argon pendant 10 mn. Puis on ajoute 3 mg d'acétate de palladium, 11 mg de 2-dicyclohexylphosphino-2',6'-diméthoxy-1,1'-biphényl, 170 mg de K₃PO₄, 110 mg d'acide 3-quinoléineboronique et quelques gouttes d'éthanol. Le mélange réactionnel est chauffé à 115°C pendant 16h, refroidi à température ambiante et concentré sous pression réduite. Le résidu est ensuite purifié par chromatographie sur gel de silice en éluant avec un mélange heptane/acétate d'éthyle. On obtient 200 mg de composé.
M+H = 472.

### 3.6 [4-(2-Quinoléin-3-yl)imidazo[1,2-a]pyridin-6-yl]thién-2-yl]méthanol

On place dans un ballon, 190 mg de 3-{6-[5-[(*tert-*butyldiméthylsilanyl)oxyméthyl]thién-3-yl]imidazo[1,2-*a*]pyridin-2-yl}quinoline dans 4 ml de tétrahydrofurane et on ajoute 210 mg de fluorure de tétrabutylammonium. Après 48 heures d'agitation à température ambiante, le mélange réactionnel est concentré sous pression réduite. Le résidu est ensuite purifié par chromatographie sur gel de silice en éluant avec un mélange dichlorométhane/méthanol. On obtient 100 mg de composé.
Spectre RMN 1H (DMSO-d6, δ en ppm) : 4,7 (d, 2H) ; 5,55 (t, 1H) ; 7,5 (s, 1H) ; de7,65 à 7,8 (m, 4H) ; de 7,9 (s, 1H) ; 8,05 à 8,15 (m, 2H) ; 8,65 (s, 1H) ; 8,9 (s, 1H) ; 9,0 (s, 1H) ; 9,55 (1H). M+H = 358.

### 3.7 Chlorhydrate (2:1) de [4-[(2-quinolin-3-yl)imidazol[1,2-a]pyridin-6-yl]thién-2-yl]méthanol

100 mg de [4-[(2-quinoléin-3-yl)imidazo[1,2-*a*]pyridin-6-yl]thién-2-yl]méthanol sont mis en suspension dans du dichlorométhane et du méthanol. La solution est passée sur fritté et on ajoute au filtrat 5,6 mil d'une solution 0.1N d'acide chlorhydrique dans l'isopropanol. Le mélange réactionnel est ensuite concentré sous pression réduite puis le résidu est repris dans l'éther diéthylique. Le précipité est recueilli par filtration et séché à l'étuve sous pression réduite. On obtient 106 mg de composé.
PF = 310 - 315°C. Spectre RMN 1H (DMSO-d6, δ en ppm) : 4,75 (s, 2H) ; 7,55 (s, 1H); 7,8 (t, 1H) ; de 7,9 à 8,0 (m, 2H) ; 8,05 (s, 1H) ; de 8,17 à 8,25 (m, 3H) ; 8,9 (s, 1H) ; 9,28 (s, 1H) ; 9,32 (s, 1H) ; 9,67 (s, 1H). M+H = 431.

### Exemple 4 : {5-[2-(1H-Indol-6-yl)imidazo[1,2-a]pyridin-6-yl]furan-2-yl}méthanol (composé 32 du tableau)

### 4.1 (5-Bromofuran-2-yl)méthanol

Dans un ballon de 11, on dissout 10 g (0,052 mole) d'acide 5-bromofuroïque dans 350 ml de THF, ajoute 5,8 g (0,057 mole) de *N*-méthylmorpholine et refroidit le mélange par un bain de glace. On ajoute par une ampoule à brome 7,5 g (0,055 mole) de chloroformiate d'isobutyle en solution dans 50 ml de THF. Lorsque l'addition est terminée, on laisse agiter une heure à froid puis élimine le solide formé par filtration sur célite. Le filtrat est refroidi au bain de glace, puis on ajoute 4,95 g de borohydrure de sodium, et enfin, très lentement, 50 ml de méthanol. On laisse agiter une heure à froid puis évapore les solvants sous pression réduite. Le résidu est repris entre l'eau et l'éther diéthylique, la phase organique est décantée, lavée à l'eau et séchée sur sulfate de sodium. Après évaporation du solvant, on obtient 9,1 g d'huile que l'on utilise sans purification pour l'étape suivante.

### 4.2 [(5-Bromofuran-2-yl)méthyloxy]tert-butyldiméthylsilane

9,1 g (0,051 mole) du composé obtenu en 4.1 sont dissous dans 150 ml de THF. On y ajoute 5,2 g (0,077 mole) d'imidazole puis 10 g de chloro-*tert*-butyldiméthylsilane et laisse agiter 16 heures à température ambiante. Puis on évapore le solvant sous pression réduite, reprend entre l'eau et l'éther diéthylique, décante, lave la phase organique à l'eau et sèche sur sulfate de sodium. Après évaporation du solvant, on purifie le produit par chromatographie en éluant par un mélange heptane/acétate d'éthyle. On obtient 10,1 g de composé.
RMN 1H (CDCl3, δ en ppm) : 0,0 (s, 6H) ; 0,8 (s, 9H) ; 4,45 (s, 2H) ; 6,2 (s, 1H) ; 7,2 (s, 1H)

### 4.3 6-[5-(tert-Butyldiméthylsilanyloxyméthyl)furan-2-yl]-2-chloroimidazo[1,2-a]pyridine

Dans un ballon de 150 ml, on fait dégazer pendant 15 minutes sous argon 68 ml de THF et 12 ml d'eau, puis on ajoute 4,56 g (15,7 mmoles) du composé obtenu en 4.2, 4,36 g (15,7 mmoles) du composé obtenu en 3.3, 15,3 g (47 mmoles) de carbonate de césium et 0,38 g de [1,1'-bis(diphénylphosphino)ferrocène]dichloropalladium et chauffe le mélange dans un bain thermostaté à 80°C pendant 1h30. Puis on évapore les solvants sous pression réduite, reprend le résidu entre l'eau et l'éther diéthylique, décante et sèche sur sulfate de sodium. Après évaporation du solvant, on purifie le produit par chromatographie en éluant par un mélange dichlorométhane/méthanol, puis recristallisation dans le cyclohexane. On obtient 4,2 g de composé.
RMN 1H (DMSO-d6, δ en ppm) : 0,0 (s, 6H) ; 0,8 (s, 9H) ; 4,6 (s, 2H) ; 6,2 (d, 1H) ; 6,45 (d, 1 H) ; 7,3 (d, 1H) ; de 7,4 à 7,45 (m, 2H) ; 8,25 (s, 1H).

### 4.4 6-[5-(tert-Butyldiméthylsilanyloxyméthyl)furan-2-yl]-2-(1H-indol-6-yl)imidazo[1,2-a]pyridine

108 mg (0,3 mmole) du composé obtenu en 4.3 sont placés dans un tube, on ajoute 0,35 mmole de phosphate de potassium, 1 ml de n-butanol et 30 mg d'acide indole-6-boronique en solution dans 2 ml de toluène dégazé. Le tube est purgé par argon et on y rajoute une solution de 0,0036 mmole d'acétate de palladium et de 0,0072 mmole de 2-dicyclohexylphosphino-2',6'-diméthoxy-1,1'-biphényle dans 1 ml de toluène. Le tube est fermé et agité 16h à 110°C. La solution refroidie est diluée par 5 ml d'acétate d'éthyle, on y ajoute 100 mg de silice-propanethiol et le mélange est agité 4h à température ambiante. Le solide est séparé par filtration et lavé par de l'acétate d'éthyle. Le filtrat est évaporé à sec et utilisé tel quel pour l'étape suivante.

### 4.5 {5-[2-(1H-Indol-6-yl)imidazo[1,2-a]pyridin-6-yl]furan-2-yl}méthanol

Le composé obtenu en 4.4 est est dissous dans 5 ml de THF contenant 0,36 mmole de fluorure de tétrabutylammonium hydraté. Le mélange est agité 16h à température ambiante, puis le solvant est évaporé sous pression réduite. Le composé est purifié parchromatographie On obtient 3,9 mg de composé.
RMN 1H (DMSO-d6, δ en ppm) : 4,45 (m, 2H) ; 5,3 (m, 1H) ; 6,45 (m, 2H) ; 6,9 (s, 1H) ; 7,4 (m, 1H) ; 7,55 à 7,65 (m, 4H) ; 8 (s, 1H) ; 8,4 (s, 1H) ; 8,85 (s, 1H) ; 11,2 (s, 1H). M+H = 330.

### Exemple 5 : {4-[2-(2-Fluorophényl)imidazo[1,2-a]pyridin-6-yl]thiazol-2-yl}méthanol (composé 66 du tableau)

### 5.1 4-Bromo-2-(tert-butyldiméthylsilanyloxyméthyl)thiazole

En procédant comme dans l'exemple 4.2 et à partir de 5,0 g de (4-bromothiazol-2-yl)méthanol dissous dans 255 mil de tétrahydrofurane, de 2,28 g d' 1*H*-imidazole et 4,66 g de *tert*-butylchlorodiméthylsilane, on obtient 8,18 g de composé.
RMN 1H (DMSO-d6, δ en ppm) : 0,0 (s, 6H) ; 0,8 (s, 9H) ; 4,85 (s, 2H); 7,7 (s, 1H).

### 5.2 5-[2-(tert-Butyl-diméthylsilanyloxyméthyl)thiazol-4-yl]pyridin-2-ylamine

8,0 g du composé obtenu à l'étape 5.1, 6,56 g de 5-(4,4,5,5-tétraméthyl-1,3,2-dioxaborolan-2-yl)pyridin-2-ylamine, 49,7 ml d'une solution 2M de carbonate de sodium et 1,01 g de [1,1'-bis(diphénylphosphino)ferrocène]dichloropalladium sont dissous dans 310 ml de *N,N-*diméthylformamide et placés dans un ballon sous courant d'argon. Le mélange est chauffé 2h30 à 80°C. Après refroidissement à température ambiante, on ajoute 600ml d'acétate d'éthyle au milieu réactionnel que l'on filtre sur célite. La phase organique est ensuite séparée, lavée 3 fois par une solution de chlorure de sodium saturée, séchée sur sulfate de magnésium et concentrée sous pression réduite. Le résidu est purifié par chromatographie sur gel de silice en éluant par un mélange dichlorométhane/acétate d'éthyle. Le solide obtenu est trituré dans l'éther diisopropylique, recueilli par filtration puis séché à l'étuve sous pression réduite. On obtient 4,5 g de composé.
RMN 1H (DMSO-d6, δ en ppm) : 0,0 (s, 6H) ; 0,8 (s, 9H) ; 4,85 (s, 2H); 5,95 (s, 2H) ; 6,35 (d, 1H) ; 7,6 (s, 1H) ; 7,75 (d, 1H) ; 8,35 (s, 1H).

### 5.3 6-[2-(tert-Butyldiméthylsilanyloxyméthyl)thiazol-4-yl]-2-(2-fluorophényl)imidazo[1,2-a]pyridine

58,8 mg (0,7 mmole) de bicarbonate de sodium sont pesés dans un tube à microondes. On y ajoute 57 mg (0,25 mmole) du composé obtenu en 5.2 en solution dans 2 ml de propan-1-ol, puis 92 mg (0,375 mmole) de 2-bromo-1-(2-fluorophényl)éthanone en solution dans 1 ml de propan-1-ol Le tube est scellé puis agité pendant 16 heures à 80°C. Le mélange réactionnel est refroidi à température ambiante, on y ajoute 200 mg de propanethiol sur silice (Biotage Si-Thiol) et le mélange est agité 6h à température ambiante puis filtré. Le résidu est lavé par 2 fois 2 ml de propan-1-ol puis le filtrat est évaporé. Le composé brut est engagé tel quel dans l'étape suivante.

### 5.4 {4-[2-(2-Fluorophényl)imidazo[1,2-a]pyridin-6-yl]thiazol-2-yl}méthanol

Le composé brut obtenu en 5.3 est dissous dans 5 ml de THF contenant 0,5 mmole de fluorure de tétrabutylammonium hydraté. Le mélange est agité 16h à température ambiante, puis le solvant est évaporé sous pression réduite. Le composé est purifié par chromatographie On obtient 4,1 mg de composé.
RMN 1H (DMSO-d6, δ en ppm) : 4,82 (s, 2H) ; 6,19 (m, 1H) ; 7,31 (m, 2H) ; 7,38 (m, 1H) ; 7,67 (d, 1H) ; 7,84 (d, 1H) ; 8,08 (s, 1H) ; 8,38 (t, 1H) ; 8,45 (d, 1H) ;9,22 (s, 1H).

### Exemple 6 : 2-{2-[2-(1H-Indol-6-yl)imidazo[1,2-a]pyridin-6-yl]pyridin-4-yl}propan-2-ol (composé 152 du tableau)

### 6.1 N-Méthoxy-N-méthylindole-6-carboxamide

On place dans un ballon 5,0 g d'acide indole-6-carboxylique, 3,3 g de chlorhydrate de *N,O-*diméthylhydroxylamine , 11,9 g de 1-(3-diméthylaminopropyl)-3-éthylcarbodiimide et 10 ml de pyridine dans 150 ml de tétrahydrofurane. Le mélange est agité à température ambiante pendant 40h. On concentre, le résidu est repris dans 150 ml d'acétate d'éthyle et 50ml d'eau. La phase organique est lavée avec 50 ml d'une solution 1N d'hydroxyde de sodium, 50 ml d'une solution saturée en chlorure de sodium, séchée sur sulfate de magnésium puis concentrée sous pression réduite. On obtient 6,8 g de composé.
RMN 1H (CDC3, δ en ppm) : 3,3 (s, 3H) ; 3,5 (s, 3H) ; 6,45 (m, 1H) ; 7,25 (t, 1H) ; 7,4 (dd, 1H) ; 7,55 (d, 1H) ; 7,75 (s, 1H) ; 8,8 (s, 1H). M+H = 205.

### 6.2 N-Méthoxy-N-méthyl-1-(phénylsulfonyl)indole-6-carboxamide

On place dans un ballon 6,8 g de composé obtenu en 6.1 dans 100 ml de *N,N-*diméthylformamide à 0°C. On ajoute 1,45g de NaH par portions puis 6,52 g de chlorure de benzènesulfonyle. Le mélange est agité à température ambiante pendant 40h. On ajoute 150 ml d'eau puis le mélange est extrait avec 60 ml d'acétate d'éthyle. La phase organique est lavée avec 50 ml d'une solution saturée en chlorure de sodium, séchée sur sulfate de magnésium puis concentrée sous pression réduite. Le résidu est purifié par chromatographie sur gel de silice en éluant par un mélange heptane/acétate d'éthyle. On obtient 9,5 g de composé.
RMN 1H (CDCl3, δ en ppm) : 3,4 (s, 3H) ; 3,55 (s, 3H) ; 6,7 (d, 1H) ; 7,45 à 7,6 (m, 5H) ; 7,7 (d, 1H) ; 7,95 (m, 2H) ; 8,4 (s, 1H). M+H = 345.

### 6.3 6-acétyl-1-(phénylsulfonyl)indole

On place dans un ballon 9,2 g de composé obtenu en 6.2 dans 250 ml de tétrahydrofurane à 0°C et sous argon. On ajoute goutte à goutte 27 ml de bromure de méthylmagnésium (3M dans l'éther diéthylique) . Le mélange est agité une heure à 0°C et 20h à température ambiante. On refroidit à 0°C et on ajoute 150 ml d'eau et 50 ml d'une solution saturée en chlorure d'ammonium. Le mélange est extrait avec 60 ml d'acétate d'éthyle. La phase organique est lavée avec 40 ml d'une solution saturée en chlorure de sodium, séchée sur sulfate de magnésium puis concentrée sous pression réduite.. On obtient 7,3 g de composé.
RMN 1H (CDCl3, δ en ppm) : 2,7 (s, 3H) ; 6,75 (d, 1H) ; 7,45 à 7,65 (m, 4H) ; 7,8 (d, 1H) ; 7,95 (m, 3H) ; 8,65 (s, 1H). M+H = 300 ; PF = 160 -163 °C.

### 6.4 2-Bromo-1-[1-(phénylsulfonyl)indol-6-yl]éthanone

On place dans un ballon 3 g de bromure de cuivre dans 120 ml d'acétate d'éthyle et on chauffe à reflux. On ajoute 2 g de 6-*a*cétyl-1-(phénylsulfonyl)indole. Le mélange est agité 4 heures à reflux. On filtre sur papier puis on verse sur 150 ml d'une solution à 20% de thiosulfate de sodium. Le mélange est extrait avec 60 ml d'acétate d'éthyle. La phase organique est lavée avec 40 ml d'une solution saturée en chlorure de sodium, séchée sur sulfate de magnésium puis concentrée sous pression réduite.. On obtient 2,6 g de composé. RMN 1H(CDCl3, δ en ppm) : 4,45 (s, 2H) ; 6,65 (d, 1H) ; 7,35 à 7,55 (m, 4H) ; 7,7 (d, 1H) ; 7,9 (m, 3H) ; 8,6 (s, 1H). M+H = 378

### 6.5 Bromhydrate de 2-(1-Phénylsulfonyl-1H-indol-6-yl)-6-(4,4,5,5-tétraméthyl-[1,3,2]dioxaborolan-2-yl)imidazo[1,2-a]pyridine

1.6 g de 5-(4,4,5,5-tétraméthyl-1,3,2-dioxaborolan-2-yl)pyridin-2-ylamine et 2,7 g de 2-bromo-1-[1-(phénylsulfonyl)indol-6-yl]éthanone sont placés dans 70 ml d'éthanol et le mélange est chauffé à reflux pendant 20 h. Il se forme un précipité que l'on recueille par filtration, lave par de l'éther diéthylique et sèche à l'étuve sous pression réduite. On obtient 2,3 g de composé.
RMN 1H (DMSO-d6, δ en ppm) : 1,4 (s, 12H) ; 6,95 (d, 1H) ; de 7,55 à 7,85 (m, 7H) ; 7,95 (d, 1H) ; 8,05 (m, 2H) ; 8,55 (s, 1H) ; 8,75 (s, 1H) ; 9,05 (s, 1H).

### 6.6 2-Bromo-N-méthoxy-N-méthylisonicotinamide

On place dans un ballon 2,0 g d'acide 2-bromoisonicotinique, 1,1 g de chlorhydrate de *N,O-*diméthylhydroxylamine, 3,8 g de 1-(3-diméthylaminopropyl)-3-éthylcarbodiimide et 3,2 ml de pyridine dans 50 ml de tétrahydrofurane. Le mélange est agité à température ambiante pendant 40h. On concentre, le résidu est repris dans 50 ml d'acétate d'éthyle et 50ml d'eau. La phase organique est lavée avec 50 ml d'une solution 1N d'hydroxyde de sodium, 50 ml d'une solution saturée en chlorure de sodium, séchée sur sulfate de magnésium puis concentrée sous pression réduite. On obtient 2,0 g de composé.
RMN 1H (CDCl3, δ en ppm) : 3,3 (s, 3H) ; 3,5 (s, 3H) ; 7,4 (d, 1H) ; 7,65 (s, 1H) ; 8,4 (d, 1H). M+H = 245.

### 6.7 2-Bromo-4-acétylpyridine

On place dans un ballon 2,0 g de composé obtenu en 6.6 dans 80 ml de tétrahydrofurane à 0°C et sous argon. On ajoute goutte à goutte 8 ml de bromure de méthylmagnésium (3M dans l'éther éthylique) . Le mélange est agité une heure à 0°C et 20h à température ambiante. On refroidit à 0°C et on ajoute 80 ml d'eau et 40 ml d'une solution saturée en chlorure d'ammonium. Le mélange est extrait avec 40 ml d'acétate d'éthyle. La phase organique est lavée avec 40 ml d'une solution saturée en chlorure de sodium, séchée sur sulfate de magnésium puis concentrée sous pression réduite. On obtient 1,6 g de composé.
RMN 1H (CDC13, δ en ppm) : 2,55 (s, 3H) ; 7,6 (d, 1H) ; 7,85 (s, 1H) ; 8,5 (d, 1H). M+H = 200.

### 6.8 2-(2-Bromopyridin-4-yl)propan-2-ol

On place dans un ballon 1,1 g de composé obtenu en 6.7 dans 60 ml de tétrahydrofurane à 0°C et sous argon. On ajoute goutte à goutte 13 ml de bromure de méthylmagnésium (3M dans l'éther éthylique) . Le mélange est agité une heure à 0°C et 20h à température ambiante. On refroidit à 0°C et on ajoute 60 ml d'eau et 30 ml d'une solution saturée en chlorure d'ammonium. Le mélange est extrait avec 40 ml d'acétate d'éthyle. La phase organique est lavée avec 40 ml d'une solution saturée en chlorure de sodium, séchée sur sulfate de magnésium puis concentrée sous pression réduite.. On obtient 1,5 g de composé. RMN 1H (CDC13, δ en ppm) : 1,55 (s, 6H) ; 7,35 (d, 1H) ; 7,6 (s, 1H) ; 8,3 (d, 1H).

### 6.9 2-Bromo-4-[1-méthyl-1-(triméthylsilanyloxy)éthyl]pyridine

On place dans un ballon 1,5 g de 2-(2-Bromo-pyridin-4-yl)propan-2-ol obtenus en 6.8 dans 35 ml de dichlorométhane à 0°C. On ajoute 2,4 ml de triéthylamine et 1,9 ml de chlorure de triméthylsilane. Le mélange est agité une heure à 0°C et 20h à température ambiante. On ajoute 20 ml d'eau et le mélange est extrait avec 20 ml de dichlorométhane. La phase organique est lavée avec 20 ml d'une solution saturée en chlorure de sodium, séchée sur sulfate de magnésium puis concentrée sous pression réduite. Le résidu est purifié par chromatographie sur gel de silice en éluant par un mélange dichlorométhane/méthanol.. On obtient 0,7 g de composé. RMN 1H (CDCl3, δ en ppm) : 0 (s, 9H) ; 1,4 (s, 6H) ; 7,1 (d, 1H) ; 7,35 (s, 1H) ; 8,1 (d, 1H).

### 6.10 2-(1-Phénylsulfonyl-1H-indol-6-yl)-6-[4-[1-méthyl-1-(triméthylsilanyloxy)éthyl]pyridin-2-yl]imidazo[1,2-a]pyridine

0,2 g de composé obtenu en 6.9 dans 4 ml de tétrahydrofurane et 1 ml d'eau sont placés dans un ballon et dégazés sous courant d'argon pendant 10 mn. On y ajoute 0,4 g de bromhydrate de composé obtenu en 6.5, 45 mg de [1,1'-bis(diphénylphosphino)ferrocène] dichloropalladium et 0,9 g de carbonate de césium et on chauffe à 80°C pendant 4 heures. Après refroidissement, le mélange réactionnel est concentré sous pression réduite. Le résidu obtenu est purifié par chromatographie sur gel de silice en éluant avec un mélange heptane/acétate d'éthyle. On obtient 200 mg de composé. RMN 1H (CDCl3, δ en ppm) : 0 (s, 9H) ; 1,4 (s , 6H) ; 6,45 (d, 1H) ; 7,1 (m, 1H) ; 7,2 à 7,4 (m, 5H) ; 7,55 (m, 3H) ; 7,7 à 7,85 (m, 4H) ; 8,35 (s, 1H) ; 8,4 (d, 1H) ; 8,75 (s, 1H). M+H = 581.

### 6.11 2-{2-[2-(1H-Indol-6-yl)imidazo[1,2-a]pyridin-6-yl]pyridin-4-yl}propan-2-ol

On place dans un ballon 0,2 g du composé obtenu en 6.10 dans 4 ml d'éthanol et on ajoute 1,7 ml d'une solution 1N d'hydroxyde de sodium. Le mélange est agité 80°C pendant 20h. On concentre sous pression réduite et le résidu obtenu est purifié par chromatographie sur gel de silice en éluant avec un mélange dichlorométhane/méthanol. On obtient 64 mg de composé.
RMN 1H (DMSO-d6, δ en ppm) : 1,55 (s, 6H) ; 5,35 (s, 1H) ; 6,45 (s, 1H) ; 7,4 (t, 1H) ; 7,45 (d, 1H) ; 7,65 (m, 2H) ; 7,7 (d, 1 H) ; 8,0 (d, 1H) ; 8,1 (m, 2H) ; 8,45 (s, 1H) ; 8,6 (d, 1H) ; 9,3 (s, 1H) ; 11,2 (s, 1H).

### Exemple 7 : 2-{2-[2-(1H-Indazol-3-yl)imidazo[1,2-a]pyridin-6-yl]pyridin-4-yl}propan-2-ol (composé 153 du tableau)

### 7.1 4-[1-Méthyl-1-(triméthylsilanyloxy)éthyl]-[2,3']bipyridinyl-6'-ylamine

0,5 g du composé obtenu en 6.9, 8 ml de tétrahydrofurane et 2 ml d'eau sont placés dans un ballon et dégazés sous courant d'argon pendant 10 mn. On y ajoute 10,4 g de 5-(4,4,5,5-tétraméthyl-1,3,2-dioxaborolan-2-yl)pyridin-2-ylamine, 110 mg de [1,1'-bis(diphénylphosphino)ferrocène] dichloropalladium et 1,1 g de carbonate de césium et on chauffe à 80°C pendant 4 heures. Après refroidissement, le mélange réactionnel est concentré sous pression réduite. Le résidu obtenu est purifié par chromatographie sur gel de silice en éluant avec un mélange heptane/acétate d'éthyle. On obtient 240 mg de composé.
RMN 1H (CDCl3, δ en ppm) : 0 (s, 9H) ; 1,4 (s , 6H) ; 4,4 (s, 2H) ; 6,45 (d, 1H) ; 7,05 (m, 1H) ; 7,55 (s, 1H) ; 7,95 (m, 1H) ; 8,4 (d, 1H) ; 8,5 (m, 1H). M+H = 302.

### 7.2 N-Méthoxy-N-méthyl-1H-indazole-3-carboxamide

On place dans un ballon 4,0 g d'acide 1*H*-indazole-3-carboxylique, 2,6 g de chlorhydrate de *N,O*-diméthylhydroxylamine, 9,4 g de 1-(3-diméthylaminopropyl)-3-éthylcarbodiimide et 8,4 ml de pyridine dans 120 ml de tétrahydrofurane. Le mélange est agité à température ambiante pendant 20h. On concentre, le résidu est repris dans l'eau. Le précipité jaune obtenu est lavé avec de l'eau puis séché sous pression réduite. On obtient 3,5 g de composé.
RMN 1H (DMSO-d6, δ en ppm) : 3,5 (s, 3H) ; 3,8 (s, 3H) ; 7,25 (m, 1H) ; 7,45 (m, 1H) ; 7,65 (d, 1H) ; 8,05 (d, 1H) ; 13,65 (s, 1H). M+H = 206.

### 7.3 1-(1H-Indazol-3-yl)éthanone

On place dans un ballon 2,4 g de *N*-méthoxy-*N*-méthyl-1*H*-indazole-3-carboxamide dans 140 ml de tétrahydrofurane à 0°C et sous argon. On ajoute goutte à goutte 19 ml de bromure de méthylmagnésium (3M dans l'éther éthylique). Le mélange est agité une heure à 0°C et 20h à température ambiante. On refroidit à 0°C et on ajoute 80 ml d'eau et 40 ml d'une solution saturée en chlorure d'ammonium. Le mélange est extrait avec 40 ml d'acétate d'éthyle. La phase organique est lavée avec 40 ml d'une solution saturée en chlorure de sodium, séchée sur sulfate de magnésium puis concentrée sous pression réduite. Le résidu est purifié par chromatographie sur gel de silice en éluant par un mélange heptane/acétate d'éthyle. On obtient 1,6 g de composé.
RMN 1H (DMSO-d6, δ en ppm) : 2,65 (s, 3H) ; 7,35 (m, 1H) ; 7,55 (m, 1H) ; 7,7 (d, 1H) ; 8,2 (d, 1H) ; 13,9 (s, 1H). M+H = 161.

### 7.4 2-Bromo-1-(1H-indazol-3-yl)éthanone

On place dans un ballon 4,5 g de bromure de cuivre dans 180 ml d'acétate d'éthyle et on chauffe à reflux. On ajoute 1,6 g de 1-(1*H*-indazol-3-yl)éthanone. Le mélange est agité 4 heures à reflux. On filtre sur papier puis on verse sur 150 ml d'une solution à 20% de thiosulfate de sodium. Le mélange est extrait avec 60 ml d'acétate d'éthyle. La phase organique est lavée avec 40 ml d'une solution saturée en chlorure de sodium, séchée sur sulfate de magnésium puis concentrée sous pression réduite. On obtient 2,4 g de composé.
RMN 1H (DMSO-d6, δ en ppm) : 4,95 (s, 2H) ; de 7,35 à 7,55 (m, 2H) ; 7,75 (d, 1H) ; 8,2 (d, 1H); 14,1 (s, 1H). M+H = 239.

### 7.5 3-(6-{4-[1-Méthyl-1-(triméthylsilanyloxy)éthyl]pyridin-2-yl}imidazo[1,2-a]pyridin-2-yl)-1H-indazole

Dans un ballon de 50 ml, on introduit 140 mg du composé obtenu en 7.1 et 110 mg du composé obtenu en 7.4 dans 4 ml d'éthanol. On ajoute 40 mg de bicarbonate de sodium et on chauffe le mélange à 80°C pendant 4h. On évapore le solvant sous pression réduite et purifie le résidu par chromatographie en éluant avec un mélange dichlorométhane/méthanol. On obtient 137 mg de composé.
RMN 1H (CDCl3, δ en ppm) : 0 (s, 9H) ; 1,45 (s, 6H) ; 7,15 (m, 2H); 7,2 à 7,35 (m, 2H) ; 7,6 (m, 3H) ; 8,1 (s, 1H) ; 8,35 (d, 1H) ; 8,45 (d, 1H) ; 9,85 (s, 1H) ; 10,2 (s, 1H). M+H = 370.

### 7.6 2-{2-[2-(1H-Indazol-3-yl)imidazo[1,2-a]pyridin-6-yl]pyridin-4-yl}propan-2-ol

130 mg du composé obtenu en 7.5 sont dissous dans 2 ml de THF, on y ajoute 90 mg de fluorure de tétrabutylammonium hydraté et on agite 2 heures à température ambiante. Puis on évapore le solvant sous pression réduite et on purifie le résidu par chromatographie en éluant avec un mélange dichlorométhane/méthanol. On obtient 99 mg de composé.
RMN 1H (DMSO-d6, δ en ppm) : 1,5 (s, 6H) ; 5,35 (s, 1H) ; 7,25 (t, 1H) ; 7,4 (t, 1H) ; 7,45 (d, 1H) ; 7,6 (d, 1H) ; 7,75 (d, 1H) ; 8,05 (m, 2H) ; 8,5 (d, 1H) ; 8,55 (s, 1H) ; 8,65 (d, 1H) ; 9,4 (s, 1H) ; 13,15 (s, 1H) ; PF = 255-259°C.

Les tableaux qui suivent illustrent les structures chimiques de formule générale (I) (tableau 2) et les caractéristiques physicochimiques (tableau 3) de quelques exemples de composés selon l'invention.

Dans ces tableaux :
- « Ph » signifie phényle ;
- « Cl » signifie chlore ;
- « F » signifie fluor ;
- « Br » signifie Brome ;
- « Me » signifie méthyle ;
- « MeO » signifie méthoxy ;
- « (F₂CH)O » signifie difluorométhoxy ;
- la colonne « PF » renseigne les points de fusion des produits en degrés Celsius (°C) ou, lorsque les produits ont été isolés sous la forme de solide amorphe ou d'huile, ils sont caractérisés par leur masse [M+H];
- dans la colonne « Sel/base », « - » représente un composé sous forme de base libre, alors que « HCl » représente un composé sous forme de chlorhydrate et le rapport entre parenthèses est le rapport (acide:base).

**Tableau 2**

| | | | | |
|---|---|---|---|---|
| 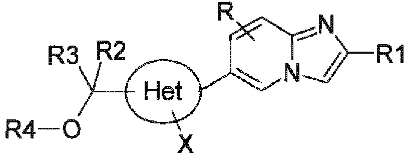 | | | | |

| **Ex** | **R₁** | **R** | 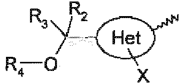 | **sel** |
|---|---|---|---|---|
| 1 | 4-Cl-Ph | H | 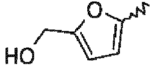 | - |
| 2 | 4-Cl-Ph | H | 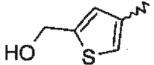 | - |
| 3 | 4-Cl-Ph | H | 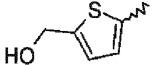 | - |
| 4 | 4-Cl-Ph | H | 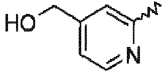 | - |
| 5 | 4-Cl-Ph | H | 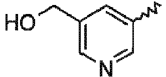 | - |
| 6 | 4-Cl-Ph | H | 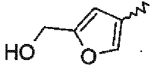 | - |
| 7 | 4-Cl-Ph | H | 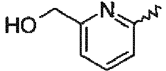 | - |
| 8 | 4-Cl-Ph | H | 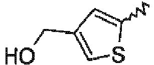 | - |
| 9 | 4-Cl-Ph | H | 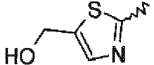 | - |
| 10 | 4-Cl-Ph | H | 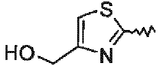 | - |
| 11 | 4-Cl-Ph | H | 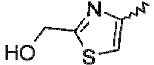 | - |
| 12 | 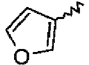 | H | 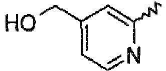 | HCl (1:1) |
| 13 | 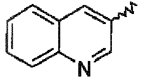 | H | 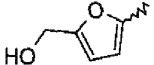 | HCl (2:1) |
| 14 | 4-Me-Ph | H | 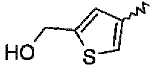 | - |
| 15 | 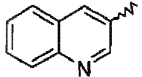 | H | 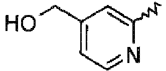 | HCl (3:2) |
| 16 | 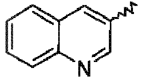 | H | 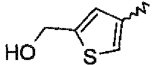 | HCl (2:1) |
| 17 | 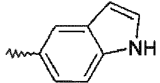 | H | 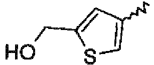 | HCl (1:1) |
| 18 | 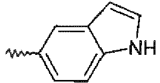 | H | 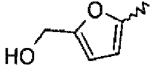 | HCl (1:1) |
| 19 | Ph | H | 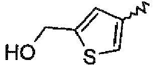 | HCl (1:1) |
| 20 | 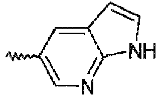 | H | 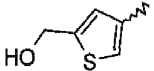 | HCl (1:1) |
| 21 | 3-MeO-Ph | H | 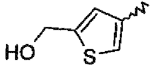 | HCl (1:1) |
| 22 | 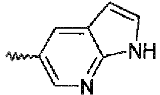 | H | 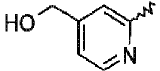 | HCl (1:1) |
| 23 | Ph | H | 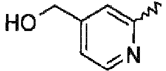 | - |
| 24 | 4-Me-Ph | H | 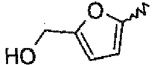 | HCl (1:1) |
| 25 | Ph | H | 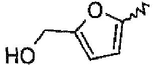 | - |
| 26 | 3-OMe-Ph | H | 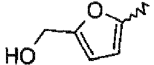 | - |
| 27 | 2,4-diF-Ph | H | 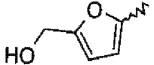 | - |
| 28 | 4-Cl-Ph | H | 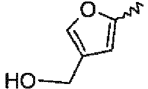 | - |
| 29 | 4-Cl-Ph | H | 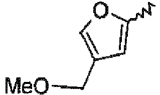 | |
| 30 | 4-(CH₂OH)-Ph | H | 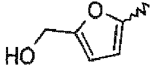 | - |
| 31 | 4-(CONMe₂)-Ph | H | 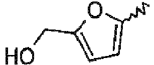 | - |
| 32 | 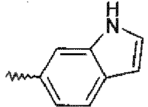 | H | 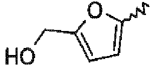 | - |
| 33 | 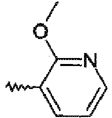 | H | 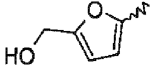 | - |
| 34 | 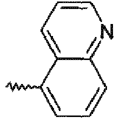 | H | 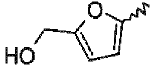 | . |
| 35 | 4-(*N*-morpholino)-Ph | H | 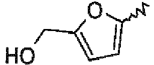 | - |
| 36 | 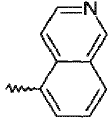 | H | 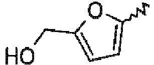 | - |
| 37 | 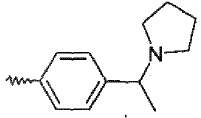 | H | 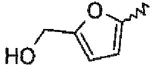 | - |
| 38 | 4-(Me₂NCO)-Ph | H | 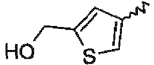 | - |
| 39 | 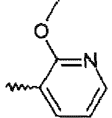 | H | 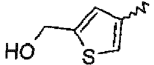 | - |
| 40 | 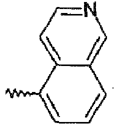 | H | 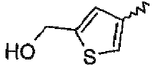 | - |
| 41 | 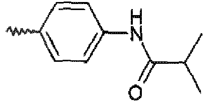 | H | 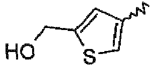 | - |
| 42 | 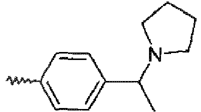 | H | 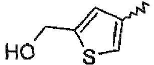 | - |
| 43 | 4-(Me₂NCH₂)-Ph | H | 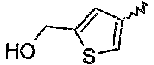 | - |
| 44 | 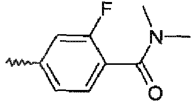 | H | 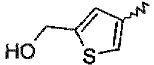 | - |
| 45 | 3-NH₂-Ph | H | 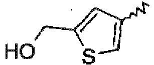 | - |
| 46 | 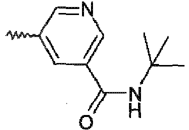 | H | 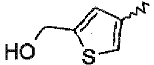 | - |
| 47 | -Ph | 3-Me | 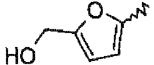 | - |
| 48 | 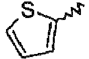 | 3-Me | 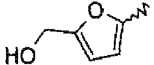 | - |
| 49 | 3-Cl-Ph | 3-Me | 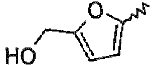 | - |
| 50 | 4-Pyrrolidino-Ph | H | 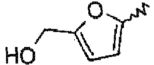 | - |
| 51 | 3-Cyano-Ph | H | 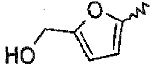 | - |
| 52 | 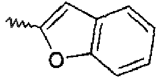 | H | 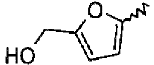 | - |
| 53 | 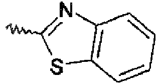 | H | 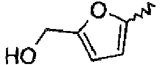 | - |
| 54 | 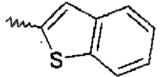 | H | 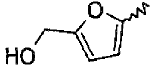 | - |
| 55 | 4-NO₂-Ph | H | 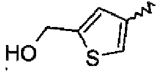 | - |
| 56 | 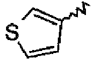 | H | 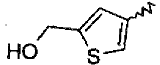 | - |
| 57 | 4-SMe-Ph | H | 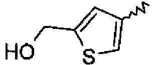 | - |
| 58 | 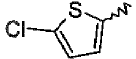 | H | 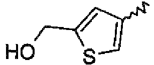 | - |
| 59 | 4-MeSO₂-Ph | H | 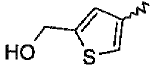 | - |
| 60 | 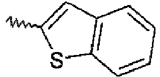 | H | 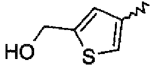 | - |
| 61 | 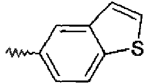 | H | 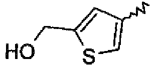 | - |
| 62 | Ph | 3-Me | 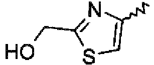 | - |
| 63 | 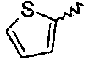 | 3-Me | 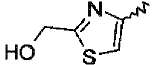 | - |
| 64 | 4-OMe-Ph | 3-Me | 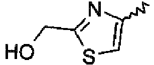 | - |
| 65 | 4-Pyrrolidino-Ph | H | 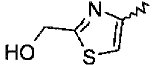 | - |
| 66 | 2-F-Ph | H | 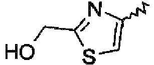 | - |
| 67 | 4-MeSO₂-Ph | H | 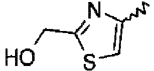 | - |
| 68 | 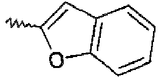 | H | 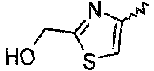 | - |
| 69 | 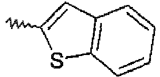 | H | 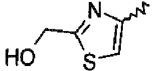 | - |
| 70 | 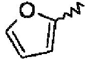 | H | 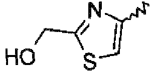 | - |
| 71 | 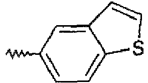 | H | 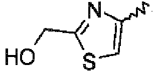 | - |
| 72 | 4-Pyrrolidino-Ph | H | 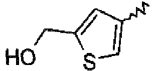 | - |
| 73 | 4-Cl-Ph | H | 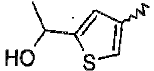 | - |
| 74 | 4-CI-Ph | H | 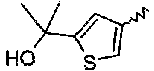 | - |
| 75 | 4-OMe-Ph | 3-Me | 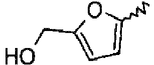 | - |
| 76 | 4-CF3-Ph | H | 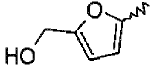 | - |
| 77 | 4-OMe-Ph | H | 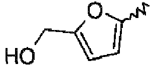 | - |
| 78 | 4-F-Ph | H | 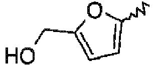 | - |
| 79 | 3,4-diF-Ph | H | 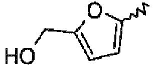 | - |
| 80 | 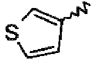 | H | 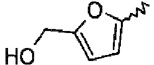 | - |
| 81 | 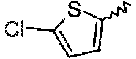 | H | 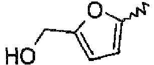 | - |
| 82 | 2-F-Ph | H | 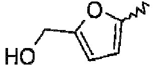 | - |
| 83 | 3-Br-Ph | H | 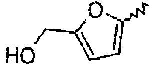 | - |
| 84 | 3-Me-4-Cl-Ph | H | 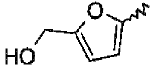 | - |
| 85 | 4-MeSO₂-Ph | H | 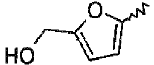 | - |
| 86 | 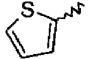 | H | 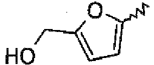 | - |
| 87 | 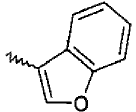 | H | 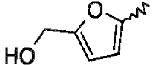 | - |
| 88 | 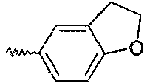 | H | 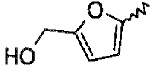 | - |
| 89 | 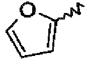 | H | 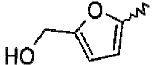 | - |
| 90 | 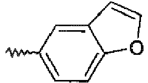 | H | 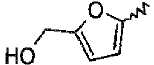 | - |
| 91 | 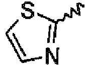 | H | 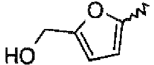 | - |
| 92 | 4-SMe-Ph | H | 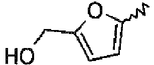 | - |
| 93 | 4-Cl-Ph | 3-Me | 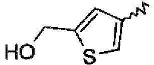 | - |
| 94 | 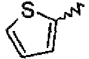 | 3-Me | 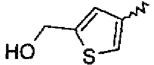 | - |
| 95 | 3-Cl-Ph | 3-Me | 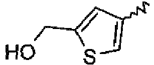 | - |
| 96 | 2,4-diF-Ph | 3-Me | 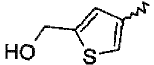 | - |
| 97 | 4-F-Ph | H | 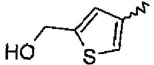 | - |
| 98 | 3,4-diF-Ph | H | 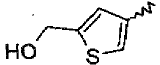 | - |
| 99 | 3-cyano-Ph | H | 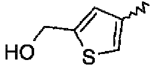 | - |
| 100 | 2-F-Ph | H | 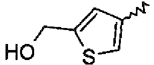 | - |
| 101 | 3-Br | H | 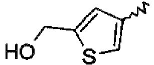 | - |
| 102 | 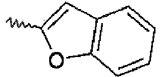 | H | 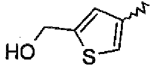 | - |
| 103 | 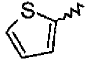 | H | 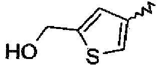 | - |
| 104 | 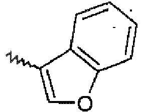 | H | 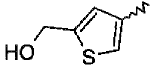 | - |
| 105 | 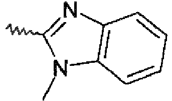 | H | 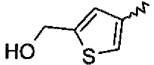 | - |
| 106 | 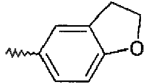 | H | 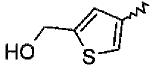 | - |
| 107 | 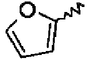 | H | 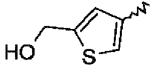 | - |
| 108 | 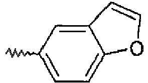 | H | 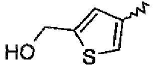 | - |
| 109 | 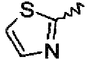 | H | 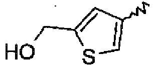 | - |
| 110 | 4-Cl-Ph | 3-Me | 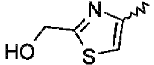 | - |
| 11 | 3-Cl-Ph | 3-Me | 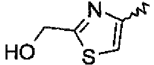 | - |
| 112 | 4-CF3-Ph | H | 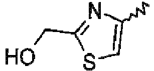 | - |
| 113 | 4-F-Ph | H | 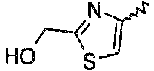 | - |
| 114 | 3,4-diF-Ph | H | 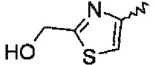 | - |
| 115 | 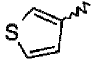 | H | 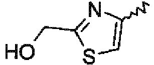 | - |
| 116 | 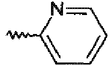 | H | 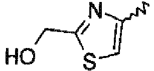 | - |
| 117 | 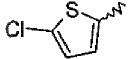 | H | 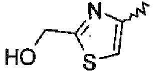 | - |
| 118 | 3-Br-Ph | H | 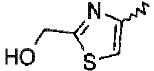 | - |
| 119 | 4-Cl-3-Me-Ph | H | 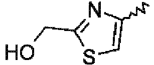 | - |
| 120 | 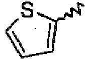 | H | 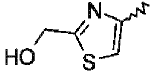 | - |
| 121 | 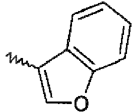 | H | 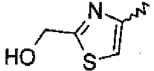 | - |
| 122 | 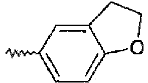 | H | 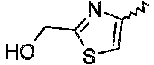 | - |
| 123 | 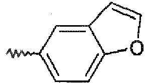 | H | 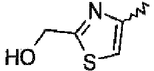 | - |
| 124 | 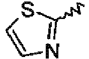 | H | 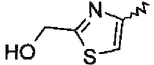 | - |
| 125 | 3-(CH₂OH)-Ph | H | 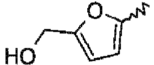 | - |
| 126 | 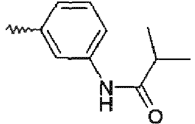 | H | 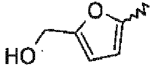 | - |
| 127 | 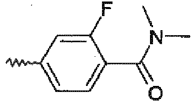 | H | 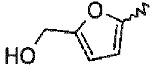 | - |
| 128 | 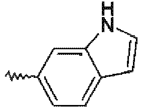 | H | 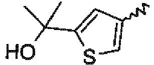 | - |
| 129 | 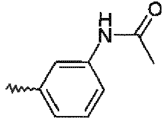 | H | 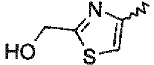 | - |
| 130 | 4-(CH₂OH)-Ph | H | 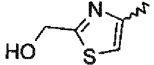 | - |
| 131 | 3-(CH₂OH)-Ph | H | 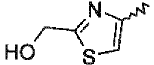 | - |
| 132 | 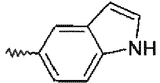 | H | 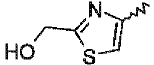 | - |
| 133 | 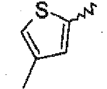 | H | 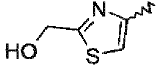 | - |
| 134 | 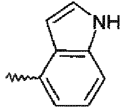 | H | 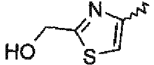 | - |
| 135 | 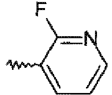 | H | 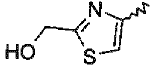 | - |
| 136 | 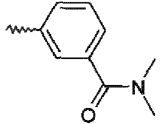 | H | 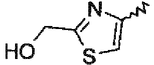 | - |
| 137 | 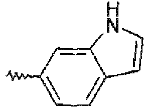 | H | 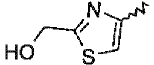 | - |
| 138 | 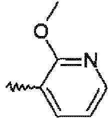 | H | 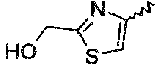 | - |
| 139 | 4-(CONHMe)-Ph | H | 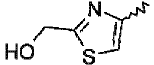 | - |
| 140 | 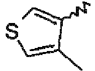 | H | 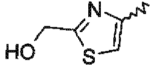 | - |
| 141 | 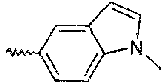 | H | 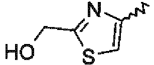 | - |
| 142 | 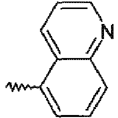 | H | 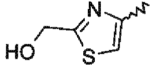 | - |
| 143 | 4-(MeCONH)-Ph | H | 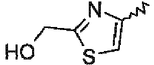 | - |
| 144 | 4-(*N*-Morpholino)-Ph | H | 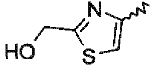 | - |
| 145 | 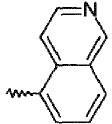 | H | 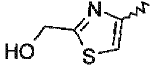 | - |
| 146 | 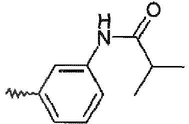 | H | 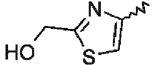 | - |
| 147 | 3-(Me₂NSO₂)-Ph | H | 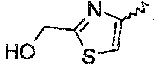 | - |
| 148 | 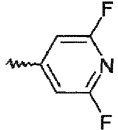 | H | 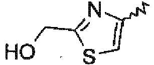 | - |
| 149 | 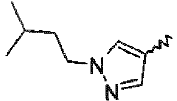 | H | 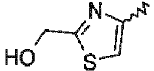 | - |
| 150 | 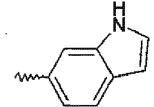 | H | 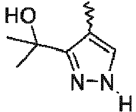 | - |
| 151 | 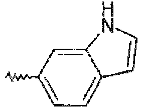 | H | 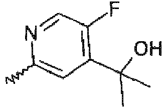 | - |
| 152 | 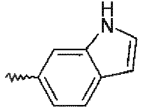 | H | 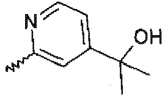 | - |
| 153 | 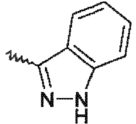 | H | 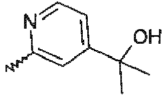 | - |
| 154 | 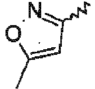 | H | 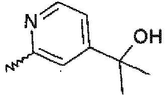 | - |
| 155 | 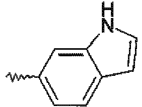 | H | 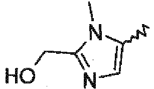 | - |
| 156 | 4-Cl-Ph | H | 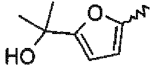 | - |

**Tableau 3**

| **Ex** | **PF ou [M+H]** | **RMN** Spectre ¹H (DMSO.-d6, δ en ppm) |
|---|---|---|
| 1 | 197-199 | 4,5 (d, 2H) ; 5,3 (t, 1H) ; 6,5 (m, 1H) ; 7,95 (m, 1H) ; 7,55 (m, 2H) ; de 7,60 à 7,70 (m, 2H) ; 8 (m, 2H) ; 8,5 (s, 1H) ; 8,85 (s, 1H). |
| 2 | 190-192 °C | 4,7 (d, 2H) ; 5,55 (t, 1H) ; 7,45 (s, 1H) ; 7,55 (d, 2H) ; de 7,60 à 7,70 (m, 2H) ; 7,85 (s, 1H) ;8,05 (d, 2H) ; 8,4 (s, 1H) ; 8,95 (s, 1H). |
| 3 | 227-228°C | 4,65 (d, 2H) ; 5,55 (t, 1H) ; 7,0 (d, 1H) ; 7,40 (d, 1H) ; de 7,50 à 7,70 (m, 4H) ; 8,0 (d, 2H) ; 8,45 (s, 1H) ; 8,85 (s, 1H). |
| 4 | 207,7-208,4 | 4,65 (d, 2H) ; 5,5 (t, 1H) ; 7,35 (d, 1H) ; 7,55 (d, 2H) ; 7,70 (d, 1H) ; 7,95 (s, 1H) ; de 8,0 à 8,10 (m, 3H) ; 8,55 (s, 1H) ; 8,65 (d, 1H) ; 9,35 (s, 1H). |
| 5 | 238,4-239,5 | 4,65 (d, 2H) ; 5,4 (t, 1H) ; 7,55 (d, 2H) ; de 7,65 à 7,75 (m, 2H) ; de 8,0 à 8,1 (m, 3H) ; 8,55 (s, 1H) ; 8,6 (s, 1H) ; 8,9 (s, 1H) ; 9,0 (s, 1H). |
| 6 | 194,7-195,2 | 4,4 (d, 2H) ; 5,3 (t, 1H) ; 6,8 (s, 1H) ; de 7,45 à 7,65 (m, 4H) ; 8,0 (d, 2H) ; 8,15 (s, 1H) ; 8,35 (s, 1H) ; 8,75 (s, 1H). |
| 7 | 197,4-198,2 | 4,7 (d, 2H) ; 5,5 (t, 1H) ; 7,5 (d, 1H) ; 7,55 (d, 2H) ; 7,7 (d, 1H) ; 7,9 (d, 1H) ; 7,95 (t, 1H) ; de 8,0 à 8,10 (m, 3H) ; 8,6 (s, 1H) ; 9,3 (s, 1H). |
| 8 | 211,3-211,9 | 4,5 (d, 2H) ; 5,2 (t, 1H) ; 7,35 (s, 1H) ; 7,5 (s, 1H) ; 7,5 (d, 2H) ; 7,6 (d, 1H) ; 7,65 (d, 1H) ; 8,0 (d, 2H) ; 8,45 ( s, 1H) ; 8,9 (s, 1H). |
| 9 | 229-231 | 4,75 (d, 2H) ; 5,65 (t, 1H) ; 7,52 (2d, 2H) ; de 7,7 à 7,8 (m, 3H); 8,0 (d, 2H) ; 8, 52 (s, 1H) ; 9,22 (s, 1H). |
| 10 | 193-194 | 4,65 (d, 2H) ; 5,4 (t, 1H) ; 7,5 (m, 3H) ; 7,7 (m, 2H) ; 8,0 (d, 2H) ;. 8,55 (s, 1H) ; 9,3 (s, 1H). |
| 11 | 190-192 | 4,85 (s, 2H) ; 6,2 (t, 1H), de 7,50 à 7,60 (m, 2H) ; 7,7 (d, 1H) ; 7,85 (d, 1H) ; de 7,95 à 8,05 (m, 2H) ; 8,1 (s, 1H) ; 8,5 (s, 1H) ; 9,1 (s, 1H). |
| 12 | 268-273 | 4,7 (s, 2H) ; 7,2 (s, 1H); 7,5 (s, 1H) ; de 7, 9 à 8,1 (m, 3H) ; de 8,5 à 8,6 (m, 3H) ; 8,7 (s, 1H) ; 9,65 (s, 1H). |
| 13 | 310-315 | 4,55 (s, 2H) ; 6,55 (s, 1H) ; 7,15 (s, 1H) ; 7,85 (m, 1H) ; 7,95 (m, 2H) ; 8,1 (m, 1H) ; 8,2 (m, 2H) ; 8,95 (s, 1H) ; 9,15 (s, 1H) ; 9,25 (s, 1H) ; 9,62 (s, 1H). |
| 14 | 197,5-198 | 2,35 (s, 3H) ; 4,7 (d, 2H) ; 5,55 (t, 1H) ; 7,25 (d, 2H) ; 7,45 (s, 1H) ; 7,6 (m, 2H) ; 7,8 (s, 1H) ; 7,87 (d, 2H) ; 8,3 (s, 1H) ; 8,9 (s, 1H). |
| 15 | 255-260 | 4,73 (s, 2H) ; 7,5 (d, 1H) ; 7,8 (t, 1H) ; 7,93 (t, 1H) ; 8,0 (d, 1H) ; 8,1 (s, 1H) ; 8,2 (m, 2H) ; 8,4 (d, 1H) ; 8,72 (d, 1H) ; 9,0 (s, 1H) ; 9,2 (s, 1H) ; 9,58 (s, 1H) ; 9,63 (s, 1H). |
| 16 | 310-315 | 4,75 (s, 2H) ; 7,55 (s, 1H) ; 7,8 (t, 1H) ; de 7,9 à 8,0 (m, 2H) ; 8,05 (s, 1H) ; de 8,17 à 8,25 (m, 3H) ; 8,9 (s, 1H) ; 9,328 (s, 1H) ; 9,32 (s, 1H) ; 9,67 (s, 1H). |
| 17 | 390-395 | 4,75 (s, 2H) ; 6,6 (s, 1H) ; 7,5 (m, 2H) ; 7,6 (d, 1H) ; 7,75 (d, 1H) ; 7,95 (d, 1H) ; 8,05 (s, 1H) ; 8,25 (m, 2H) ; 8,6 (s, 1H) ; 9,25 (s, 1H) ; 11,45 (s, 1H). |
| 18 | 390-395 | 4,55 (s, 2H) ; 6,55 (s, 1H) ; 6,6 (s, 1H) ; 7,15 (m, 1H) ; 7,5 (m, 1H) ; 7,6 (d, 1H) ; 7,7 (d, 1H) ; 7,95 (d, 1H) ; 8,15 (d, 1H) ; 8,25 (s, 1H) ; 8,65 (s, 1H) ; 9,1 (s, 1H) ; 11,45 (s, 1H). |
| 19 | 267 | 4,75 (s, 2H) ; de 7,5 à 7,6 (m, 2H) ; 7,65 (m, 2H) ; 8,0 (m, 2H) ; 8,1 (d, 2H) ; 8,3 (d, 1H) ; 8,7 (s, 1H) ; 9,3 (s, 1H). |
| 20 | 405-410 | 4,75 (s, 2H) ; 6,65 (m, 1H) ; 7,55 (s, 1H) ; 7,65 (m, 1H) ; 7,95 (d, 1H) ; 8,05 (s, 1H) ; 8,25 (d, 1H) ; 8,6 (s, 1H) ; 8,7 (s, 1H) ; 8,9 (s, 1H) ; 9,25 (s, 1H) ; 12,05 (s, 1H). |
| 21 | 305 | 3,9 (s, 3H) ; 4,7 (s, 2H) ; 7,1 (d, 1H) ; 7,55 (m, 2H) ; 7,65 (d, 1H) ; 7,7 (s, 1H) ; 7,95 (d, 1H) ; 8,0 s, 1H) ; 8,25 (d, 1H) ; 8,7 (s, 1H) ; 9,2 (s, 1H). |
| 22 | 305-310 | 4,7 (s, 2H) ; 6,65 (m, 1H) ; 7,5 (m, 1H) ; 7,65 (m, 1H) ; 8,05 (s, 1H) ; 8,1 (s, 1H) ; 8,6 (d, 1H) ; 8,65 (s, 1H) ; 8,7 (d, 1H) ; 8,85 (s, 1H) ; 8,9 (s, 1 H) ; 9,65 (s, 1H) ; 12,1 (s, 1 H). |
| 23 | 225-226 | 4,65 (d, 2H) ; 5,55 (t, 1H) ; 7,35 (m, 2H) ; 7,5 (m, 2H) ; 7,7 (d, 1H) ; de 7,95 à 8,05 (m, 4H) ; 8,5 (s, 1H) ; 8,65 (d, 1H) ; 9,35 (s, 1H). |
| 24 | 248-254 | 2,4 (s, 3H) ; 4,5 (s, 2H) ; 6,55 (d, 1H) ; 7,15 (d, 1H) ; 7,45 (d, 2H) ; de 7,85 à 8,0 (m, 3H) ; 8,15 (d, 1H) ; 8,7 (s, 1H) ; 9,1 (s, 1H). |
| 25 | 202-204 | 4,5 (s, 2H) ; 5,3 (m, 1H) ; 6,45 (d, 1H) ; 6,95 (d, 1H) ; 7,35 (m, 1H) ; 7,5 (m, 2H) ; 7,65 (m, 2H) ; 7,95 (d, 2H) ; 8,5 (s, 1H) ; 8,9 (s, 1H). |
| 26 | 172-174 | 3,85 (s, 3H) ; 4,5 (s, 2H) ; 5,3 (s, 1H) ; 6,45 (s, 1H) ; 6,95 (m, 2H) ; 7,4 (m, 1H) ; 7,55 (m, 2H) ; 7,65 (m, 2H) ; 8,5 (s, 1H) ; 8,85 (s, 1H). |
| 27 | 207-208 | 4,5 (d, 2H) ; 5,3 (t, 1H) ; 6,5 (s, 1H) ; 6,95 (s, 1H) ; 7,25 (m, 1H) ; 7,4 (m, 1H) ; 7,65 (m, 2H) ; 8,3 (m, 1H) ; 8,45 (s, 1H) ; 8,95 (s, 1H). |
| 28 | 174-176 | 4,45 (d, 2H) ; 5,05 (t, 1H) ; 7,00 (s, 1H) ; de 7,55 (d, 2H) ; de 7,6 à 7,7 (m, 3H) ; 8,0 (d, 2H) ; 8,45 (s, 1H) ; 8.85 (s, 1H). |
| 29 | 176-178 | 3,3 (s, 3H) ; 4,3 (s, 2H); 7,0 (s, 1H) ; 7,5 (d, 2H); de 7,65 à 7,8 (m, 3H) ; 8,0 (d, 2H) ; 8,45 (s, 1H) ; 8,9 (s, 1H). |
| 30 | [321] | 4,47 (d, 2H) ; 4,55 (d, 2H) ; 5,2 (t, 1H) ; 5,31 (t, 1H) ; 6,42 (s, 1H) ; 6,91 (s, 1H) ; 7,4 (m, 2H) ; 7,6 (m, 2H) ; 7,91 (m, 2H) ; 8,41 (s, 1H) ; 8,82 (s, 1H). |
| 31 | [362] | 3 (m, 6H) ; 4,49 (m, 2H) ; 5,3 (m, 1H) , 6,42 (s, 1H) ; 6,92(s, 1H) ; 7,5 (m, 2H) ; 7,62 (m, 2H) ; 8 (m, 2H) ; 8,51 (s, 1H) ; 8,75 (s, 1H). |
| 32 | [330] | 4,45 (m, 2H) ; 5,3 (m, 1H) ; 6,45 (m, 2H) ; 6,9 (s, 1H) ; 7,4 (m, 1H) ; 7,55 à 7,65 (m, 4H) ; 8 (s, 1H) ; 8,4 (s, 1H) ; 8,85 (s, 1H) ; 11,2 (s, 1H). |
| 33 | [322] | 4,08 (s, 3H) ; 4,49 (m, 2H) ; 5,3 (m, 1H) ; 6,42 (d, 1H) ; 6,9 (d, 1H) ; 7,12 (m, 1H) ; 7,6 (m, 2H) ; 8,26 (m, 1H) ; 8,55 (m, 2H) ; 8,95 (s, 1H). |
| 34 | [342] | 4,5 (d, 2H) ; 5,32 (t, 1H) ; 6,49 (s, 1H) ; 6,94 (s, 1H) ; 7,6 (m, 1H) ; 7,68 (d, 1H) ; 7,76 (d, 1H) ; 7,83 (m, 1H); 7,95 (d, 1H) ; 8,07 (d, 1H) ; 8,5 (s, 1H) ; 8,92 (m, 2H) ; 9,31 (d, 1H). |
| 35 | [376] | 3,15 (m, 4H) ; 3,75 (m, 4H) ; 4,49 (m, 2H) ; 5,3 (m, 1H) ; 6,42 (d, 1H) ; 6,9 (d, 1H) ; 7 (m, 2H) ; 7,56 (m, 2H) ; 7,8 (m, 2H) ; 8,3 (s, 1H) ; 9,89 (s, 1H). |
| 36 | [342] | 4,5 (d, 2H) ; 5,32 (t, 1H) ; 6,49 (s, 1H) ; 6,99 (s, 1H) ; 7,69 (m, 1H) ; 7,73 (m, 2H) ; 8,18 (m, 2H) ; 8,51 (s, 1H) ; 8,58 (d, 1H) ; 8,29 (d, 1H) ; 8,94 (s, 1H) ; 9,4 (s, 1H). |
| 37 | [388] | 1,3 (d, 3H) ; 1,7 (m, 4H) ; 2,3 (m, 2H) ; 2,5 (m, 2H) ; 3,2 (q, 1H), 4,5 (d, 2H) ; 5,3 (t, 1H) ; 6,45 (d 1H) ; 6,9 (d, 1H) ; 7,4 (m, 2H) ; 7,6 (m, 2H) ; 7,9 (m, 2H) ; 8,4 (s, 1H) ; 8,8 (s, 1H) |
| 38 | [378] | 3 (m, 6H) ; 4,7 (m, 2H) ; 5,59 (m, 1H) ; 7,42 (m, 1H) ; 7,5 (m, 2H) ; 7,65 (m, 2H) ; 7,81 (s, 1H) ; 8,01 (m, 2H) ; 8,41 (s, 1H) ; 8,91 (s, 1H). |
| 39 | [338] | 4,08 (s, 3H) ; 4,69 (m, 2H) ; 5,58 (m, 1H) ; 7,15 (m, 1H) ; 7,4 (s, 1H) ; 7,62 (m, 2H) ; 7,8 (s, 1H) ; 8,16 (m, 1H) ; 8,41 (s, 1H) ; 8,59 (d, 1H) ; 9,01 (s, 1H). |
| 40 | [358] | 4,7 (d, 2H) ; 5,59 (t, 1H) ; 7,44 (s, 1H) ; 7,75 (m, 3H) ; 7,85 (s, 1H) ; 8,15 (m, 2H) ; 8,4 (s, 1H) ; 8,58 (d, 1H) ; 8,9 (d, 1H) ; 9 (s, 1H) ; 9,39 (s, 1H). |
| 41 | [392] | 1,11 (d, 6H) ; 2,51 (m, 1H) ; 4,68 (m, 2H) ; 5,57 (m, 1H) ; 7,33 (m, 1H) ; 7,41 (s, 1H) ; 7,6 (m, 4H) ; 7,8 (s, 1H) ; 8,29 (s, 2H) ; 8,9 (s, 1H) ; 9,9 (s, 1H). |
| 42 | [404] | 1,3 (d, 3H) ; 1,7 (m, 4H) ; 2,3 (m, 2H) ; 2,5 (m, 2H) ; 3,2 (q, 1H}, 4,7 (d, 2H) ; 5,55 (t, 1H) ; 7,4 (m, 3H) ; 7,6 (m, 2H) ; 7,8 (s, 1H) ; 7,9 (d, 2H) ; 8,3 (s, 1H) ; 8,9 (s, 1H) |
| 43 | [364] | 2,18 (s, 6H) ; 3,4 (s, 2H) ; 4,68 (d, 2H) ; 5,58 (t, 1H) ; 7,32 (d, 2H) ; 7,41 (s, 1H) ; 7,6 (m, 2H) ; 7,71 (s, 1H) ; 7,92 (m, 2H) ; 8,31 (s, 1H) ; 8,9 (s, 1 H). |
| 44 | [396] | 2,9 (s, 3H) ; 3,2 (s, 3H) ; 4,68 (m, 2H) ; 5,56 (m, 1H) ; 7,43 (m, 2H) ; 7,64 (m, 2H) ; 7,83 (s, 1H) ; 7,89 (m, 2H) ; 8,47 (s, 1H) ; 8,91 (s, 1H). |
| 45 | [322] | 4,68 (m, 2H) ; 5,11 (m, 2H) ; 5,55 (m, 1H) ; 6,5 (d, 1H) ; 7,05 (m, 2H) ; 7,22 (s, 1H) ; 7,41 (s, 1H) ; 7,59 (m, 2H) ; 7,8 (s, 1H) ; 8,18 (s, 1H) ; 8,9 (s, 1H). |
| 46 | [407] | 1,42 (s, 9H) ; 4,69 (m, 2H) ; 5,08 (m, 1H) ; 7,45 (s, 1H) ; 7,68 (m, 2H) ; 7,85 (s, 1H) ; 8,17 (s, 1H) ; 8,52 (s, 1H) ; 8,61 (m, 1H) ; 8,86 (s, 1H) ; 8,95 (s, 1H) ; 9,27 (s, 1H). |
| 47 | [305] | 2,71 (s, 3H) ; 4,5 (d, 2H) ; 5,32 (t, 1H) ; 6,44 (d, 1H) ; 6,99 (d, 1H) ; 7,35 (m, 1H) ; 7,48 (m, 2H) ; 7,63 (m, 2H) ; 7,8 (d, 2H) ; 8,49 (s, 1H). |
| 48 | [311] | 2,73 (s, 3H) ; 4,49 (d, 2H) ; 5,31 (t, 1H); 6,44 (d, 1H) ; 6,97 (d, 1H) ; 7,18 (m, 1H) ; 7,48 (m, 1H) ; 7,55 (m, 1H) ; 7,6 (m, 2H) ; 8,46 (s, 1H). |
| 49 | [339] | 2,72 (s, 3H) ; 4,49 (m, 2H) ; 5,31 (m, 1H) ; 6,44 (d, 1H) ; 6,99 (d, 1H) ; 7,41 (m, 1H) ; 7,51 (m, 1H) ; 7,65 (m, 2H) ; 7,77 (m, 1H) ; 7,82 (m, 1H) ; 8,5 (s, 1H). |
| 50 | [360] | 2 (m, 4H) ; 3,29 (m, 4H) ; 4,48 (d, 2H) ; 5,29 (t, 1H) ; 6,41 (d, 1H) ; 6,61 (m, 2H) ; 6,88 (d, 1H) ; 7,53 (m, 2H) ; 7,75 (m, 2H) ; 8,2 (s, 1H) ; 8,76 (s, 1H). |
| 51 | [316] | 4,48 (d, 2H) ; 5,31 (t, 1H) ; 6,45 (d, 1H) ; 6,95 (d, 1H) ; 7,65 (m, 3H) ; 7,77 (m, 1H) ; 8,29 (m, 1H) ; 8,37 (m, 1H) ; 8,51 (s, 1H) ; 8,85 (s, 1H). |
| 52 | [331] | 4,49 (m, 2H) ; 5,33 (m, 1H) ; 6,49 (d, 1H) ; 6,96 (d, 1H) ; 7,3 (m, 3H) ; 7,65 (m, 4H) ; 8,49 (s, 1H) ; 8,91 (s, 1H). |
| 53 | [348] | 4,49 (m, 2H) ; 5,35 (m, 1H) ; 6,49 (d, 1H) ; 7,01 (d, 1H) ; 7,45 (m, 1H) ; 7,55 (m, 1H) ; 7,72 (m, 2H) ; 8,05 (d, 1H) ; 8,15 (d, 1H) ; 8,76 (s, 1H); 8,96 (s, 1H). |
| 54 | [347] | 4,49 (m, 2H) ; 5,31 (m, 1H) ; 6,47 (d, 1H) ; 6,94 (d, 1H) ; 7,35 (m, 2H) ; 7,64 (m, 2H) ; 7,84 (m, 2H) ; 8 (d, 1H) ; 8,5 (s, 1H) ; 8,9 (s, 1H) |
| 55 | [352] | 4,7 (m, 2H) ; 5,6 (m, 1H) ; 7,45 (s, 1H) ; 7,7 (m, 2H) ; 7,85 (s, 1H) ; 8,3 (m, 4H) ; 8,6 (s, 1H) ; 8,95 (s, 1H). |
| 56 | [313] | 4,7 (m, 2H) ; 5,6 (m, 1H) ; 7,41 (s, 1H) ; 7,6 (m, 4H) ; 7,8 (s, 1H) ; 7,91 (s, 1H) ; 8,2 (s, 1H) ; 8,9 (s, 1H). |
| 57 | [353] | 2,51 (s. 3H) ; 4,7 (m, 2H) ; 5,58 (m, 1H); 7,31 (m, 2H) ; 7,42 (s, 1H) ; 7,6 (m, 2H) ; 7,8 (s, 1H) ; 7,91 (m, 2H) ; 8,31 (s, 1H) ; 8,88 (s, 1H). |
| 58 | [347] | 4,68 (m, 2H) ; 5,59 (m, 1H) ; 7,16 (d, 1H) ; 7,4 (m, 2H) ; 7,62 (m, 2H) ; 7,81 (s, 1 H) ; 8,27 (s, 1H) ; 8,9 (s, 1H). |
| 59 | [385] | 3,25 (s, 3H) ; 4,7 (m, 2H) ; 5,6 (m, 1H) ; 7,45 s, 1H) ; 7,68 (m, 2H) ; 7,35 (s, 1H) ; 7,98 (d, 2H) ; 8,23 (d, 2H) ; 8,53 (s, 1H) ; 8,94 (s, 1H). |
| 60 | [363] | 4,7 (m, 2H) ; 5,58 (m, 1H) ; 7,35 (m, 2H) ; 7,43 (s, 1H) ; 7,65 (m, 2H) ; 7,85 (m, 2H) ; 7,9 (s, 1H) ; 7,98 (d, 1H) ; 8,4 (s, 1H) ; 8,95 (s, 1H). |
| 61 | [363] | 4,7 (m, 2H) ; 5,58 (m, 1H) ; 7,43 (s, 1H) ; 7,52 (d, 1H) ; 7,63 (m, 2H) ; 7,8 (m, 2H) ; 7,98 (m, 1H) ; 8,06 (m, 1H) ; 8,4 (s, 1H) ; 8,95 (s, 1H) ; 8,92 (s, 1H). |
| 62 | [322] | 2,72 (s, 3H) ; 4,85 (d, 2H) ; 6,15 (t, 1H) ; 7,35 (t, 1H) ; 7,48 (t, 2H) ; 7,65 (d, 1H) ; 7,84 (m, 3H) ; 8,15(s, 1H) ; 8,75 (s, 1H) |
| 63 | [328] | 2,73 (s, 3H) ; 4,85 (d, 2H) ; 6,18 (t, 1H) ; 7,17 (d, 1H) ; 7,48 (d, 1H) ; 7,55 (d, 1H) ; 7,63 (d, 1H) ; 7,82 (d, 1H) ; 8,15 (s, 1H); 8,73.(s, 1H) |
| 64 | [352] | 2,70 (s, 3H) ; 3,82 s, 3H) ; 4,82 (d, 2H) ; 6,16 (t, 1H) ; 7,05 (d, 2H) ; 7,63 (d, 1H) ; 7,75 (d, 2H) ; 7,82 (d, 1H) ; 8,15 (s, 1H) ; 8,75 (s, 1H) |
| 65 | [377] | 1,98 (t, 4H) ; 3,28 (t, 4H) ; 4,82 (t, 2H) ; 6,18 (t, 1H) ; 6,60 (d, 2H) ; 7,56 (d, 1H) ; 7,75 (m, 3H) ; 8,05 (s, 1H) ; 8,25 (s, 1H) ; 9,03 (s, 1H) |
| 66 | [326] | 4,82 (s, 2H) ; 6,19 (m, 1H) ; 7,31 (m, 2H) ; 7,38 (m, 1H) ; 7,67 (d, 1H) ; 7,84 (d, 1H) ; 8,08 (s, 1H) ; 8,38 (t, 1H) ; 8,45 (d, 1H) ;9,22 (s, 1H) |
| 67 | [386] | 3,25 (s, 3H) ; 4,85 (s, 2H) ; 6,18 (m, 1H) ; 7,71 (d, 1H) ; 7,87 (d, 1H) ; 8,00 (d, 2H) ; 8,14 (s, 1H) ; 8,22 (d, 2H) 8,67 (s, 1H) ; 9,14 (s, 1H) |
| 68 | [348] | 4,83 (s, 2H) ; 6,19 (m, 1H} ; 7,28 (m, 2H) ; 7,35 (m, 1H) ; 7,67 (m, 3H) ; 7,79 (d, 1H) ; 8,12 (s, 1H) 8,53 (s, 1H) ; 9,20 (s, 1H) |
| 69 | [364] | .4,83 (s, 2H) ; 6,19 (m, 1H) ; 7,35 (m, 2H) ; 7,68 (d, 1H) ; 7,85 (m, 3H) ; 7,98 (d, 1H) ; 8,12 (s, 1H) 8,55 (s, 1H) ; 9,15 (s, 1H) |
| 70 | [298] | 4,82 (s, 2H) ; 6,18 (m, 1H) ; 6,60 (d, 1H) ; 6,82 (d, 1H) ; 7,60 (d, 1H) ; 7,74 (s, 1H) ; 7,82 (d, 1H) ; 8,08 (s, 1H) 8,38 (s, 1H) ; 9,14 (s, 1H) |
| 71 | [364] | 4,83 (s, 2H) ; 6,18 (m, 1H) ; 7,55 (d, 1H) ; 7,67 (d, 1H) ; 7,82 (m, 2H) ; 7,96 (d, 1H) ; 8,08 (d, 1H) 8,12 (s, 1H) 8,50 (s, 1H) 8,55 (s, 1H) ; 9,15 (s, 1H) |
| 72 | [376] | 2 (m, 4H) ; 3,32 (m, 4H) ; 4,68 (d, 2H) ; 5,58 (t, 1H) ; 6,6 (d, 2H) ; 7,41 (s, 1H) ; 7,55 (m, 2H) ; 7,75 (m, 3H) ; 8,11 (s, 1H) ; 8,82 (s,1H). |
| 73 | 181,5-182 | 1,5 (d, 3H) ; de 4,95 à 5,05 (m, 1H) ; 5,65 (d, 1H) ; 7,45 (s, 1H) ; 7,55 (d, 2H) ; de 7,6 à 7,7 (m, 2H) ; 7,8 (s, 1H) ; 8,05 (d, 2H) ; 8,4 (s, 1H) ; 8,95 (s, 1H). |
| 74 | 206-210 | 1,6 (s, 6H) ; 5,5 (s, 1H) ; 7,4 (s, 1H) ; 7,5 (d, 2H) ; de 7,6 à 7,7 (m, 2H) ; 7,75 (s, 1H) ; 8,0 (d, 2H) ; 8,35 (s, 1H) ; 8,9 (s, 1H). |
| 75 | [335] | 2,68 (s, 3H) ; 3,81 (s, 3H) ; 4,49 (d, 2H) ; 5,32 (t, 1H) ; 6,45 (d, 1H) ; 6,98 (d, 1H) ; 7,06 (d, 2H) ; 7,61 (m, 2H) ; 7,75 (m, 2H) ; 8,48 (s, 1H). |
| 76 | [358] | 4,5 (d, 2H) ; 5,32 (t, 1H) ; 6,45 (d, 1H) ; 6,95 (d, 1H) ; 7,66 (m, 2H) ; 7,81 (m, 2H) ; 8,18 (m, 2H) ; 8,6 (s, 1H) ; 8,87 (s, 1H). |
| 77 | [321] | 3,8 (s, 3H) ; 4,48 (d, 2H) ; 5,31 (t, 1H) ; 6,42 (d, 1H) ; 6,9 (d, 1H) ; 7,02 (d, 2H) ; 7,6 (m, 2H) ; 7,89 (d, 2H) ; 8,32 (s, 1H) ; 8,81 (s, 1H). |
| 78 | [308] | 4,48 (d, 2H) ; 5,31 (t, 1H) ; 6,42 (d, 1H) ; 6,92 (d, 1H) ; 7,3 (m, 2H) ; 7,61 (m, 2H) ; 7,99 (m, 2H) ; 8,41 (s, 1H) ; 8,82 (s, 1H). |
| 79 | [327] | 4,48 (m, 2H) ; 5,31 (m, 1H) ; 6,43 (d, 1H) ; 6,93 (d, 1H) ; 7,51 (m, 1H) ; 7,65 (m, 2H) ; 7,8 (m, 1H) ; 7,97 (m, 1H) ; 8,49 (s, 1H) ; 8,82 (s, 1H). |
| 80 | [297] | 4,48 (d, 2H) ; 5,31 (t, 1H) ; 6,42 (d, 1H) ; 6,91 (d, 1H) ; 7,6 (m, 4H) ; 7,9 (d, 1H) ; 8,29 (s, 1H) ; 8,82 (s, 1H). |
| 81 | [331] | 4,48 (m, 2H) ; 5,31 (m, 1H) ; 6,42 (d, 1H) ; 6,92 (d, 1H) ; 7,16 (d, 1H) ; 7,4 (d, 1H) ; 7,6 (m, 2H) ; 8,33 (s, 1H) ; 8,82 (s, 1H). |
| 82 | [309] | 4,48 (m, 2H) ; 5,31 (m, 1H) ; 6,43 (d, 1H) ; 6,91 (d, 1H) ; 7,38 (m, 3H) ; 7,65 (m, 2H) ; 8,26 (m, 1H) ; 8,41 (d, 1H) ; 8,92 (s, 1H). |
| 83 | [369] | 4,48 (m, 2H) ; 5,31 (m, 1H) ; 6,45 (d, 1H) ; 6,94 (d, 1H) ; 7,42 (m, 1H) ; 7,51 (m, 1H) ; 7,62 (m, 2H) ; 7,96 (d, 1H) ; 8,15 (s, 1H) ; 8,52 (s, 1H); 8,82 (s, 1H). |
| 84 | [339] | 2,41 (s, 3H), 4,49 (m, 2H) ; 5,31 (m, 1H) ; 6,44 (d, 1H) ; 6,92 (d, 1H) ; 7,45 (m, 1H) ; 7,61 (m, 2H) ; 7,79 (m, 1H) ; 7,98 (m, 1H) ; 8,45 (s, 1H) ; 8,82 (s, 1H). |
| 85 | [369] | 3,25 (s, 3H) , 4,49 (m, 2H) ; 5,31 (m, 1H) ; 6,44 (d, 1H) ; 6,95 (d, 1H) ; 7,69 (m, 2H) ; 8 (d, 2H) ; 8,21 (d, 2H) ; 8,62 (s, 1H) ; 8,87 (s, 1H). |
| 86 | [297] | 4,48 (m, 2H) ; 5,31 (m, 1H) ; 6,44 (d, 1H) ; 6,91 (d, 1H) ; 7,12 (m, 1H) ; 7,51 (m, 2H) ; 7,6 (m, 2H) ; 8,32 (s, 1H) ; 8,81 (s, 1H). |
| 87 | [331] | 4,49 (m, 2H) ; 5,31 (m, 1H) ; 6,45 (d, 1H) ; 6,92 (d, 1H) ; 7,41 (m, 2H) ; 7,66 (m, 3H) ; 8,19 (m, 1H) ; 8,51 (s, 1H) ; 8,56 (s, 1H) ; 8,88 (s, 1H). |
| 88 | [333] | 3,25 (t, 2H) ; 4,49 (m, 2H) ; 4,58 (t, 2H) ; 5,31 (m, 1H) ; 6,45 (d, 1H) ; 6,82 (d, 1H) ; 6,9 (d, 1H) ; 7,57 (m, 2H) ; 7,7 (d, 1H) ; 7,81 (s, 1H) ; 8,29 (s, 1H) ; 8,8 (s, 1H). |
| 89 | [281] | 4,49 (d, 2H) ; 5,31 (t, 1H) ; 6,45 (d, 1H) , 6,6 (m, 1H) ; 6,83 (d, 1H) ; 6,91 (d, 1H) ; 7,61 (m, 2H) ; 7,76 (s, 1H) ; 8,25 (s, 1H) ; 8,88 (s, 1H). |
| 90 | [331] | 4,49 (m, 2H) ; 5,31 (m, 1H) ; 6,45 (d, 1H) , 6,91 (s, 1H) ; 7,02 (s, 1H) ; 7,61 (m, 3H) ; 7,91 (d, 1H) ; 8,01 (s, 1H) ; 8,25 (s, 1H) ; 8,45 (s, 1H) ; 8,85 (s, 1H). |
| 91 | [298] | 4,48 (m, 2H) ; 5,31 (m, 1H) ; 6,44 (d, 1H) ; 6,94 (d, 1H) ; 7,67 (m, 2H) ; 7,75 (d, 1H) ; 7,91 (d, 1H) ; 8,52 (s, 1H) ; 8,91 (s, 1H). |
| 92 | [337] | 3,32 (s, 3H) ; 4,48 (m, 2H) ; 5,31 (m, 1H) ; 6,44 (d, 1H) ; 6,91 (d, 1H) ; 7,32 (d, 2H) ; 7,6 (m, 2H) ; 7,8 (m, 2H) ; 8,41 (s, 1H) ; 8,81 (s, 1H). |
| 93 | [355] | 2,72 (s, 3H) ; 4,7 (d, 2H) ; 5,59 (t, 1H) ; 7,52 (d, 2H) ; 7,62 (m, 3H) ; 7,85 (m, 3H) ; 8,6 (s, 1H). |
| 94 | [327] | 2,76 (s, 3H) ; 4,68 (d, 2H) ; 5,58 (t, 1H) ; 7,18 (m, 1H) ; 7,48 (d, 1H) ; 7,57 (m, 3H) ; 7,62 (m, 1H) ; 7,86 (s, 1H), 8,58 (s, 1H). |
| 95 | [355] | 2,72 (s, 3H) ; 4,68 (d, 2H) ; 5,58 (t, 1H) ; 7,41 (m, 1H) ; 7,51 (m, 1H) ; 7,65 (m, 3H) ; 7,79 (d, 1H) ; 7,86 (m, 2H), 8,61 (s, 1H). |
| 96 | [357] | 3,3 (s, 3H) ; 4,69 (m, 2H) ; 5,59 (m, 1H) ; 7,21 (m, 1H) ; 7,39 (m, 1H) ; 7,66 (m, 4H) ; 7,88 (s, 1H) ; 8,6 (s, 1 H). |
| 97 | [325] | 4,68 (m, 2H) ; 5,57 (m, 1H) ; 7,28 (m, 2H) ; 7,41 (s, 1H) ; 7,61 (m, 2H) ; 7,81 (s, 1H) ; 8 (m, 2H) ; 8,31 (s, 1H) ; 8,9 (s, 1H). |
| 98 | [343] | 4,68 (m, 2H) ; 5,57 (m, 1H) ; 7,42 (s, 1H) ; 7,5 (m, 1H) ; 7,61 (m, 2H) ; 7,8 (m, 2H) ; 7,98 (m, 1H) ; 8,4 (s, 1H) ; 8,9 (s, 1H). |
| 99 | [332] | 4,7 (m, 2H) ; 5,58 (m, 1H) ; 7,48 (s, 1H) ; 7,66 (m, 3H) ; 7,79 (d, 1H) ; 7,85 (s, 1H) ; 8,32 (d, 1H) ; 8,41 (s, 1H) ; 8,5 (s, 1H) ; 8,92 (s, 1H). |
| 100 | [325] | 4,7 (m, 2H) ; 5,58 (m, 1H) ; 7,32 (m, 4H) ; 7,67 (m, 2H) ; 7,8 (s, 1H) ; 8,29 (m, 2H) ; 9 (s, 1H). |
| 101 | [384] | 4,68 (d, 2H) ; 5,57 (m, 1H) ; 7,41 (m, 2H) ; 7,50 (d, 1H) ; 7,64 (m, 2H) ; 7,82 (s, 1H) ; 7,97 (d, 1H) ;8,15(s, 1H) ; 8,43 (s, 1H); 8,88 (s, 1H) |
| 102 | [347] | 4,69 (s, 2H) ; 5,58 (m, 1H) ; 7,31 (m, 3H) ; 7,42 (s, 1H) ; 7,68 (m, 4H) ; 7,82 (s, 1H) ; 8,46 (s, 1H); 8,98 (s, 1H) |
| 103 | [313] | 4,68 (d, 2H) ; 5,57 (m, 1H) ; 7,13 (m, 1H) ; 7,41 (s, 1H) ; 7,57 (m, 4H) ; 7,80 (s, 1H) ; 8,22 (s, 1H) ; 8,88 (s, 1H) |
| 104 | [347] | 4,69 (d, 2H) ; 5,57 (m, 1H) ; 7,41 (m, 3H) ; 7,65 (m, 3H) ; 7,82 (s, 1H) ; 8,21(m, 1H) ; 8,44 (s, 1H); 8,53 (s, 1H); 8,95 (s, 1H) |
| 105 | [361] | 4,33 (s, 3H) ;4,69 (s, 2H) ; 5,58 (m, 1H) ; 7,26 (m, 2H) ; 7,43 (s, 1H) ; 7,65 (dd, 2H) ; 7,75 (s, 2H) ; 7,86 (s, 1H) ; 8,56 (s, 1H); 9,04 (s, 1H) |
| 106 | [349] | 3,25 (t, 2H) ;4,55 (t, 2H) ;4,68 (s, 2H) ; 5,57 (m, 1H) ; 6,81 (d, 1H) ; 7,42 (s, 1H) ; 7,57 (m, 2H) ; 7,71 (d, 1H) ; 7,78 (s, 1H) ; 7,83 (s, 1H) ; 8,20 (s, 1H); 8,86 (s, 1H) |
| 107 | [297] | 4,65 (s, 2H) ; 5,57 (m, 1H) ; 6,60 (m, 1H) ; 6,82 (m, 1H) ; 7,40 (s, 1H) ; 7,57 (d, 1H) ; 7,65 (d, 1H) ; 7,75 (s, 1H) ; 7,80 (s, 1H) ; 8,12 (s, 1H); 8,92 (s, 1H) |
| 108 | [347] | 4,68 (s, 2H) ; 5,57 (m, 1H) ; 7,02 (d, 1H) ; 7,44 (s, 1H) ; 7,64 (m, 3H) ; 7,81 (s, 1H) ; 7,95 (d, 1H) ; 8,02 (s, 1H) ; 8,27 (s, 1H) ; 8,34 (s, 1H); 8,91 (s, 1H) |
| 109 | [314] | 4,68 (s, 2H) ; 5,58 (m, 1H) ; 7,40 (s, 1H) ; 7,68 (dd, 2H) ; 7,75 (d, 1H) ; 7,83 (s, 1H) ; 7,92 (d, 1H) ; 8,40 (s, 1H); 8,98 (s, 1H) |
| 110 | [356] | 2,70 (s, 3H) ; 4,82 (s, 2H) ; 6,18 (m, 1H) ; 7,54 (d, 2H) ; 7,65 (d, 1H) ; 7,85 (m, 3H) ; 8,17 (s, 1H); 8,75 (s, 1H) |
| 11 | [356] | 2,72 (s, 3H) ; 4,82 (s, 2H) ; 6,19 (m, 1H) ; 7,42 (d, 1H) ; 7,52 (t, 1H) ; 7,67 (d, 1H) ; 7,78 (d, 1H) ; 7,85 (m, 2H) ; 8,16 (s, 1H); 8,77 (s, 1H). |
| 112 | [376] | 4,81 (s, 2H) ; 6,19 (m, 1H) ; 7,68 (d, 1H) ; 7,82 (m, 3H) ; 8,10 (s, 1H) ; 8,15 (d, 2H) ; 8,62 (s, 1H); 9,12 (s, 1H) |
| 113 | [326] | 4,81 (s, 2H) ; 6,19 (m, 1H) ; 7,29 (t, 2H) ; 7,65 (d, 1H) ; 7,81 (d, 1H) ; 7,99 (m, 2H) ; 8,10 (s, 1H) ; 8,47 (s, 1H); 9,11 (s, 1H) |
| 114 | [344] | 4,81 (s, 2H) ; 6,19 (m, 1H) ; 7,52 (q, 1H) ; 7,65 (d, 1H) ; 7,80 (m, 1H) ; 7,82 (d, 1H) ; 7,96 (m, 1H) ; 8,12 (s, 1H) ; 8,52 (s, 1H); 9,10 (s, 1H) |
| 115 | [314] | 4,81 (s, 2H) ; 6,19 (m, 1H) ; 7,60 (m, 3H) ; 7,79 (d, 1H) ; 7,90 (d, 1H) ; 8,07 (s, 1H) ; 8,32 (s, 1H); 9,09 (s, 1H) |
| 116 | [309] | 4,82 (s, 2H) ; 6,20 (m, 1H) ; 7,32 (m, 1H) ; 7,67 (d, 1H) ; 7,83 (d, 1H) ; 7,87 (t, 1H) ; 8,10 (m, 2H) ; 8,59 (m, 2H); 9,20 (s, 1H) |
| 117 | [348] | 4,80 (s, 2H) ; 6,20 (m, 1H) ; 7,15 (d, 1H) ; 7,39 (d, 1H) ; 7,61 (d, 1H) ; 7,82 (d, 1H) ; 8,10 (m, 1H) ; 8,39 (s, 1H); 9,08 (s, 1H) |
| 118 | [385] | 4,81 (s, 2H) ; 6,19 (m, 1H) ; 7,42 (t, 1H) ; 7,53 (d, 1H) ; 7,67 (d, 1H) ; 7,84 (d, 1H) ; 7,95 (d, 1H) ; 8,12 (m, 2H) ; 8,57 (s, 1H); 9,08 (s, 1H) |
| 119 | [356] | 2,4 (s, 3H) ; 4,82 (s, 2H) ; 6,20 (m, 1H) ; 7,48 (d, 1H) ; 7,63 (d, 1H) ; 7,80 (m, 2H) ; 7,95 (s, 1H) ; 8,10 (s, 1H) ; 8,47 (s, 1H); 9,08 (s, 1H) |
| 120 | [314] | 4,80 (s, 2H) ; 6,18 (m, 1H) ; 7,12 (m, 1H) ; 7,51 (m, 2H) ; 7,61 (d, 1H) ; 7,80 (d, 1H) ; 8,09 (s, 1H) ; 8,35 (s, 1H); 9,08 (s, 1H) |
| 121 | [347] | 4,82 (s, 2H) ; 6,18 (m, 1H) ; 7,40 (m, 2H) ; 7,67 (m, 2H) ; 7,83 (d, 1H) ; 8,10 (s, 1H) ; 8,19 (m, 1H) ; 8,50 (s, 1H); 8,59 (m, 1H); 9,17 (s, 1H) |
| 122 | [350] | 3,23 (t, 2H) ; 4,57 (t, 2H) ; 4,81 (s, 2H) ; 6,17 (m, 1H) ; 6,82 (d, 1H) ; 7,60 (d, 1H) ; 7,70 (d, 1H) ; 7,75 (d, 1H) ; 7,82 (s, 1H) ; 8,07 (s, 1H) ; 8,33 (s, 1H); 9,07 (s, 1H) |
| 123 | [348] | 4,82 (s, 2H) ; 6,17 (m, 1H) ; 7,03 (s, 1H) ; 7,65 (m, 2H) ; 7,80 (d, 1H) ; 7,92 (d, 1H) ; 8,02 (s, 1H) ; 8,09 (s, 1H) ; 8,35 (s, 1H); 8,49 (s, 1H) ; 9,11 (s, 1H) |
| 124 | [315] | 4,80 (d, 2H) ; 6,18 (t, 1H) ; 7,68 (d, 1H) ; 7,75 (s, 1H) ; 7,90 (m, 2H) ; 8,12 (s, 1H) ; 8,58 (s, 1H); 9,20 (s, 1H) |
| 125 | [321] | 4,48 (s, 2H) ; 4,56 (s, 2H) ; 5,25 (m, 1H) ; 5,31 (m, 1H) ; 6,44 (d, 1H) ; 6,92 (d, 1H) ; 7,28 (d, 1H) ; 7,40 (t, 1H) ; 7,60 (m, 2H) 7,81 (d, 1H) ; 7,95 (s, 1H) ; 8,45 (s, 1H) ; 8,85 (s, 1H) |
| 126 | [376] | 1,89 (s, 3H) ; 4,28 (d, 2H) ; 4,48 (s, 2H) ; 5,32 (m, 1H) ; 6,44 (d, 1H) ; 6,92 (d, 1H) ; 7,32 (d, 2H) ; 7,61 (m, 2H) ; 7,90 (d, 2H) 8,36 (t, 1H) ; 8,42 (s, 1H) ; 8,82 (s, 1H) |
| 127 | [380] | 2,9 (s, 3H) ; 3 (s, 3H) ; 4,5 (s, 2H) ; 5,3 (s, 1H) ; 6,45 (d 1H) ; 6,95 (d, 1H) ; 7,45 (t, 1H) ; 7,65 (m, 2H) ; 7,85 (m, 2H) ; 8,55 (s, 1H) ; 8,85 (s, 1H) |
| 128 | 182-185 | 1,55 (s, 6H) ; 5,5 (s,1H) ; 6,45 (s, 1H) ; 7,4 (m, 2H) ; 7,6 (m, 4H) ; 7,7 (s, 1H) ; 8,05 (s, 1H) ; 8,3 (s, 1H) ; 8,9 (s, 1H) ; 11,15 (s, 1H). |
| 129 | [365] | 2,07 (s, 3H) ; 4,31 (m, 2H) ; 6,18 (m, 1H) ; 7,38 (m, 1H) ; 7,59 (m, 3H) ; 7,67 (d, 1H) ; 7,85 (d, 1H) ; 8,11 (s, 1H) ; 8,21 (s, 1H) ; 8,42 (s, 1H) ; 9,15 (s, 1H). |
| 130 | [338] | 4,53 (m, 2H) ; 4,82 (m, 2H) ; 5,21 (m, 1H) ; 6,18 (m, 1H) ; 7,41 (d, 2H) ; 7,69 (m, 1H) ; 7,9 (m, 3H) ; 8,12 (s, 1H) ; 8,5 (s, 1H) ; 9,12 (s, 1H). |
| 131 | [338] | 4,59 (d, 2H) ; 4,83 (d, 2H) ; 5,28 (t, 1H) ; 6,19 (t, 1H) ; 7,29 (d, 1H) ; 7,4 (m, 1H) ; 7,65 (d, 1H) ; 7,8 (m, 2H) ; 7,94 (s, 1H) ; 8,1 (s, 1H) ; 8,48 (s, 1H) ; 9,1 (s, 1H). |
| 132 | [347] | 4,82 (m, 2H) ; 6,19 (m, 1H) ; 6,52 (s, 1H) ; 7,4 (m, 1H) ; 7,5 (d, 1H) ; 7,7 (m, 2H) ; 7,91 (m, 1H) ; 8,15 (m, 2H) ; 8,48 (s, 1H) ; 9,2 (s, 1H) ; 11,2(s, 1H). |
| 133 | [328] | 2,25 (s, 3H) ; 4,81 (m, 2H) ; 6,18 (m, 1H) ; 7,1 (s, 1H) ; 7,35 (s, 1H) ; 7,61 (d, 1H) ; 7,81 (d, 1H) ; 8,1 (s, 1H) ; 8,32 (s, 1H) ; 9,11 (s, 1H). |
| 134 | [347] | 4,85 (m, 2H) ; 6,2 (m, 1H) ; 7 (s, 1H) ; 7,25 (m, 1H) ; 7,5 (m, 2H) ; 7,6 (d, 2H) ; 7,7 (d, 1H) ; 7,9 (d, 1H) ; 8,11 (d, 1H) ; 9,11 (s, 1H) ; 11,3 (s, 1H). |
| 135 | [327] | 4,83 (d, 2H) ; 6,2 (t, 1H) ; 7,5 (m, 1H) ; 7,7 (d, 1H) ; 7,9 (d, 1H) ; 8,11 (m, 1H) ; 8,21 (d, 1H) ; 8,52 (d, 1H) ; 8,7 (m, 1H) ; 9,25 (s, 1H). |
| 136 | [379] | 3 (pic élargi, 6H) ; 4,81 (d, 2H) ; 6,18 (t, 1H) ; 7,49 (m, 2H) ; 7,65 (d, 1H) ; 7,83 (d, 1H) ; 8 (d, 2H) ; 8,11 (s, 1H) ; 8,52 (s, 1H) ; 9,1 (s, 1H). |
| 137 | [347] | 4,83 (d, 2H) ; 6,18 (t, 1H) ; 6,43 (s, 1H) ; 7,4 (m, 1H) ; 7,6 (m, 3H) ; 7,85 (m, 1H) ; 8,02 (s, 1H) ; 8,11 (s, 1H) ; 8,48 (s, 1H) ; 9,15 (s, 1H) ; 11,3 (s, 1H). |
| 138 | [339] | 4,09 (s, 3H) ; 4,81 (d, 2H) ; 6,18 (t, 1H) ; 7,1 (m, 1H) ; 7,63 (d, 1H) ; 7,81 (d, 1H) ; 8,09 (s, 1H) ; 8,18 (m, 1H) ; 8,55 (m, 2H) ; 9,21 (s, 1H). |
| 139 | [365] | 2,8 (d, 3H) ; 4,81 (m, 2H) ; 6,18 (m, 1H) ; 7,69 (d, 1H) ; 7,9 (m, 3H) ; 8,05 (m, 2H) ; 8,11 (s, 1H) ; 8,48 (m, 1H) ; 8,58 (s, 1H) ; 9,12 (s, 1H). |
| 140 | [328] | 2,48 (s, 3H) ; 4,81 (d, 2H) ; 6,18 (t, 1H) ; 7,18 (m, 1 H) ; 7,62 (d, 1H) ; 7,82 (m, 2H) ; 8,09 (s, 1H) ; 8,3 (s, 1H) ; 9,18 (s, 1H). |
| 141 | [359] | 3,83 (s, 3H) ; 4,82 (m, 2H) ; 6,21 (m, 1H) ; 6,55 (d, 1H) ; 7,4 (d, 1H) ; 7,58 (d, 1H) ; 7,75 (d, 2H) ; 8,02 (m, 1H) ; 8,18 (m, 2H) ; 8,55 (s, 1H) ; 9,2 (s, 1H). |
| 142 | [359] | 4,82 (m, 2H) ; 6,18 (m, 1H) ; 7,6 (m, 1H) ; 7,79 (d, 1H) ; 7,9 (m, 2H) ; 8,06 (d, 1H) ; 8,12 (m, 1H) ; 8,52 (s, 1H) ; 8,95 (m, 1H) ; 9,11 (s, 1H) ; 9,21 (s, 1H) ; 9,35 (d, 1H). |
| 143 | [365] | 2,08 (s, 3H) ; 4,82 (m, 2H) ; 6,18 (m, 1H) ; 7,29 (m, 1H) ; 7,55 (d, 1H) ; 7,68 (d, 2H) ; 7,85 (d, 2H) ; 8,11 (s, 1H) ; 8,42 (s, 1H) ; 9,11 (s, 1H) ; 10,1 (s, 1H). |
| 144 | [393] | 3,2 (m, 4H) ; 3,75 (m, 4H) ; 4,82 (m, 2H) ; 6,2 (m, 1H) ; 7,06 (d, 1H) ; 7,65 (m, 1H) ; 7,8 (d, 2H) ; 7,85 (m, 1H) ; 8,12 (m, 2H) ; 8,4 (m, 1H) ; 9,11 (m, 1H). |
| 145 | [359] | 4,82 (m, 2H) ; 6,18 (m, 1H) ; 7,85 (m, 2H) ; 8,18 (m, 3H) ; 8,6 (m, 2H) ; 8,81 (d, 1H) ; 9,11 (s, 1H) ; 9,25 (s, 1H) ; 9,45 (s, 1H). |
| 146 | [393] | 1,12 (d, 6H) ; 2,62 (m, 1H) ; 4,82 (d, 2H) ; 6,18 (t, 1H) ; 7,35 (m, 1H) ; 7,61 (m, 3H) ; 7,81 (d, 1H) ; 8,09 (s, 1H) ; 8,26 (s, 1H) ; 8,42 (s, 1H) ; 9,12 (s, 1H) ; 10 (s, 1H). |
| 147 | [415] | 2,68 (pic élargi, 6H) ; 4,82 (m, 2H) ; 6,18 (m, 1H) ; 7,69 (m, 3H) ; 7,85 (d, 1H) ; 8,11 (s, 1H) ; 8,26 (d, 1H) ; 8,32 (s, 1H) ; 8,68 (s, 1H) ; 9,12 (m, 1H). |
| 148 | [345] | 4,82 (d, 2H) ; 6,19 (t, 1H) ; 7,63 (s, 2H) ; 7,72 (d, 1H) ; 7,91 (d, 1H) ; 8,18 (s, 1H) ; 8,8 (s, 1H) ; 9,13 (m, 1H). |
| 149 | [368] | 0,92 (d, 6H) ; 1,51 (m, 1H) ; 1,72 (m, 2H) ; 4,2 (m, 2H) ; 4,81 (m, 2H) ; 6,21 (m, 1H) ; 7,79 (d, 1H) ; 7,95 (s, 1H) ; 8,1 (m, 1H) ; 8,21 (s, 1H) ; 8,31 (s, 2H) ; 9,27 (s, 1H). |
| 150 | 185-190 | 1,55 (s, 6H) ; 6,5 (d, 1H) ; 7,4 (d, 1H) ; de 7,55 à 7,8 (m , 6H) ; 8,0 (s, 1H) ; 8,8 (s, 1H) ; 11,4 (s, 1H) ; 12,8 (s, 1H) |
| 151 | 200-205 | 1,6 (s, 6H) ; 5,7 (s, 1H) ; 6,45 (s, 1H) ; 7,4 (m, 1H) ; 7,6 (m, 2H) ; 7,7 (d, 1H) ; 7,9 (d, 1H) ; 8,1 (s, 1H) ; 8,15 (d, 1H) ; 8,5 (s, 1H) ; 8,6 (s, 1H) ; 9,25 (s, 1H) ; 11,2 (s, 1H). |
| 152 | 228-232 | 1,55 (s, 6H) ; 5,35 (s, 1H) ; 6,45 (s, 1H) ; 7,4 (t, 1H) ; 7,45 (d, 1H) ; 7,65 (m, 2H) ; 7,7 (d, 1H) ; 8,0 (d, 1H) ; 8,1 (m, 2H) ; 8,45 (s, 1H) ; 8,6 (d, 1H) ; 9,3 (s, 1H) ; 11,2 (s, 1H). |
| 153 | 255-259 | 1,5 (s, 6H) ; 5,35 (s, 1H) ; 7,25 (t, 1H) ; 7,4 (t, 1H) ; 7,45 (d, 1H) ; 7,6 (d, 1H) ; 7,75 (d, 1H) ; 8,05 (m, 2H) ; 8,5 (d, 1H) ; 8,55 (s, 1H) ; 8,65 (d, 1H) ; 9,4 (s, 1H) ; 13,15 (s, 1H) |
| 154 | 195-198 | 1,55 (s, 6H) ; 2,5 (s, 3H) ; 5,35 (s, 1H) ; 6,7 (s, 1H) ; 7,5 (m, 1H) ; 7,75 (d, 1H) ; 8,05 (m, 2H) ; 8,55 (s, 1H) ; 8,65 (d, 1H) ; 9,4 (s, 1H). |
| 155 | 263-266 | 3,7 (s, 3H) ; 4,55 (d, 2H) ; 5,35 (t, 1H) ; 6,45 (m, 1H) ; 7,05 (s, 1H) ; 7,35 (d, 1H) ; 7,4 (m, 1H) ; 7,65 (m, 3H) ; 8,05 (s, 1H) ; 8,35 (s, 1H) ; 8,65 (s, 1H) ; 11,15 (s, 1H). |
| 156 | 210-210,5 (dec) | 1,55 (s, 6H) ; 5,2 (s, 1H) ; 6,35 (d, 1H) ; 6,9 (d, 1H) ; 7,55 (d, 2H) ; 7,65 (m, 2H) ; 8,0 (d, 2H) ; 8,5 (s, 1H) ; 8,85 (s, 1H). |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,1 nM et 10 µM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 4, 10, 30,36,59 et 64 ont montré une EC₅₀ de respectivement 4,5 ; 2 ; 48 ; 137 ; 74 et 102 nM.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcereuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés de formule (I) : 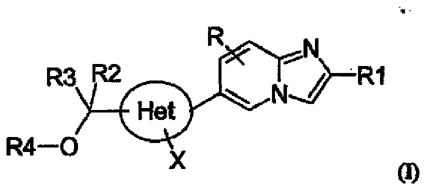 dans laquelle :
Het représente un groupe hétéroaryle monocyclique comportant de 5 à 6 atomes dont de 1 à 3 hetéroatomes choisis parmi N, O et S ;
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes au groupes choisis indépendamment les uns des autres parmi halogène, (C₁-C₁₀)alkyle, (C₁-C₁₀)alkoxy, CONRaRb, NRaRb , (C₁-C₁₀)thioalkyle, -S(O)₂(C₁-C₁₀-alkyle), halo(C₁-C₁₀)alkyle, hydroxy(C₁,C₁₀)alkylène, NRaRb(C₁-C₁₀)alkylène, NRcCORd, SO₂NRaRb, Cyano, nitro ; Ra et Rb représentent, indépendamment l'un de l'autre, un atome d'hydrogène ou un groupe (C₁-C₁₀)alkyle ;
Rc et Rd représentent, indépendamment l'un de l'autre, un atome d'hydrogène ou un groupe (C₁-C₁₀)alkyle.
X représente de 1 à 3 substituants identiques ou différents l'un de l'autre choisis parmi parmi un hydrogène, un halogène, les groupes (C₁-C₁₀)alkyle.
R représente en position 3 de l'imidazo[1,2-α]pyridine 1 substituant choisi parmi un hydrogène, (C₁-C₁₀)alkyle.
R₂ et R₃ indépendamment l'un de l'autre, un atome d'hydrogène ou un groupe (C₁-C₁₀)alkyle.
R₄ représente un atome d'hydrogène ou un groupe (C₁-C₁₀)alkyle.
A l'état de base ou de sel d'addition à un acide.

2. Composés de formule (I) selon l'une quelconque des revendications précédentes, **caractérisé en ce que** :
R₁ représente un groupe phényle, furyle, quinoléinyle, indolyle, pyrrolopyridinyle, pyridinyle, isoquinoléinyle, thiényle, benzofuranyle, benzothiazolyle, benzothiényle, dihydrobenzofuranyle, thiazolyle, pyrazolyle, isoxazolyle, benzimidazolyle, indazolyle, ces groupes pouvant éventuellement être substitués par un ou plusieurs atomes ou groupes choisis indépendamment les uns des autres parmi halogène, méthyle, méthoxy, hydroxyméthyle, -CON(CH₃)₂, morpholinyle, pyrrolidinyl-éthyle, -NHCO-CH(CH₃)₂, - CH₂N(CH₃)₂ , -NH₂, -CONHCH(CH3)₂, pyrrolidinyle, méthylsulfonyle, trifluorométhyle, méthylthio, cyano, nitro, -NHCO(CH₃), CONH(CH₃), CONHC(CH₃) -SO₂N(CH₃)₂, isopentyl ;
Het représente un groupe furyle, thiényle, pyridinyle, thiazolyle, pyrazolyle, imidazolyle ;
X représente de 1 à 3 substituants identiques ou différents l'un de l'autre choisis parmi un hydrogène, un fluor, un groupe méthyle;
R représente en position 3 de l'imidazo[1,2-*a*]pyridine 1 atome d'hydrogène, ou groupe méthyle;
R₂ et R₃ représentent, indépendamment l'un de l'autre, un atome d'hydrogène ou un groupe méthyle;
R₄ représente : un atome d'hydrogène ou un groupe méthyle;
à l'état de base ou de sel d'addition à un acide.

3. Composés de formule (I) selon la revendication 7, **caractérisé en ce que** R représente en position 3, de l'imidazo[1,2-*a*]pyridine 1 atome d'hydrogène, ou 1 groupe méthyle.

4. Composés de formule (I) selon la revendication 1, **caractérisé en ce que**
R₁ représente un groupe phényle, furyle ou quinoléinyle, ces groupes pouvant éventuellement être substitués par un ou plusieurs atomes ou groupes choisis indépendamment les uns des autres parmi halogène, (C₁-C₁₀)alkyle ;
Het représente un groupe furyle, un groupe thiényle, un groupe pyridinyle ou un groupe thiazolyle;
X représente un hydrogène ;
R est un hydrogéne;
R₂ et R₃ représentent, indépendamment l'un de l'autre, un atome d'hydrogène; R₄ représente un atome d'hydrogène,
à l'état de base ou de sel d'addition à un acide.

5. Composés selon la revendication 1, choisis parmi :
• {5-[2-(4-Chlorophényl)imidazo[1,2-*a*]pyridin-6-yl]furan-2-yl}méthanol;
• {4-[2-(4-Chlorophenyl)imidazo[1,2-*a*]pyridin-6-yl]thien-2-yl}méthanol;
• {5-[2-(4-Chlorophényl)imidazo[1,2-*a*]pyridin-6-yl]thién-2-yl}méthanol;
• {2-[2-(4-Chlorophényl)imidazo[1,2-*a*]pyridin-6-yl]pyridin-4-yl}méthanol;
• {5-[2-(4-Chlorophényl)imidazo[1,2-*a*]pyridin-6-yl]pyrindin-3-yl}méthanol;
• {4-[2-(4-Chlorophényl)imidazo[1,2-*a*]pyridin-6-yl]furan-2-yl}methanol,
• {6-[2-(4-Chlorophényl)imidazo[1,2-*a*]pyridin-6-yl]pyridin-2-yl}méthanol;
• {5-[2-(4-Chlorophényl)imidazo[1,2-*a*]pyridin-6-yl]thién-3-yl}méthanol ;
• {2-[2-(4-Chlorophényl)imidazo[1,2-*a*]pyridin-6-yl]thiazol-5-yl}méthanol ;
• {2-[2-(4-Chlorophényl)imidazo[1,2-*a*]pyridin-6-yl]thiazol-4-yl}methanol ;
• {4-[2-(4-Chlorophényl)imidazo[1,2-*a*]pyridin-6-yl]thiazol-2-yl}méthanol ;
• [2-(2-Furan-3-ylimidazo[1,2-*a*]pyridin-6-yl)pyridin-4-yl]méthanol et son chlorhydrate ;
• [5-(2-Quinolin-3-ylimidazo[1,2-*a*]pyridin-6-yl)furan-2-yl]méthanol et son chlorhydrate ;
• [4-(2-pTolylimidazo[1,2-*a*]pyridin-6-yl)thién-2-yl]methanol ;
• [2-(2-Quinoléin-3-ylimidazo[1,2-*a*]pyridin-6-yl)pyridin-4-yl]méthanol et son chlorhydrate;
• [4-(2-Quinoléin-3-ylimidazo[1,2-*a*]pyridin-6-yl)thièn-2-yl]méthanol et son chlorhydrate;
• {4-[2-(1*H*-Indol-5-yl)imidazo[1,2-*a*]pyridin-6-yl]thién-2-yl}méthanol ;
• {5-[2-(1*H*-Indol-5-yl)imidazo[1,2-*a*]pyridin-6-yl]furan-2-yl}méthanol et son Chlorhydrate ;
• [4-(2-Phénylimidazo[1,2-*a*]pyridin-6-yl)thién-2-yl]méthanol et son chlorhydrate;
• {4-[2-(1*H*-Pyrrolo[2,3-b]pyridin-5-yl)imidazo[1,2-*a*]pyridin-6-yl]thién-2-yl}méthanol et son chlorhydrate ;
• {4-[2-(3-Méthoxyphényl)imidazo[1,2-*a*]pyridin-6-yl]thién-2-yl}méthanol et son chlorhydrate ;
• {2-[2-(1*H*-Pyrrolo[2,3-b]pyridin-5-yl)imidazo[1,2-*a*]pyridin-6-yl]pyridin-4-yl}méthanol et son chlorhydrate ;
• [2-(2-Phénylimidazo[1,2-*a*]pyridin-6-yl)pyridin-4-yl]méthanol ;
• [5-(2-pTolylimidazo[1,2-*a*]pyridin-6-yl)furan-2-yl]methanol ;
• [5-(2-Phénylimidazo[1,2-*a*]pyridin-6-yl)furan-2-yl]méthanol ;
• {5-[2-(3-Méthoxyphényl)imidazo[1,2-*a*]pyridin-6-yl]furan-2-yl}méthanol ;
• {5-[2-(2,4-Difluorophényl)imidazo[1,2-*a*]pyridin-6-yl]furan-2-yl}méthanol ;
• {5-[2-(4-Chlorophényl)imidazo[1,2-*a*]pyridin-6-yl]furan-3-yl}methanol ;
• 2-(4-Chlorophényl)-6-(4-méthoxymethyl-furan-2-yl)imidazo[1,2-*a*]pyridine;
• {5-[2-(4-Hydroxyméthyl-phényl)imidazo[1,2-*a*]pyridin-6-yl]furan-2-yl}méthanol;
• 4-[6-(5-Hydroxyméthyl-furan-2-yl)imidazo[1,2-*a*]pyridin-2-yl]-*N,N-*diméthylbenzamide ;
• {5-[2-(1*H*-Indol-6-yl)imidazo[1,2-*a*]pyridin-6-yl]furan-2-yl}méthanol ;
• {5-[2-(2-Méthoxypyridin-3-yl)imidazo[1,2-*a*]pyridin-6-yl]furan-2-yl}méthanol;
• [5-(2-Quinoléin-5-ylimidazo[1,2-*a*]pyridin-6-yl)furan-2-yl]méthanol;
• {5-[2(4-Morpholin-4-ylphényl)imidazo[1,2-*a*]pyridin-6-yl]furan-2-yl}méthanol ;
• [5-(2-Isoquiniléin-5-ylimidazo[1,2-*a*]pyridin-6-yl)furan-2-yl]méthanol ;
• (5-{2-[4-(1-Pyrrolidin-1-yléthyl)phényl]imidazo[1,2-*a*]pyridin-6-yl}furan-2-yl)méthanol;
• 4-[6-(5-Hydroxyméthyl-thién-3-yl)imidazo[1,2-*a*]pyridin-2-yl]-*N,N-*diméthylbenzamide ;
• {4-[2(2-Méthoxypyridin-3-yl)imidazo[1,2-*a*]pyridin-6-yl]thién-2-yl}méthanol ;
• [4-2-Isoquinolin-5-ylimidazo[1,2-*a*]pyridin-6-yl)thién-2-yl]méthanol;
• *N*-{3-[6-(5-Hydroxyméthyl-thién-3-yl)imidazo[1,2-*a*]pyridin-2-yl]phényl}isobutyramide ;
• (4-{2-[4-(1-Pyrrolidin-1-yléthyl)phényl]imidazo[1,2-*a*]pyridin-6-yl}thién-2-yl)méthanol;
• {4-[2-(4-Diméthylaminométhylphényl)imidazo[1,2-*a*]pyridin-6-yl]thién-2-yl}méthanol ;
• 2-Fluoro-4-[6-(5-hydroxyméthyl-thién-3-yl)imidazo[1,2-*a*]pyridin-2-yl]-*N,N-*diméthylbenzamide;
• {4-[2-(3-aminophényl)imidazo[1,2-*a*]pyridin-6-yl]thién-2-yl}méthanol;
• *N*-tertButyl-5-[6-(5-hydroxyméthylthién-3-yl)imidazo[1,2-*a*]pyridin-2-yl]nicotinamide;
• [5-(3-Méthyl-2-phénylimidazo[1,2-*a*]pyridin-6-yl)furan-2-yl]méthanol;
• [5-(3-Méthyl-2-thién-2-ylimidazo[1,2-*a*]pyridin-6-yl)furan-2-yl]méthanol ;
• {5-[2-(3-Chlorophényl)-3-méthylimidazo[1,2-*a*]pyridin-6-yl]furan-2-yl}méthanol ;
• {5-[2-(4-Pyrrolidin-1-ylphényl)imidazo[1,2-*a*]pyridin-6-yl]furan-2-yl}méthanol;
• 3-[6-(5-Hydroxyméthylfuran-2-yl)imidazo[1,2-*a*]pyriidin-2-yl]*benzonitrile ;
• [5-(2-Benzothiazol-2-ylimiazo[1,2-*a*]pyridin-6-yl)furan-2-yl]méthanol;
• [5-(2-Benzothiazol-2-ylimidazo[1,2-*a*]pyridin-6-yl)furan-2-yl]méthanol;
• [5-(2-Benzo[b]thién-2-ylimidazo[1,2-*a*]pyridin-6-yl)furan-2-yl]méthanol;
• {4-[2-(4-Nitrophényl)imidazo[1,2-*a*]pyridin-6-yl]thién-2-yl}méthanol;
• [4-(2-Thién-3-ylimidazo[1,2-*a*]pyridin-6-yl)thién-2-yl]méthanol;
• {4-[2-(4-Méthylthiophényl)imidazo[1,2-*a*]pyridin-6-yl]thién-2-yl}méthanol;
• {4-[2-(5-Chlorothién-2-yl)imidazo[1,2-*a*]pyridin-6-yl]thién-2-yl}métanol;
• {4-[2-(4-Méthylsulfonyl-phényl)imidazo[1,2-*a*]pyridin-6-yl]thién-2-yl}méthanol;
• [4-(2-Benzo[b]thién-2-ylimidazo[1,2-*a*]pyridin-6-yl)thién-2-yl]méthanol;
• [4-(2-Benzo[b]thién-5-ylimidazo[1,2-*a*]pyridin-6-yl)thién-2-yl]méthanol ;
• [4-(3-Méthyl-2-phénylimidazo[1,2-*a*]pyridin-6-yl)thiazol-2-yl]méthanol;
• [4-(3-Méthyl-2-thién-2-ylimidazo[1,2-*a*]pyridin-6-yl)thiazol-2-yl]méthanol;
• {4-[2-(4-Méthoxyphényl)-3-méthylimidazo[1,2-*a*]pyridin-6-yl]thiazol-2-yl}méthanol ;
• {4-[2-(4-Pyrrolidin-1-yl-phényl)imidazo[1,2-*a*]pyridin-6-yl]thiazol-2-yl}méthanol ;
• {4-[2-(2-Fluorophényl)imidazo[1,2-*a*]pyridin-6-yl]thiazol-2-yl}méthanol;
• {4-[2-(4-Méthylsulfonyl-phényl)imidazo[1,2-*a*]pyridin-6-yl]thiazol-2-yl}méthanol ;
• [4-(2-Benzofuran-2-ylimidazo[1,2-*a*]pyridin-6-yl)thiazol-2-yl]méthanol;
• [4-(2-Benzo[b]thién-2-ylimidazo[1,2-*a*]pyridin-6-yl)thiazol-2-yl]méthanol ;
• [4-(2-Furan-2-ylimidazo[1,2-a]pyridin-6-yl)thiazol-2-yl]méthanol;
• [4-(2-Benzo[b]thién-5-ylimidazo[1,2-*a*]pyridin-6-yl)thiazol-2-yl]méthanol;
• {4-[2-(4-Pyrrolidin-1-ylphényl)imidazo[1,2-*a*]pyridin-6-yl]thién-2-yl}méthanol ;
• 1-{4-[2-(4-Chlorophényl)imidazo[1,2-*a*]pyridin-6-yl]thién-2-yl}éthanol;
• 2-{4-[2-(4-Chlorophényl)imidazo[1,2-*a*]pyridin-6-yl]thién-2-yl}propan-2-ol;
• {5-[2-(4-Méthoxyphényl)-3-méthylimidazo[1,2-*a*]pyridin-6-yl]furan-2-yl}méthanol ;
• {5-[2-(4-Trifluorométhylphényl)imidazo[1,2-*a*]pyridin-6-yl]furan-2-yl-méthanol;
• {5-[2-(4-Méthoxyphényl)imidazo[1,2-*a*]pyridin-6-yl]furan-2-yl}méthanol ;
• {5-[2-(4-Fluorophényl)imidazo[1,2-*a*]pyridin-6-yl]furan-2-yl}méthanol;
• {5-[2-(3,4-Difluorophényl)imidazo[1,2-*a*]pyridin-6-yl]furan-2-yl}méthanol ;
• [5-(2-Thién-3-ylimidazo[1,2-*a*]pyridin-6-yl)furan-2-yl]méthanol ;
• {5-[2-(5-(Chlorothién-2-yl)imidazo[1,2-*a*]pyridin-6-yl]furan-2-yl}méthanol ;
• {5-[2-(2-Flurorophényl)imidazo[1,2-*a*]pyridin-6-yl]furan-2-yl}méthanol;
• {5-[2-(3-Bromophényl)imidazo[1,2-*a*]pyridin-6-yl]furan-2-yl}méthanol;
• {5-[2-(4-Chloro-3-méthylphényl)imidazo[1,2-*a*]pyridin-6-yl]furan-2-yl}méthanol;
• {5-[2-(4-Méthylsulfonyl-phényl)imidazo[1,2-*a*]pyridin-6-yl]furan-2-yl}méthanol;
• [5-(2-Thién-2-ylimidazo[1,2-*a*]pyridin-6-yl)furan-2-yl]méthanol ;
• [5-(2-Benzofuran-3-ylimidazo[1,2-*a*]pyridin-6-yl)furan-2-yl]méthanol;
• {5-[2-(2,3-Dihydrobenzofuran-5-yl)imidazo[1,2-*a*]pyridin-6-yl]furan-2-yl}méthanol;
• [5-(2-Furan-2-ylimidazo[1,2-*a*]pyridin-6-yl)furan-2-yl]méthanol;
• [5-(2-Benzofuran-5-ylimidazo[1,2-*a*]pyridin-6-yl)furan-2-yl]méthanol;
• [5(2-Thiazol-2-ylimidazo[1,2-*a*]pyridin-6-yl)furan-2-yl]méthanol;
• {5-[2-(4-Méthylthiophényl)imidazo[1,2-*a*]pyridin-6-yl]furan-2-yl}méthanol;
• {4-[2-(4-Chlorophényl)-3-méthylimidazo[1,2-*a*]pyridin-6-yl]thién-2-yl}méthanol;
• [4-(3-Méthyl-2-thén-2-ylimidazo[1,2-*a*]pyridin-6-yl)thién-2-yl]méthanol;
• {4-[2-(3-Chlorophényl)-3-méthylimidazo[1,2-*a*]pyridin-6-yl]thién-2-yl}méthanol;
• {4-[2-(2,4-Difluorophényl)-3-méthylimidazo[1,2-*a*]pyridin-6-yl]thién-2-yl}méthanol ;
• {4-[2-(4-Flurorophényl)imidazo[1,2-*a*]pyridin-6-yl]thién-2-yl}méthanol;
• {4-[2-(3,4-Difluorophényl)imidazo[1,2-*a*]pyridin-6-yl]thién-2-yl}méthanol;
• 3-[6-(5-Hydroxyméthyl-thién-3-yl)imidazo[1,2-*a*]pyridin-2-yl]benzonitrile;
• {4-[2-(2-Fluorophényl)imidazo[1,2-*a*]pyridin-6-yl]thién-2-yl}méthianol;
• {4-[2(3-Bromophényl)imidazo[1,2-*a*]pyridin-6-yl]thién-2-yl}méthanol;
• [4(2-Benzofuran-2-ylimidazo[1,2-*a*]pyridin-6-yl)thién-2-yl]méthanol;
• [4-(2-Thién-2-ylimidazo[1,2-*a*]pyridin-6-yl)thién-2-yl]méthanol;
• [4-(2-Benzofuran-3-ylimidazo[1,2-*a*]pyridin-6-yl)thién-2-yl]méthanol;
• {4-[2-(1-Méthanol-1*H*-benzimidazol-2-yl)imidazo[1,2-*a*]pyridin-6-yl]thién-2-yl}méthanol;
• {4-[2-(2,3-Dihydrobenzofuran-5-yl)imidazo[1,2-*a*]pyridin-6-yl]thién-2-yl}méthanol;
• [4-(2-Furan-2-ylimidazo[1,2-*a*]pyridin-6-yl)thién-2-yl]méthanol;
• [4-(2-Benzofuran-5-ylimidazo[1,2-*a*]pyridin-6-yl)thién-2-yl]méthanol ;
• [4-(2-Thiazol-2-ylimidazo[1,2-*a*]pyridin-6-yl)thién-2-yl]méthanol;
• {4-[2-(4-Chlorophényl)-3-méthylimidazo[1,2-*a*]pyridin-6-yl]thiazol-2-yl}méthanol;
• {4-[2-(3-Chlorophényl)-3-méthylimidazo[1,2-*a*]pyridin-6-yl]thiazol-2-yl}méthanol ;
• {4-[2-(4-Trifluorométhylphényl)imidazo[1,2-*a*]pyridin-6-yl]thiazol-2-yl}méthanol ;
• {4-[2-(4-Fluorophényl)imidazo[1,2-*a*]pyridin-6-yl]thiazol-2-yl}méthanol;
• {4-[2-(4-Fluorophényl)imidazo[1,2-*a*]pyridin-6-yl]thiazol-2-yl}méthanol;
• {4-[2-(3,4-Difluorophényl)imidazo[1,2-*a*]pyridin-6-yl]thiazol-2-yl}méthanol;
• [4-(2-Thién-3-ylimidazo[1,2-*a*]pyridin-6-yl)thiazol-2-yl]méthanol ;
• [4-(2-Pyridin-2-ylimidazo[1,2-*a*]pyridin-6-yl)thiazol-2-yl]méthanol ;
• {4-[2(5-Chlorothién-2-yl)imidazo[1,2-*a*]pyridin-6-yl]thiazol-2-yl}méthanol ;
• {4-[2-(3-Bromophényl)imidazo[1,2-*a*]pyridin-6-yl]thiazol-2-yl}méthanol ;
• {4-[2(4-Chloro-3-méthylphényl)imidazo[1,2-*a*]pyridin-6-yl]thiazol-2-yl}méthanol ;
• [4-(2-Thién-2-ylimidazo[1,2-*a*]pyridin-6-yl)thiazol-2-yl]méthanol ;
• [4-(2-Benzofuran-3-ylimidazo[1,2-*a*]pyridin-6-yl)thiazol-2-yl]méthanol;
• {4-[2-(2,3-Dihydrobenzofuran-5-yl)imidaza[1,2-*a*]pyridin-6-yl]thiazol-2-yl}méthanol;
• [4-(2-Benzofuran-5-ylimidazo[1,2-*a*]pyridin-6-yl)thiazol-2-yl]méthanol ;
• [4-(2-Thiazol-2-ylimidazo[1,2-*a*]pyridin-6-yl)thiazol-2-yl]méthanol;
• {5-[2-(3-Hydrozyméthylphényl)imidazo[1,2-*a*]pyridin-6-yl]furan-2-yl}méthanol;
• *N*-{3-[6-(5-Hydroxyméthylfuran-2-yl)imidazo[1,2-*a*]pyridin-2-yl]phényl}isobutyramide;
• 2-Fluoro-4-[6-(5-hydroxyméthyl-furan-2-yl)imidazo[1,2-*a*]pyridin-2-yl]-*N,N-*diméthylbenzamide;
• 2-{4-[2-(*1H*-Indol-6-yl)imidazo[1,2-*a*]pyridin-6-yl]thién-2-ylpropan-2-ol;
• N-{3-[6-(2-Hydroxyméthylthiazol-4-yl)imidazo[1,2-*a*]pyridin-2-yl]phényl}acétamide;
• {4-[2-(4-Hydroxyméthylphényl)imidazo[1,2-*a*]pyridin-6-yl]thiazol-2-}méthanol ;
• {4-[2-(3-Hydroxyméthylphényl)imidazo[1,2-*a*]pyridin-6-yl]thiazol-2-yl}méthanol
• {4-[2-(*1H*-Indol-5-yl)imidazo[1,2-*a*]pyridin-6-yl]thiazol-2-yl}méthanol;
• {4-[2-(4-Méthylthién-2-yl)imidazo[1,2-*a*]pyridin-6-yl]thiazol-2-yl}méthanol;
• {4-[2-(*1H*-Indol-4-yl)imidazo[1,2-*a*]pyridin-6-yl]thiazol-2-yl}méthanol ;
• {4-[2-(2-Fluoropyridin-3-yl)imidazo[1,2-*a*]pyridin-6-yl]thiazol-2-yl}méthanol ;
• 3-[6-(2-Hydroxyméthylthiazol-4-yl)imidazo[1,2-*a*]pyridin-2-yl]-*N,N-*diméthylbenzamide ;
• {4-[2-(1H-Indol-6-yl)imidazo[1,2-*a*]pyridin-6-yl]thiazol-2-yl}méthanol ;
• {4-[2-(2-Méthoxypyridin-3-yl)imidazo[1,2-*a*]pyridin-6-yl]thiazol-2-yl}méthanol ;
• 4-[6-(2-Hydroxyméthyl-thiazol-4-yl)imidazo[1,2-*a*]pyridin-2-yl]-*N-*méthylbenzamide;
• {4-[2-(4-Méthylthién-3-yl)imidazo[1,2-*a*]pyridin-6-yl]thiazol-2-yl}méthanol;
• {4-[2(1-Méthyl-*1H*-indol-5-indol-5-yl)imidazo[1,2-*a*]pyridin-6-yl]thiazol-2-yl}méthanol;
• [4-(2-Quinoléin-5-ylimidazo[1,2-*a*]pyridin-6-yl)-thiazol-2-yl]méthanol
• *N*-{4-[6-(2-Hydroxyméthylthiazol-4-yl)imidazo[1,2-*a*]pyridin-2-yl]phenyl}acetamide
• {4-[2-(4-Morpholin-4-ylphényl)imidazo[1,2-*a*]pyridin-6-yl]thiazol-2-yl}méthanol
• [4-(2-Isoquinoléin-5-ylimidazo[1,2-*a*]pyridin-6-yl)thiazol-2-yl]méthanol
• *N*-{3-[6-(2-hydroxyméthyl-thiazol-4-yl)imidazo[1,2-*a*]pyri*din-2-yl]phényl}isobutyramide;
• 3-[6-(2-Hydroxyméthylthiazol-4-yl)imidazo[1,2-*a*]pyridin-2-yl]-*N,N-*diméthylbenzènesulfonamide;
• {4-[2-(2,6-Difluoropyridin-4-yl)imidazo[1,2-*a*]pyridin-6-yl]thiazol-2-yl}méthanol
• (4-{2-[1-(3-Méthylbutyl)-1*H*-pyrazol-4-yl]imidazo[1,2-*a*]pyridin-6-yl}thiazol-2-yl)méthanol;
• 2-{4-[2-(1*H*-Indol-6-yl)imidazo[1,2-*a*]pyridin-6-yl]-1*H*-pyrazol-3-yl}propan-2-ol ;
• 2-{5-Fluoro-2-[2-(1*H*-indol-6-yl)imidazo[1,2-*a*]pyridin-6-yl]pyridin-4-yl}propan-2-ol;
• 2-{2-[2(*1H*-Indol-6-yl)imidazo[1,2-*a*]pyridin-6-yl]pyridin-4-yl}propan-2-ol ;
• 2-{2-[2-(1*H*-Indazol-3-yl)imidazo[1,2-*a*]pyridin-6-yl]pyridin-4-yl}propan-2-ol ;
• 2-{2-[2-(5-Méthylisoxazol-3-yl)imidazo[1,2-*a*]pyridin-6-yl]pyridin-4-yl}propan-2-ol ;
• {5-[2-(*1H*-Indol-6-yl)imidazo[1,2-*a*]pyridin-6-yl]-1-méthyl-*1H*-imidazol-2-yl}méthanol;
• 2-{5-[2-(4-Chlorophényl)imidazo[1,2-*a*]pyridin-6-yl]furan-2-yl}propan-2-ol.

6. Médicament, **caractérisé en ce qu'**il comprend un composé de formule (I) selon l'une quelconque des revendications 1 à 5, ou un sel d'addition de ce composé à un acide pharmaceutiquement acceptable.

7. Composition pharmaceutique, **caractérisée en ce qu'**elle comprend un composé de formule (I) selon l'une quelconque des revendications 1 à 5, ou un sel pharmaceutiquement acceptable, de ce composé, ainsi qu'au moins un excipient pharmaceutiquement acceptable.

8. Utilization d'un composé de formule (I) selon l'une quelconque des revendications 1 à 5 pour la préparation d'un médicammt destiné au traitement et à la prévention des maladies neurodégénératives.

9. Utilisation d'un composé de formule (I) selon l'une quelconque des revendications 1 à 5 pour la préparation d'un médicament destiné au traitement et à la prévention des traumatismes cérébraux et de l'épilepsie.

10. Utilisation d'un composé de formule (I) selon l'une quelconque des revendications 1 à 5 pour la préparation d'un médicament destiné au traitement et à la prévention des maladies psychiatriques.

11. Utilization d'un composé de formule (I) selon l'une quelconque des revendications 1 à 5 pour la préparation d'un médicament destiné au traitement et à la prévention des maladies inflammatoires.

12. Utilisation d'un composé de formule (I) selon l'une quelconque des revendications 1 à 5 pour la préparation d'un médicaments destiné au traitement et à la prévention de l'ostéoporose et les cancers.

13. Utilisation d'un composé de formule (I) selon l'une quelconque des revendications 1 à 5 pour la préparation d'un médicament destiné au traitement et à la prévention de la maladie de Parkinson, d'Alzheimer, des tauopathies, de la sclérose en plaque.

14. Utilisation d'un composé de formule (I) selon l'une quelconque des revendications 1 à 5 pour la préparation d'un médicament destiné au traitement et à la prévention de la schizophrénie, la dépression, la dépendance à une substance, les troubles du déficit de l'attention et de l'hyperactivité.

15. Procédé de synthèse des Composés de formule (I) par réaction de composés de formule (VII) 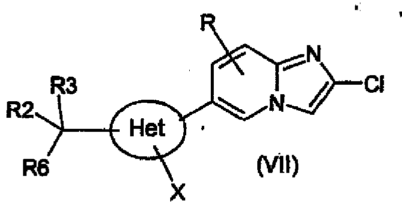 dans laquelle R6 représente un groupement OPG, R2, R3, Het X, R sont tels que définis dans la revendication 1 et GP est un groupement protecteur de la fonction hydroxyle avec R1-Z avec R1 est tel que défini dans la revendication 1 et Z est un dérivé de bore ou d'étain, puis le produit obtenu est déprotégé.

16. Procédé de synthèse des composés de formule (VII) par réaction de composés de formule (V) 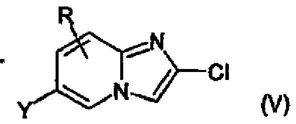 dans laquelle Y est un dérivé de bore, R est tel que défini dans la revendication 1 avec un composé de formule (VI) 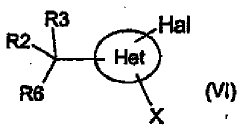 dans laquelle R6 représente un groupement OPG, R2, R3, Het et X sont tels que définis dans la revendication 1 et GP est un groupement protecteur de la fonction hydroxyle.

17. Procédé de synthèse des composés de formule (I) par réaction de composés de formule (II) 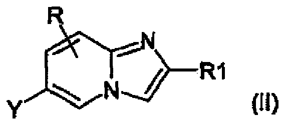 dans laquelle R et R1 sont tels que définies dans la revendication 1 et Y est un dérivé de bore avec un composé de formule (III) 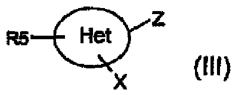 dans laquelle R5 représente un groupement 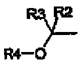 et R2, R3, R4, Het et X sont tels que définis dans la revendication 1 et Z est un halogène.

18. Procédé de synthèse des composés de formule générale (I) selon la revendication 1, **caractérisé en ce que** l'on fait réagir une aminopyridine de formule générale (XI), 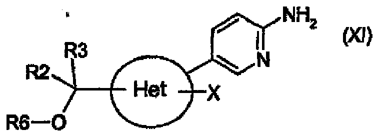 dans laquelle R2, R3 et X sont définis selon la revendication 1, et R6 représente R4, avec une bromocétone de formule générale (XII), 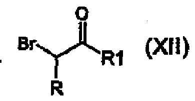 dans laquelle R1 est défini selon la revendication 1 et R représente un atome d'hydrogène ou un groupe (C₁-C₁₀)alkyle.

19. Procédé de synthèse des composés de formule générale (I) selon la revendication 1, **caractérisé en ce que** :
a) on fait réagir une aminopyridine de formule générale (XI), 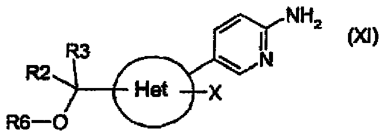 dans laquelle R2, R3 et X sont définis selon la revendication 1, et R6 représente R4, avec une bromocétone de formule générale (XII), 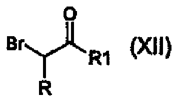 dans laquelle R1 est défini selon la revendication 1 et R représente un atome d'hydrogène ou un groupe (C₁-C₁₀)alkyle pour obtenir un composé de formule générale (XII) 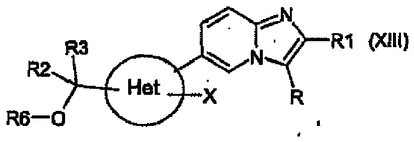 dans laquelle R6 représente un groupement protecteur de fonction hydroxyle PG ;
b) on soumet le compose de formule générale (XIII) obtenu à l'étape b), à une réaction de déprotection.

20. Composés intermédiaires

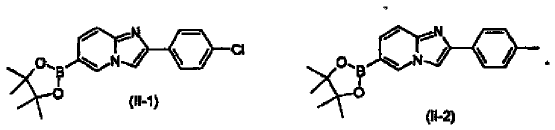

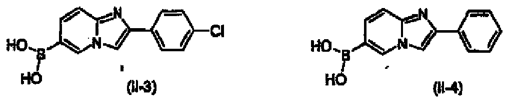

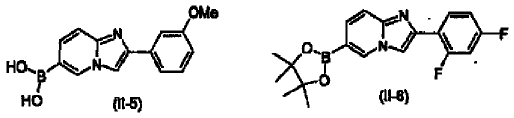

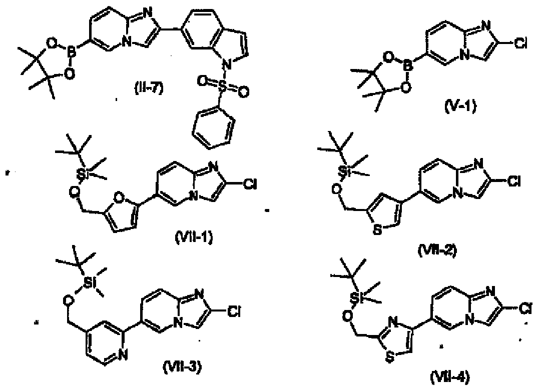

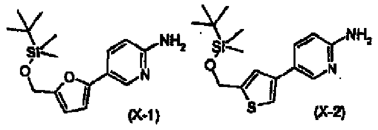

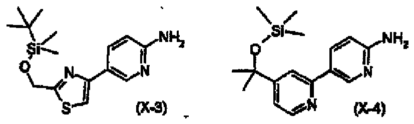

21. Procédé de synthèse selon l'une quelconque des revendications 15 à 19 où les composés de formules générales (II), (V), (VII) et (X) sont les composés de formules générales (II-1), (II-2), (II-3), (II-4), (II-5), (II-6), (II-7), (V-1), (VII-1), (VII-2), (VII-3), (VII-4), (X-1), (X-2) (X-3) et (X-4).

## Patentansprüche

1. Verbindungen der Formel (I): 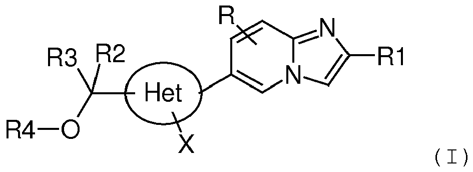 worin:
Het für eine monocyclische Heteroarylgruppe mit 5 bis 6 Atomen, darunter 1 bis 3 Heteroatome, die aus N, O und S ausgewählt sind, steht;
R₁ für eine Phenyl-, Furyl-, Chinolinyl-, Indolyl-, Pyrrolopyridinyl-, Pyridinyl-, Isochinolinyl-, Thienyl-, Benzofuranyl-, Benzothiazolyl-, Benzothienyl-, Dihydrobenzofuranyl-, Thiazolyl-, Pyrazolyl-, Isoxazolyl-, Benzimidazolyl- oder Indazolylgruppe steht, wobei diese Gruppen gegebenenfalls durch ein oder mehrere Atome bzw. eine oder mehrere Gruppen substituiert sein können, die unabhängig voneinander aus Halogen, (C₁-C₁₀)-Alkyl, (C₁-C₁₀)-Alkoxy, CONRaRb, NRaRb, (C₁-C₁₀)-Thioalkyl, -S(O)₂(C₁-C₁₀-Alkyl), Halogen-(C₁-C₁₀)-alkyl, Hydroxy-(C₁-C₁₀)-alkylen, NRaRb-(C₁-C₁₀)-Alkylen, NRcCORd, SO₂NRaRb, Cyano und Nitro ausgewählt sind;
Ra und Rb unabhängig voneinander für ein Wasserstoffatom oder eine C₁-C₁₀-Alkylgruppe stehen;
Rc und Rd unabhängig voneinander für ein Wasserstoffatom oder eine (C₁-C₁₀)-Alkylgruppe stehen;
X für 1 bis 3 gleiche oder voneinander verschiedene Substituenten, die aus einem Wasserstoff, einem Halogen und (C₁-C₁₀)-Alkylgruppen ausgewählt sind, steht;
R für 1 aus Wasserstoff und (C₁-C₁₀)-Alkyl ausgewählten Substituenten in 3-Position des Imidazo[1,2-a]pyridins steht;
R₂ und R₃ unabhängig voneinander für ein Wasserstoffatom oder eine (C₁-C₁₀)-Alkylgruppe stehen;
R₄ für ein Wasserstoffatom oder eine (C₁-C₁₀) - Alkylgruppe steht;
in Basen- oder Säureadditionssalzform.

2. Verbindungen der Formel (I) nach einem der vorhergehenden Ansprüche, **dadurch gekennzeichnet, dass**:
R₁ für eine Phenyl-, Furyl-, Chinolinyl-, Indolyl-, Pyrrolopyridinyl-, Pyridinyl-, Isochinolinyl-, Thienyl-, Benzofuranyl-, Benzothiazolyl-, Benzothienyl-, Dihydrobenzofuranyl-, Thiazolyl-, Pyrazolyl-, Isoxazolyl-, Benzimidazolyl- oder Indazolylgruppe steht, wobei diese Gruppen gegebenenfalls durch ein oder mehrere Atome bzw. eine oder mehrere Gruppen substituiert sein können, die unabhängig voneinander aus Halogen, Methyl, Methoxy, Hydroxymethyl, -CON(CH₃)₂, Morpholinyl, Pyrrolidinylethyl, -NHCO-CH(CH₃)₂, -CH₂N (CH₃)₂, -NH₂, -CONHCH(CH₃)₂, Pyrrolidinyl, Methylsulfonyl, Trifluormethyl, Methylthio, Cyano, Nitro, -NHCO(CH₃), CONH(CH₃), CONHC(CH₃)₃, -SO₂N(CH₃)₂ und Isopentyl ausgewählt sind;
Het für eine Furyl-, Thienyl-, Pyridinyl-, Thiazolyl-, Pyrazolyl- oder Imidazolylgruppe steht;
X für 1 bis 3 gleiche oder voneinander verschiedene Substituenten, die aus einem Wasserstoff, einem Fluor und einer Methylgruppe ausgewählt sind, steht;
R für 1 Wasserstoffatom oder Methylgruppe in 3-Position des Imidazo[1,2-a]pyridins steht;
R₂ und R₂ unabhängig voneinander für ein Wasserstoffatom oder eine Methylgruppe stehen;
R₄ für ein Wasserstoffatom oder eine Methylgruppe steht;
in Basen- oder Säureadditionssalzform.

3. Verbindung der Formel (I) nach Anspruch 7, **dadurch gekennzeichnet, dass** R in 3-Position des Imidazo[1,2-a]pyridins für 1 Wasserstoffatom oder 1 Methylgruppe steht.

4. Verbindung der Formel (I) nach Anspruch 1, **dadurch gekennzeichnet, dass**
R₁ für eine Phenyl-, Furyl- oder Chinolinylgruppe steht, wobei diese Gruppen gegebenenfalls durch ein oder mehrere Atome bzw. eine oder mehrere Gruppen substituiert sein können, die unabhängig voneinander aus Halogen und (C₁-C₁₀)-Alkyl ausgewählt sind;
Het für eine Furylgruppe, eine Thienylgruppe, eine Pyridinylgruppe oder eine Thiazolylgruppe steht;
X für einen Wasserstoff steht;
R für einen Wasserstoff steht;
R₂ und R₂ unabhängig voneinander für ein Wasserstoffatom stehen;
R₄ für ein Wasserstoffatom steht;
in Basen- oder Säureadditionssalzform.

5. Verbindungen nach Anspruch 1, ausgewählt aus:
• {5-[2-(4-Chlorphenyl)imidazo[1,2-*a*]pyridin-6-yl]furan-2-yl}methanol;
• {4-[2-(4-Chlorphenyl)imidazo[1,2-*a*]pyridin-6-yl]thien-2-yl}methanol;
• {5-[2-(4-Chlorphenyl)imidazo[1,2-*a*]pyridin-6-yl]thien-2-yl}methanol;
• {2-[2-(4-Chlorphenyl)imidazo[1,2-*a*]pyridin-6-yl]pyridin-4-yl}methanol;
• {5-[2-(4-Chlorphenyl)imidazo[1,2-*a*]pyridin-6-yl]pyridin-3-yl}methanol;
• {4-[2-(4-Chlorphenyl)imidazo[1,2-*a*]pyridin-6-yl]furan-2-yl}methanol;
• {6-[2-(4-Chlorphenyl)imidazo[1,2-*a*]pyridin-6-yl]pyridin-2-yl}methanol;
• {5-[2-(4-Chlorphenyl)imidazo[1,2-*a*]pyridin-6-yl]thien-3-yl}methanol;
• {2-[2-(4-Chlorphenyl)imidazo[1,2-*a*]pyridin-6-yl]thiazol-5-yl}methanol;
• {2-[2-(4-Chlorphenyl)imidazo[1,2-*a*]pyridin-6-yl]thiazol-4-yl}methanol;
• {4-[2-(4-Chlorphenyl)imidazo[1,2-*a*]pyridin-6-yl]thiazol-2-yl}methanol;
• [2-(2-Furan-3-ylimidazo[1,2-*a*]pyridin-6-yl)pyridin-4-yl]methanol und dessen Hydrochlorid;
• [5-(2-Chinolin-3-ylimidazo[1,2-*a*]pyridin-6-yl)furan-2-yl]methanol und dessen Hydrochlorid;
• [4-(2-*p*-Tolylimidazo[1,2-*a*]pyridin-6-yl)thien-2-yl]methanol;
• [2-(2-Chinolin-3-ylimidazo[1,2-*a*]pyridin-6-yl)pyridin-4-yl]methanol und dessen Hydrochlorid;
• [4-(2-Chinolin-3-ylimidazo[1,2-*a*]pyridin-6-yl)thien-2-yl]methanol und dessen Hydrochlorid;
• {4-[2-(1*H*-Indol-5-yl)imidazo[1,2-*a*]pyridin-6-yl]thien-2-yl}methanol;
• {5-[2-(1*H*-Indol-5-yl)imidazo[1,2-*a*]pyridin-6-yl]furan-2-yl}methanol und dessen Hydrochlorid;
• [4-(2-Phenylimidazo[1,2-*a*]pyridin-6-yl)thien-2-yl]methanol und dessen Hydrochlorid;
• {4-[2-(1*H*-Pyrrolo[2,3-b]pyridin-5-yl)imidazo[1,2-*a*]pyridin-6-yl]thien-2-yl}methanol und dessen Hydrochlorid;
• {4-[2-(3-Methoxyphenyl)imidazo[1,2-*a*]pyridin-6-yl]thien-2-yl}methanol und dessen Hydrochlorid;
• {2-[2-(1*H*-Pyrrolo[2,3-b]pyridin-5-yl)imidazo[1,2-*a*]pyridin-6-yl]pyridin-4-yl}methanol und dessen Hydrochlorid;
• [2-(2-Phenylimidazo[1,2-*a*]pyridin-6-yl)pyridin-4-yl]methanol;
• [5-(2-p-Tolylimidazo[1,2-*a*]pyridin-6-yl)furan-2-yl]methanol;
• [5-(2-Phenylimidazo[1,2-*a*]pyridin-6-yl)furan-2-yl]methanol;
• {5-[2-(3-Methoxyphenyl)imidazo[1,2-*a*]pyridin-6-yl]furan-2-yl}methanol;
• {5-[2-(2,4-Difluorphenyl)imidazo[1,2-*a*]pyridin-6-yl]furan-2-yl}methanol;
• {5-[2-(4-Chlorphenyl)imidazo[1,2-*a*]pyridin-6-yl]furan-3-yl}methanol;
• 2-(4-Chlorphenyl)-6-(4-methoxymethyl-furan-2-yl)imidazo[1,2-*a*]pyridin;
• {5-[2-(4-Hydroxymethylphenyl)imidazo[1,2-*a*]pyridin-6-yl]furan-2-yl}methanol;
• 4-[6-(5-Hydroxymethylfuran-2-yl)imidazo[1,2-*a*]pyridin-2-yl]-*N,N*-dimethylbenzamid;
• {5-[2-(1*H*-Indol-6-yl)imidazo[1,2-*a*]pyridin-6-yl]furan-2-yl}methanol;
• {5-[2-(2-Methoxypyridin-3-yl)imidazo[1,2-*a*]pyridin-6-yl]furan-2-yl}methanol;
• [5-(2-Chinolin-5-ylimidazo[1,2-*a*]pyridin-6-yl)furan-2-yl]methanol;
• {5-[2-(4-Morpholin-4-ylphenyl)imidazo[1,2-*a*]pyridin-6-yl]furan-2-yl}methanol;
• [5-(2-Isochinolin-5-ylimidazo[1,2-*a*]pyridin-6-yl)furan-2-yl]methanol;
• (5-{2-[4-(1-Pyrrolidin-1-ylethyl)phenyl]imidazo[1,2-*a*]pyridin-6-yl}furan-2-yl)methanol;
• 4-[6-(5-Hydroxymethylthien-3-yl)imidazo[1,2-*a*]pyridin-2-yl]-*N,N*-dimethylbenzamid;
• {4-[2-(2-Methoxypyridin-3-yl)imidazo[1,2-*a*]pyridin-6-yl]thien-2-yl}methanol;
• [4-(2-Isochinolin-5-ylimidazo[1,2-*a*]pyridin-6-yl)thien-2-yl]methanol;
• *N*-{3-[6-(5-Hydroxymethylthien-3-yl)imidazo[1,2-*a*]pyridin-2-yl]phenyl}isobutyramid;
• (4-{2-[4-(1-Pyrrolidin-1-ylethyl)phenyl]imidazo[1,2-*a*]pyridin-6-yl}thien-2-yl)methanol;
• {4-[2-(4-Dimethylaminomethylphenyl)imidazo[1,2-*a*]pyridin-6-yl]thien-2-yl}methanol;
• 2-Fluor-4-[6-(5-hydroxymethylthien-3-yl)imidazo[1,2-*a*]pyridin-2-yl]-N,N-dimethylbenzamid;
• {4-[2-(3-Aminophenyl)imidazo[1,2-*a*]pyridin-6-yl]thien-2-yl}methanol;
• *N*-tert-Butyl-5-[6-(5-hydroxymethylthien-3-yl)imidazo[1,2-a]pyridin-2-yl]nicotinamid;
• [5-(3-Methyl-2-phenylimidazo[1,2-*a*]pyridin-6-yl)furan-2-yl]methanol;
• [5-(3-Methyl-2-thien-2-ylimidazo[1,2-*a*]pyridin-6-yl)furan-2-yl]methanol;
• {5-[2-(3-Chlorphenyl)-3-methylimidazo[1,2-*a*]pyridin-6-yl]furan-2-yl}methanol;
• {5-[2-(4-Pyrrolidin-1-ylphenyl)imidazo[1,2-*a*]pyridin-6-yl]furan-2-yl}methanol;
• 3-[6-(5-Hydroxymethylfuran-2-yl)imidazo[1,2-*a*]pyridin-2-yl]benzonitril;
• [5-(2-Benzofuran-2-ylimidazo[1,2-*a*]pyridin-6-yl)furan-2-yl]methanol;
• [5-(2-Benzothiazol-2-ylimidazo[1,2-*a*]pyridin-6-yl)furan-2-yl]methanol;
• [5-(2-Benzo[b]thien-2-ylimidazo[1,2-*a*]pyridin-6-yl)furan-2-yl]methanol;
• {4-[2-(4-Nitrophenyl)imidazo[1,2-*a*]pyridin-6-yl]thien-2-yl}methanol;
• [4-(2-Thien-3-ylimidazo[1,2-*a*]pyridin-6-yl)thien-2-yl]methanol;
• {4-[2-(4-Methylthiophenyl)imidazo[1,2-*a*]pyridin-6-yl]thien-2-yl}methanol;
• {4-[2-(5 Chlorthien-2-yl)imidazo[1,2-*a*]pyridin-6-yl]thien-2-yl}methanol;
• {4-[2-(4-Methylsulfonylphenyl)imidazo[1,2-*a*]pyridin-6-yl]thien-2-yl}methanol;
• [4-(2-Benzo[b]thien-2-ylimidazo[1,2-*a*]pyridin-6-yl)thien-2-yl]methanol;
• [4-(2-Benzo[b]thien-5-ylimidazo[1,2-*a*]pyridin-6-yl)thien-2-yl]methanol;
• [4-(3-Methyl-2-phenylimidazo[1,2-*a*]pyridin-6-yl)thiazol-2-yl]methanol;
• [4-(3-Methyl-2-thien-2-ylimidazo[1,2-*a*]pyridin-6-yl)thiazol-2-yl]methanol;
• {4-[2-(4-Methoxyphenyl)-3-methylimidazo[1,2-*a*]pyridin-6-yl]thiazol-2-yl}methanol;
• {4-[2-(4-Pyrrolidin-1-ylphenyl)imidazo[1,2-*a*]pyridin-6-yl]thiazol-2-yl}methanol;
• {4-[2-(2-Fluorphenyl)imidazo[1,2-*a*]pyridin-6-yl]thiazol-2-yl}methanol;
• {4-[2-(4-Methylsulfonylphenyl)imidazo[1,2-*a*]pyridin-6-yl]thiazol-2-yl}methanol;
• [4-(2-Benzofuran-2-ylimidazo[1,2-*a*]pyridin-6-yl)thiazol-2-yl]methanol;
• [4-(2-Benzo[b]thien-2-ylimidazo[1,2-*a*]pyridin-6-yl)thiazol-2-yl]methanol;
• [4-(2-Furan-2-ylimidazo[1,2-*a*]pyridin-6-yl)thiazol-2-yl]methanol;
• [4-(2-Benzo[b]thien-5-ylimidazo[1,2-*a*]pyridin-6-yl)thiazol-2-yl]methanol;
• {4-[2-(4-Pyrrolidin-1-ylphenyl)imidazo[1,2-*a*]pyridin-6-yl]thien-2-yl}methanol;
• 1-{4-[2-(4-Chlorphenyl)imidazo[1,2-*a*]pyridin-6-yl]thien-2-yl}ethanol;
• 2-{4-[2-(4-Chlorphenyl)imidazo[1,2-*a*]pyridin-6-yl]thien-2-yl}propan-2-ol;
• {5-[2-(4-Methoxyphenyl)-3-methylimidazo[1,2-*a*]pyridin-6-yl]furan-2-yl}methanol;
• {5-[2-(4-Trifluormethylphenyl)imidazo[1,2-*a*]pyridin-6-yl]furan-2-yl-methanol;
• {5-[2-(4-Methoxyphenyl)imidazo[1,2-*a*]pyridin-6-yl]furan-2-yl}methanol;
• {5-[2-(4-Fluorphenyl)imidazo[1,2-*a*]pyridin-6-yl]furan-2-yl}methanol;
• {5-[2-(3,4-Difluorphenyl)imidazo[1,2-*a*]pyridin-6-yl]furan-2-yl}methanol;
• [5-(2-Thien-3-ylimidazo[1,2-*a*]pyridin-6-yl)furan-2-yl]methanol;
• {5-[2-(5 Chlorthien-2-yl)imidazo[1,2-*a*]pyridin-6-yl]furan-2-yl}methanol;
• {5-[2-(2-Fluorphenyl)imidazo[1,2-*a*]pyridin-6-yl]furan-2-yl}methanol;
• {5-[2-(3-Bromphenyl)imidazo[1,2-*a*]pyridin-6-yl]furan-2-yl}methanol;
• {5-[2-(4-Chlor-3-methylphenyl)imidazo[1,2-*a*]pyridin-6-yl]furan-2-yl}methanol;
• {5-[2-(4-Methylsulfonylphenyl)imidazo[1,2-*a*]pyridin-6-yl]furan-2-yl}methanol;
• [5-(2-Thien-2-ylimidazo[1,2-*a*]pyridin-6-yl)furan-2-yl]methanol;
• [5-(2-Benzofuran-3-ylimidazo[1,2-*a*]pyridin-6-yl)furan-2-yl]methanol;
• {5-[2-(2,3-Dihydrobenzofuran-5-yl)imidazo[1,2-*a*]pyridin-6-yl]furan-2-yl}methanol;
• [5-(2-Furan-2-ylimidazo[1,2-*a*]pyridin-6-yl)furan-2-yl]methanol;
• [5-(2-Benzofuran-5-ylimidazo[1,2-*a*]pyridin-6-yl)furan-2-yl]methanol;
• [5-(2-Thiazol-2-ylimidazo[1,2-*a*]pyridin-6-yl)furan-2-yl]methanol;
• {5-[2-(4-Methylthiophenyl)imidazo[1,2-*a*]pyridin-6-yl]furan-2-yl}methanol;
• {4-[2-(4-Chlorphenyl)-3-methylimidazo[1,2-*a*]pyridin-6-yl]thien-2-yl}methanol;
• [4-(3-Methyl-2-thien-2-ylimidazo[1,2-*a*]pyridin-6-yl)thien-2-yl]methanol;
• {4-[2-(3 Chlorphenyl)-3-methylimidazo[1,2-*a*]pyridin-6-yl]thien-2-yl}methanol;
• {4-[2-(2,4-Difluorphenyl)-3-methylimidazo[1,2-*a*]pyridin-6-yl]thien-2-yl}methanol;
• {4-[2-(4-Fluorphenyl)imidazo[1,2-*a*]pyridin-6-yl]thien-2-yl}methanol;
• {4-[2-(3,4-Difluorphenyl)imidazo[1,2-*a*]pyridin-6-yl]thien-2-yl}methanol;
• 3-[6-(5-Hydroxymethylthien-3-yl)imidazo[1,2-*a*]pyridin-2-yl]benzonitril;
• {4-[2-(2-Fluorphenyl)imidazo[1,2-*a*]pyridin-6-yl]thien-2-yl}methanol;
• {4-[2-(3-Bromphenyl)imidazo[1,2-*a*]pyridin-6-yl]thien-2-yl}methanol;
• [4-(2-Benzofuran-2-ylimidazo[1,2-*a*]pyridin-6-yl)thien-2-yl]methanol;
• [4-(2-Thien-2-ylimidazo[1,2-*a*]pyridin-6-yl)thien-2-yl]methanol;
• [4-(2-Benzofuran-3-ylimidazo[1,2-*a*]pyridin-6-yl)thien-2-yl]methanol;
• {4-[2-(1-Methyl-1*H*-benzimidazol-2-yl)imidazo[1,2-*a*]pyridin-6-yl]thien-2-yl}methanol;
• {4-[2-(2,3-Dihydrobenzofuran-5-yl)imidazo[1,2-*a*]pyridin-6-yl]thien-2-yl}methanol;
• [4-(2-Furan-2-ylimidazo[1,2-*a*]pyridin-6-yl)thien-2-yl]methanol;
• [4-(2-Benzofuran-5-ylimidazo[1,2-*a*]pyridin-6-yl)thien-2-yl]methanol;
• [4-(2-Thiazol-2-ylimidazo[1,2-*a*]pyridin-6-yl)thien-2-yl]methanol;
• {4-[2-(4-Chlorphenyl)-3-methylimidazo[1,2-*a*]pyridin-6-yl]thiazol-2-yl}methanol;
• {4-[2-(3-Chlorphenyl)-3-methylimidazo[1,2-*a*]pyridin-6-yl]thiazol-2-yl}methanol;
• {4-[2-(4-Trifluormethylphenyl)imidazo[1,2-*a*]pyridin-6-yl]thiazol-2-yl}methanol;
• {4-[2-(4-Fluorphenyl)imidazo[1,2-*a*]pyridin-6-yl]thiazol-2-yl}methanol;
• {4-[2-(3,4-Difluorphenyl)imidazo[1,2-*a*]pyridin-6-yl]thiazol-2-yl}methanol;
• [4-(2-Thien-3-ylimidazo[1,2-*a*]pyridin-6-yl)thiazol-2-yl]methanol;
• [4-(2-Pyridin-2-ylimidazo[1,2-*a*]pyridin-6-yl)thiazol-2-yl]methanol;
• {4-[2-(5-Chlorthien-2-yl)imidazo[1,2-*a*]pyridin-6-yl]thiazol-2-yl}methanol;
• {4-[2-(3-Bromphenyl)imidazo[1,2-*a*]pyridin-6-yl]thiazol-2-yl}methanol;
• {4-[2-(4-Chlor-3-methylphenyl)imidazo[1,2-*a*]pyridin-6-yl]thiazol-2-yl}methanol;
• [4-(2-Thien-2-ylimidazo[1,2-*a*]pyridin-6-yl)thiazol-2-yl]methanol;
• [4-(2-Benzofuran-3-ylimidazo[1,2-*a*]pyridin-6-yl)thiazol-2-yl]methanol;
• {4-[2-(2,3-Dihydrobenzofuran-5-yl)imidazo[1,2-*a*]pyridin-6-yl]thiazol-2-yl}methanol;
• [4-(2-Benzofuran-5-ylimidazo[1,2-*a*]pyridin-6-yl)thiazol-2-yl]methanol;
• [4-(2-Thiazol-2-ylimidazo[1,2-*a*]pyridin-6-yl)thiazol-2-yl]methanol;
• {5-[2-(3-Hydroxymethylphenyl)imidazo[1,2-*a*]pyridin-6-yl]furan-2-yl}methanol;
• N-{3-[6-(5-Hydroxymethylfuran-2-yl)imidazo[1,2-*a*]pyridin-2-yl]phenyl}isobutyramid;
• 2-Fluor-4-[6-(5-hydroxymethylfuran-2-yl)imidazo[1,2-*a*]pyridin-2-yl]-N,N-dimethylbenzamid;
• 2-{4-[2-(1*H*-Indol-6-yl)imidazo[1,2-a]pyridin-6-yl]thien-2-ylpropan-2-ol;
• N-{3-[6-(2-Hydroxymethylthiazol-4-yl)imidazo[1,2-*a*]pyridin-2-yl]phenyl}acetamid;
• {4-[2-(4-Hydroxymethylphenyl)imidazo[1,2-*a*]pyridin-6-yl]thiazol-2-yl}methanol;
• {4-[2-(3-Hydroxymethylphenyl)imidazo[1,2-*a*]pyridin-6-yl]thiazol-2-yl}methanol;
• {4-[2-(1*H*-Indol-5-yl)imidazo[1,2-*a*]pyridin-6-yl]thiazol-2-yl}methanol;
• {4-[2-(4-Methylthien-2-yl)imidazo[1,2-*a*]pyridin-6-yl]thiazol-2-yl}methanol;
• {4-[2-(1*H*-Indol-4-yl)imidazo[1,2-*a*]pyridin-6-yl]thiazol-2-yl}methanol;
• {4-[2-(2-Fluorpyridin-3-yl)imidazo[1,2-*a*]pyridin-6-yl]thiazol-2-yl}methanol;
• 3-[6-(2-Hydroxymethylthiazol-4-yl)imidazo[1,2-*a*]pyridin-2-yl]-N,N-dimethylbenzamid;
• {4-[2-(1H-Indol-6-yl)imidazo[1,2-*a*]pyridin-6-yl]thiazol-2-yl}methanol;
• {4-[2-(2-Methoxypyridin-3-yl)imidazo[1,2-*a*]pyridin-6-yl]thiazol-2-yl}methanol;
• 4-[6-(2-Hydroxymethylthiazol-4-yl)imidazo[1,2-*a*]pyridin-2-yl]-N-methylbenzamid;
• {4-[2-(4-Methylthien-3-yl)imidazo[1,2-*a*]pyridin-6-yl]thiazol-2-yl}methanol;
• {4-[2-(1-Methyl-1*H*-indol-5-yl)imidazo[1,2-*a*]pyridin-6-yl]thiazol-2-yl}methanol;
• [4-(2-Chinolin-5-ylimidazo[1,2-*a*]pyridin-6-yl)-thiazol-2-yl]methanol;
• N-{4-[6-(2-Hydroxymethylthiazol-4-yl)imidazo[1,2-*a*]pyridin-2-yl]phenyl}acetamid;
• {4-[2-(4-Morpholin-4-ylphenyl)imidazo[1,2-*a*]pyridin-6-yl]thiazol-2-yl}methanol;
• [4-(2-Isochinolin-5-ylimidazo[1,2-*a*]pyridin-6-yl)thiazol-2-yl]methanol;
• *N*-{3-[6-(2-Hydroxymethyl-thiazol-4-yl)imidazo[1,2-a]pyridin-2-yl]phenyl}isobutyramid;
• 3-[6-(2-Hydroxymethylthiazol-4-yl)imidazo[1,2-*a*]pyridin-2-yl]-N,N-dimethylbenzolsulfonamid;
• {4-[2-(2,6-Difluorpyridin-4-yl)imidazo[1,2-*a*]pyridin-6-yl]thiazol-2-yl}methanol;
• (4-{2-[1-(3-Methylbutyl)-1*H*-pyrazol-4-yl]imidazo[1,2-a]pyridin-6-yl}thiazol-2-yl)methanol;
• 2-{4-[2-(1*H*-Indol-6-yl)imidazo[1,2-*a*]pyridin-6-yl]-1*H*-pyrazol-3-yl}propan-2-ol;
• 2-{5-Fluor-2-[2-(1*H*-indol-6-yl)imidazo[1,2-*a*]pyridin-6-yl]pyridin-4-yl}propan-2-ol;
• 2-{2-[2-(1*H*-Indol-6-yl)imidazo[1,2-*a*]pyridin-6-yl]pyridin-4-yl}propan-2-ol;
• 2-{2-[2-(1*H*-Indazol-3-yl)imidazo[1,2-*a*]pyridin-6-yl]pyridin-4-yl}propan-2-ol;
• 2-{2-[2-(5-Methylisoxazol-3-yl)imidazo[1,2-*a*]pyridin-6-yl]pyridin-4-yl}propan-2-ol;
• {5-[2-(1*H*-Indol-6-yl)imidazo[1,2-*a*]pyridin-6-yl]-1-methyl-1*H*-imidazol-2-yl}methanol;
• 2-{5-[2-(4-Chlorphenyl)imidazo[1,2-*a*]pyridin-6-yl]furan-2-yl}propan-2-ol.

6. Medikament, **dadurch gekennzeichnet, dass** es eine Verbindung der Formel (I) nach einem der Ansprüche 1 bis 5 oder ein Additionssalz dieser Verbindung mit einer pharmazeutisch unbedenklichen Säure enthält.

7. Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz dieser Verbindung sowie mindestens einen pharmazeutisch unbedenklichen Hilfsstoff enthält.

8. Verwendung einer Verbindung der Formel (I) nach einem der Ansprüche 1 bis 5 zur Herstellung eines Medikaments zur Behandlung und Prävention von neurodegenerativen Erkrankungen.

9. Verwendung einer Verbindung der Formel (I) nach einem der Ansprüche 1 bis 5 zur Herstellung eines Medikaments zur Behandlung und Prävention von Hirntraumen und Epilepsie.

10. Verwendung einer Verbindung der Formel (I) nach einem der Ansprüche 1 bis 5 zur Herstellung eines Medikaments zur Behandlung und Prävention von psychiatrischen Erkrankungen.

11. Verwendung einer Verbindung der Formel (I) nach einem der Ansprüche 1 bis 5 zur Herstellung eines Medikaments zur Behandlung und Prävention von entzündlichen Erkrankungen.

12. Verwendung einer Verbindung der Formel (I) nach einem der Ansprüche 1 bis 5 zur Herstellung eines Medikaments zur Behandlung und Prävention von Osteoporose und Krebserkrankungen.

13. Verwendung einer Verbindung der Formel (I) nach einem der Ansprüche 1 bis 5 zur Herstellung eines Medikaments zur Behandlung und Prävention von Parkinson-Krankheit, Alzheimer-Krankheit, Tauopathien und multipler Sklerose.

14. Verwendung einer Verbindung der Formel (I) nach einem der Ansprüche 1 bis 5 zur Herstellung eines Medikaments zur Behandlung und Prävention von Schizophrenie, Depression, Substanzabhängigkeit und Aufmerksamkeitsdefizit-Hyperaktivitätssyndrom.

15. Verfahren zur Synthese der Verbindungen der Formel (I) durch Umsetzung von Verbindungen der Formel (VII) 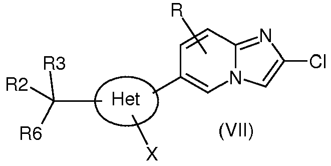 worin R6 für eine OPG-Gruppe steht, R2, R3, Het, X und R die in Anspruch 1 angegebene Bedeutung besitzen und PG für eine Schutzgruppe der Hydroxylfunktion steht, mit R1-Z, wobei R1 die in Anspruch 1 angegebene Bedeutung besitzt und Z für ein Bor- oder Zinnderivat steht, und anschließende Entschützung des erhaltenen Produkts.

16. Verfahren zur Synthese der Verbindungen der Formel (VII) durch Umsetzung von Verbindungen der Formel (V) 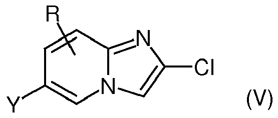 worin Y für ein Borderivat steht und R die in Anspruch 1 angegebene Bedeutung besitzt, mit einer Verbindung der Formel (VI) 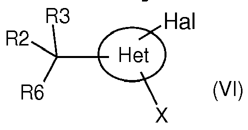 worin R6 für eine OPG-Gruppe steht, R2, R3, Het, X und R die in Anspruch 1 angegebene Bedeutung besitzen und PG für eine Schutzgruppe der Hydroxylfunktion steht.

17. Verfahren zur Synthese der Verbindungen der Formel (I) durch Umsetzung von Verbindungen der Formel (II) 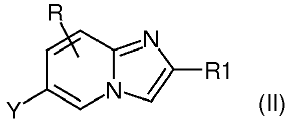 worin R und R1 die in Anspruch 1 angegebene Bedeutung besitzen und Y für ein Borderivat steht, mit einer Verbindung der Formel (III) 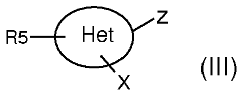 worin R5 für eine Gruppe 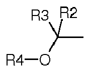 steht und R2, R3, R4, Het und X die in Anspruch 1 angegebene Bedeutung besitzen und Z für ein Halogen steht.

18. Verfahren zur Synthese der Verbindungen der allgemeinen Formel (I) nach Anspruch 1, **dadurch gekennzeichnet, dass** man ein Aminopyridin der allgemeinen Formel (XI) 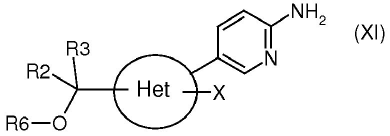 worin R2, R3 und X die in Anspruch 1 angegebene Bedeutung besitzen und R6 für R4 steht, mit einem Bromketon der allgemeinen Formel (XII) 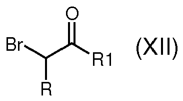 worin R1 die in Anspruch 1 angegebene Bedeutung besitzt und R für ein Wasserstoffatom oder eine (C₁-C₁₀)-Alkylgruppe steht, umsetzt.

19. Verfahren zur Synthese der Verbindungen der allgemeinen Formel (I) nach Anspruch 1, **dadurch gekennzeichnet, dass** man:
a) ein Aminopyridin der allgemeinen Formel (XI) 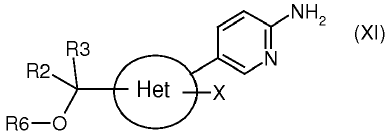 worin R2, R3 und X die in Anspruch 1 angegebene Bedeutung besitzen und R6 für R4 steht, mit einem Bromketon der allgemeinen Formel (XII) 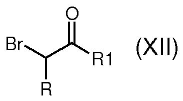 worin R1 die in Anspruch 1 angegebene Bedeutung besitzt und R für ein Wasserstoffatom oder eine (C₁-C₁₀)-Alkylgruppe steht, zu einer Verbindung der allgemeinen Formel (XIII) 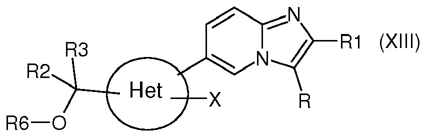 worin R6 für eine Schutzgruppe PG der Hydroxylfunktion steht, umsetzt;
b) die in Schritt b) erhaltene Verbindung der allgemeinen Formel (XIII) einer Entschützungsreaktion unterwirft.

20. Zwischenverbindungen

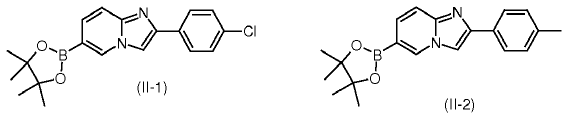

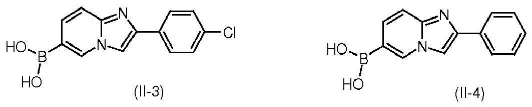

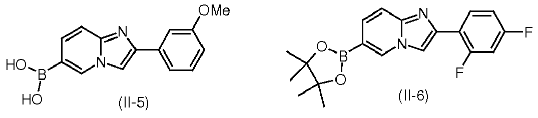

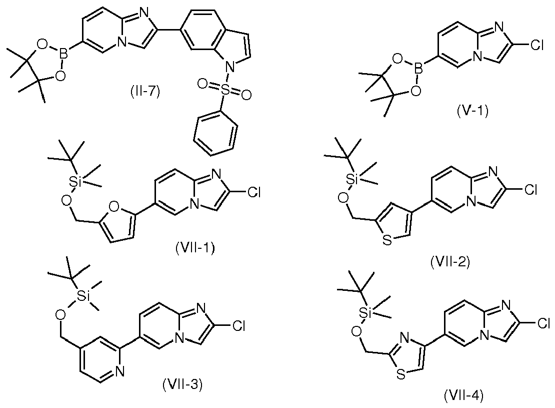

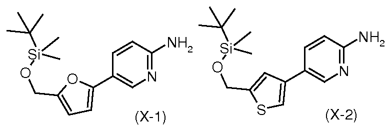

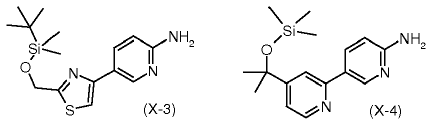

21. Syntheseverfahren nach einem der Ansprüche 15 bis 19, bei dem es sich bei den Verbindungen der allgemeinen Formeln (II), (V), (VII) und (X) um die Verbindungen der allgemeinen Formel (II-1), (II-2), (II-3), (II-4), (II-5), (II-6), (II-7), (V-1), (VII-1), (VII-2), (VII-3), (VII-4), (X-1), (X-2), (X-3) und (X-4) handelt.

## Claims

1. Compounds of formula (I): 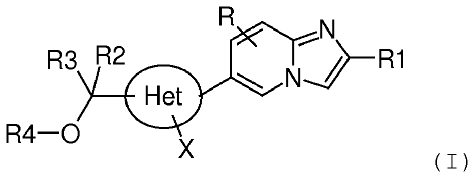 in which:
Het represents a monocyclic heteroaryl group containing from 5 to 6 atoms, including from 1 to 3 heteroatoms chosen from N, O and S;
R₁ represents a phenyl, furyl, quinolinyl, indolyl, pyrrolopyridinyl, pyridinyl, isoquinolinyl, thienyl, benzofuranyl, benzothiazolyl, benzothienyl, dihydrobenzofuranyl, thiazolyl, pyrazolyl, isoxazolyl, benzimidazolyl or indazolyl group, it being possible for these groups to be optionally substituted with one or more groups or atoms chosen, independently of one another, from halogen, (C₁-C₁₀)alkyl, (C₁-C₁₀)alkoxy, CONRaRb, NRaRb, (C₁-C₁₀)thioalkyl, -S(O)₂(C₁-C₁₀-alkyl), halo(C₁-C₁₀)alkyl, hydroxy(C₁-C₁₀)alkylene, NRaRb(C₁-C₁₀)alkylene, NRcCORd, SO₂NRaRb, cyano and nitro;
Ra and Rb represent, independently of one another, a hydrogen atom or a (C₁-C₁₀)alkyl group;
Rc and Rd represent, independently of one another, a hydrogen atom or a (C₁-C₁₀)alkyl group;
X represents from 1 to 3 substituents, which are identical to or different from one another, chosen from a hydrogen, a halogen and (C₁-C₁₀)alkyl groups;
R represents, at position 3 of the imidazo[1,2-a]pyridine, 1 substituent chosen from hydrogen and (C₁-C₁₀)alkyl;
R₂ and R₃ represent, independently of one another, a hydrogen atom or a (C₁-C₁₀)alkyl group;
R₄ represents a hydrogen atom or a (C₁-C₁₀)alkyl group;
in the form of a base or of an addition salt with an acid.

2. Compounds of formula (I) according to any one of the preceding claims, **characterized in that**:
R₁ represents a phenyl, furyl, quinolinyl, indolyl, pyrrolopyridinyl, pyridinyl, isoquinolinyl, thienyl, benzofuranyl, benzothiazolyl, benzothienyl, dihydrobenzofuranyl, thiazolyl, pyrazolyl, isoxazolyl, benzimidazolyl or indazolyl group, it being possible for these groups to be optionally substituted with one or more atoms or groups chosen, independently of one another, from halogen, methyl, methoxy, hydroxymethyl, -CON(CH₃)₂, morpholinyl, pyrrolidinylethyl, -NHCO-CH(CH₃)₂, -CH₂N(CH₃)₂, -NH₂, -CONHCH(CH3)₂, pyrrolidinyl, methylsulphonyl, trifluoromethyl, methylthio, cyano, nitro, -NHCO(CH₃), CONH(CH₃), CONHC(CH₃)₃, -SO₂N(CH₃)₂ and *iso*pentyl;
Het represents a furyl, thienyl, pyridinyl, thiazolyl, pyrazolyl or imidazolyl group;
X represents from 1 to 3 substituents, which are identical to or different from one another, chosen from a hydrogen, a fluorine and a methyl group;
R represents, at position 3 of the imidazo[1,2-a]pyridine, 1 hydrogen atom or methyl group;
R₂ and R₃ represent, independently of one another, a hydrogen atom or a methyl group;
R₄ represents: a hydrogen atom or a methyl group;
in the form of a base or of an addition salt with an acid.

3. Compounds of formula (I) according to Claim 7, **characterized in that** R represents, at position 3 of the imidazo[1,2-*a*]pyridine, 1 hydrogen atom or 1 methyl group.

4. Compounds of formula (I) according to Claim 1, **characterized in that**:
R₁ represents a phenyl, furyl or quinolinyl group, it being possible for these groups to be optionally substituted with one or more atoms or groups chosen, independently of one another, from halogen and (C₁-C₁₀)alkyl;
Het represents a furyl group, a thienyl group, a pyridinyl group or a thiazolyl group;
X represents a hydrogen;
R is a hydrogen;
R₂ and R₃ represent, independently of one another, a hydrogen atom;
R₄ represents a hydrogen atom,
in the form of a base or of an addition salt with an acid.

5. Compounds according to Claim 1, chosen from:
• {5-[2-(4-Chlorophenyl)imidazo[1,2-*a*]pyridin-6-yl]furan-2-yl}methanol;
• {4-[2-(4-Chlorophenyl)imidazo[1,2-*a*]pyridin-6-yl]thien-2-yl}methanol;
• {5-[2-(4-Chlorophenyl)imidazo[1,2-*a*]pyridin-6-yl]thien-2-yl}methanol;
• {2-[2-(4-Chlorophenyl)imidazo[1,2-*a*]pyridin-6-yl]pyridin-4-yl}methanol;
• {5-[2-(4-Chlorophenyl)imidazo[1,2-*a*]pyridin-6-yl]pyridin-3-yl}methanol;
• {4-[2-(4-chlorophenyl)imidazo[1,2-*a*]pyridin-6-yl]furan-2-yl}methanol;
• {6-[2-(4-Chlorophenyl)imidazo[1,2-*a*]pyridin-6-yl]pyridin-2-yl}methanol;
• {5-[2-(4-Chlorophenyl)imidazo[1,2-*a*]pyridin-6-yl]thien-3-yl}methanol;
• {2-[2-(4-Chlorophenyl)imidazo[1,2-*a*]pyridin-6-yl]thiazol-5-yl}methanol;
• {2-[2-(4-Chlorophenyl)imidazo[1,2-*a*]pyridin-6-yl]thiazol-4-yl}methanol;
• {4-[2-(4-Chlorophenyl)imidazo[1,2-*a*]pyridin-6-yl]thiazol-2-yl}methanol;
• [2-(2-Furan-3-ylimidazo[1,2-*a*]pyridin-6-yl)pyridin-4-yl]methanol and the hydrochloride thereof;
• [5-(2-Quinolin-3-ylimidazo[1,2-*a*]pyridin-6-yl)furan-2-yl]methanol and the hydrochloride thereof;
• [4-(2-p-Tolylimidazo[1,2-*a*]pyridin-6-yl)thien-2-yl]methanol;
• [2-(2-Quinolin-3-ylimidazo[1,2-*a*]pyridin-6-yl)pyridin-4-yl]methanol and the hydrochloride thereof;
• [4-(2-Quinolin-3-ylimidazo[1,2*-a*]pyridin-6-yl)thien-2-yl]methanol and the hydrochloride thereof;
• {4-[2-(1*H*-Indol-5-yl)imidazo[1,2-*a*]pyridin-6-yl]thien-2-yl}methanol;
• {5-[2-(1*H*-Indol-5-yl)imidazo[1,2-*a*]pyridin-6-yl]furan-2-yl}methanol and the hydrochloride thereof;
• [4-(2-Phenylimidazo[1,2-*a*]pyridin-6-yl)thien-2-yl]methanol and the hydrochloride thereof;
• {4-[2-(1*H*-Pyrrolo[2,3-b]pyridin-5-yl)imidazo[1,2-*a*]pyridin-6-yl]thien-2-yl}methanol and the hydrochloride thereof;
• {4-[2-(3-Methoxyphenyl)imidazo[1,2-*a*]pyridin-6-yl]thien-2-yl}methanol and the hydrochloride thereof;
• {2-[2-(1H-Pyrrolo[2,3-b]pyridin-5-yl)imidazo[1,2-*a*]pyridin-6-yl]pyridin-4-yl}methanol and the hydrochloride thereof;
• [2-(2-Phenylimidazo[1,2-*a*]pyridin-6-yl)pyridin-4-yl]methanol;
• [5-(2-p-Tolylimidazo[1,2-*a*]pyridin-6-yl)furan-2-yl]methanol;
• [5-(2-Phenylimidazo[1,2-*a*]pyridin-6-yl)furan-2-yl]methanol;
• {5-[2-(3-Methoxyphenyl)imidazo[1,2-*a*]pyridin-6-yl]furan-2-yl}methanol;
• {5-[2-(2,4-Difluorophenyl)imidazo[1,2-*a*]pyridin-6-yl]furan-2-yl}methanol;
• {5-[2-(4-Chlorophenyl)imidazo[1,2*-a*]pyridin-6-yl]furan-3-yl}methanol;
• 2-(4-Chlorophenyl)-6-(4-methoxymethyl-furan-2-yl)imidazo[1,2-*a*]pyridine;
• {5-[2-(4-Hydroxymethylphenyl)imidazo[1,2-*a*]pyridin-6-yl]furan-2-yl}methanol;
• 4-[6-(5-Hydroxymethylfuran-2-yl)imidazo[1,2-*a*]pyridin-2-yl]-N,N-dimethylbenzamide;
• {5-[2-(1*H*-Indol-6-yl)imidazo[1,2-a]pyridin-6-yl]furan-2-yl}methanol;
• {5-[2-(2-Methoxypyridin-3-yl)imidazo[1,2-*a*]pyridin-6-yl]furan-2-yl}methanol;
• [5-(2-Quinolin-5-ylimidazo[1,2-*a*]pyridin-6-yl)furan-2-yl]methanol;
• {5-[2-(4-Morpholin-4-ylphenyl)imidazo[1,2-*a*]pyridin-6-yl]furan-2-yl}methanol;
• [5-(2-Isoquinolin-5-ylimidazo[1,2-*a*]pyridin-6-yl)furan-2-yl]methanol;
• (5-{2-[4-(1-Pyrrolidin-1-ylethyl)phenyl]imidazo[1,2-*a*]pyridin-6-yl}furan-2-yl)methanol;
• 4-[6-(5-Hydroxymethylthien-3-yl)imidazo[1,2-*a*]pyridin-2-yl]-N,N-dimethylbenzamide;
• {4-[2-(2-Methoxypyridin-3-yl)imidazo[1,2-*a*]pyridin-6-yl]thien-2-yl}methanol;
• [4-(2-Isoquinolin-5-ylimidazo[1,2-*a*]pyridin-6-yl)thien-2-yl]methanol;
• *N*-{3-[6-(5-Hydroxymethylthien-3-yl)imidazo[1,2-*a*]pyridin-2-yl]phenyl}isobutyramide;
• (4-{2-[4-(1-Pyrrolidin-1-ylethyl)phenyl]imidazo[1,2-*a*]pyridin-6-yl}thien-2-yl)methanol;
• {4-[2-(4-Dimethylaminomethylphenyl)imidazo[1,2-*a*]pyridin-6-yl]thien-2-yl}methanol;
• 2-Fluoro-4-[6-(5-hydroxymethylthien-3-yl)imidazo[1,2-a]pyridin-2-yl]-*N,N-*dimethylbenzamide;
• {4-[2-(3-Aminophenyl)imidazo[1,2-*a*]pyridin-6-yl]thien-2-yl}methanol;
• *N*-tert-Butyl-5-[6-(5-hydroxymethylthien-3-yl)imidazo[1,2-*a*]pyridin-2-yl]nicotinamide;
• [5-(3-Methyl-2-phenylimidazo[1,2-*a*]pyridin-6-yl)furan-2-yl]methanol;
• [5-(3-Methyl-2-thien-2-ylimidazo[1,2-*a*]pyridin-6-yl)furan-2-yl]methanol;
• {5-[2-(3-Chlorophenyl)-3-methylimidazo[1,2-*a*]pyridin-6-yl]furan-2-yl}methanol;
• {5-[2-(4-Pyrrolidin-1-ylphenyl)imidazo[1,2-*a*]pyridin-6-yl]furan-2-yl}methanol;
• 3-[6-(5-Hydroxymethylfuran-2-yl)imidazo[1,2-*a*]pyridin-2-yl]benzonitrile;
• [5-(2-Benzofuran-2-ylimidazo[1,2-a]pyridin-6-yl)furan-2-yl]methanol;
• [5-(2-Benzothiazol-2-ylimidazo[1,2-*a*]pyridin-6-yl)furan-2-yl]methanol;
• [5-(2-Benzo[b]thien-2-ylimidazo[1,2-*a*]pyridin-6-yl)furan-2-yl]methanol;
• {4-[2-(4-Nitrophenyl)imidazo[1,2-*a*]pyridin-6-yl]thien-2-yl}methanol;
• [4-(2-Thien-3-ylimidazo[1,2-*a*]pyridin-6-yl)thien-2-yl]methanol;
• {4-[2-(4-Methylthiophenyl)imidazo[1,2-*a*]pyridin-6-yl]thien-2-yl}methanol;
• {4-[2-(5-Chlorothien-2-yl)imidazo[1,2-*a*]pyridin-6-yl]thien-2-yl}methanol;
• {4-[2-(4-Methylsulphonylphenyl)imidazo[1,2-*a*]pyridin-6-yl]thien-2-yl}methanol;
• [4-(2-Benzo[b]thien-2-ylimidazo[1,2-*a*]pyridin-6-yl)thien-2-yl]methanol;
• [4-(2-Benzo[b]thien-5-ylimidazo[1,2-*a*]pyridin-6-yl)thien-2-yl]methanol;
• [4-(3-Methyl-2-phenylimidazo[1,2-*a*]pyridin-6-yl)thiazol-2-yl]methanol;
• [4-(3-Methyl-2-thien-2-ylimidazo[1,2-*a*]pyridin-6-yl)thiazol-2-yl]methanol;
• {4-[2-(4-Methoxyphenyl)-3-methylimidazo[1,2-*a*]pyridin-6-yl]thiazol-2-yl}methanol;
• {4-[2-(4-Pyrrolidin-1-ylphenyl)imidazo[1,2-*a*]pyridin-6-yl]thiazol-2-yl}methanol;
• {4-[2-(2-Fluorophenyl)imidazo[1,2-*a*]pyridin-6-yl]thiazol-2-yl}methanol;
• {4-[2-(4-Methylsulphonylphenyl)imidazo[1,2-*a*]pyridin-6-yl]thiazol-2-yl}methanol;
• [4-(2-Benzofuran-2-ylimidazo[1,2-*a*]pyridin-6-yl)thiazol-2-yl]methanol;
• [4-(2-Benzo[b]thien-2-ylimidazo[1,2-*a*]pyridin-6-yl)thiazol-2-yl]methanol;
• [4-(2-Furan-2-ylimidazo[1,2-*a*]pyridin-6-yl)thiazol-2-yl]methanol;
• [4-(2-Benzo[b]thien-5-ylimidazo[1,2-*a*]pyridin-6-yl)thiazol-2-yl]methanol;
• {4-[2-(4-Pyrrolidin-1-ylphenyl)imidazo[1,2-*a*]pyridin-6-yl]thien-2-yl}methanol;
• 1-{4-[2-(4-Chlorophenyl)imidazo[1,2-*a*]pyridin-6-yl]thien-2-yl}ethanol;
• 2-{4-[2-(4-Chlorophenyl)imidazo[1,2-*a*]pyridin-6-yl]thien-2-yl}propan-2-ol;
• {5-[2-(4-Methoxyphenyl)-3-methylimidazo[1,2-*a*]pyridin-6-yl]furan-2-yl}methanol;
• {5-[2-(4-Trifluoromethylphenyl)imidazo[1,2-*a*]pyridin-6-yl]furan-2-yl-methanol;
• {5-[2-(4-Methoxyphenyl)imidazo[1,2-*a*]pyridin-6-yl]furan-2-yl}methanol;
• {5-[2-(4-Fluorophenyl)imidazo[1,2-*a*]pyridin-6-yl]furan-2-yl}methanol;
• {5-[2-(3,4-Difluorophenyl)imidazo[1,2-*a*]pyridin-6-yl]furan-2-yl}methanol;
• [5-(2-Thien-3-ylimidazo[1,2-*a*]pyridin-6-yl)furan-2-yl]methanol;
• {5-[2-(5-Chlorothien-2-yl)imidazo[1,2-*a*]pyridin-6-yl]furan-2-yl}methanol;
• {5-[2-(2-Fluorophenyl)imidazo[1,2-*a*]pyridin-6-yl]furan-2-yl}methanol;
• {5-[2-(3-Bromophenyl)imidazo[1,2-*a*]pyridin-6-yl]furan-2-yl}methanol;
• {5-[2-(4-Chloro-3-methylphenyl)imidazo[1,2-*a*]pyridin-6-yl]furan-2-yl}methanol;
• {5-[2-(4-Methylsulphonylphenyl)imidazo[1,2-*a*]pyridin-6-yl]furan-2-yl}methanol;
• [5-(2-Thien-2-ylimidazo[1,2-*a*]pyridin-6-yl)furan-2-yl]methanol;
• [5-(2-Benzofuran-3-ylimidazo[1,2-*a*]pyridin-6-yl)furan-2-yl]methanol;
• {5-[2-(2,3-Dihydrobenzofuran-5-yl)imidazo[1,2-*a*]pyridin-6-yl]furan-2-yl}methanol;
• [5-(2-Furan-2-ylimidazo[1,2-*a*]pyridin-6-yl)furan-2-yl]methanol;
• [5-(2-Benzofuran-5-ylimidazo[1,2-*a*]pyridin-6-yl)furan-2-yl]methanol;
• [5-(2-Thiazol-2-ylimidazo[1,2-*a*]pyridin-6-yl)furan-2-yl]methanol;
• {5-[2-(4-Methylthiophenyl)imidazo[1,2-*a*]pyridin-6-yl]furan-2-yl}methanol;
• {4-[2-(4-Chlorophenyl)-3-methylimidazo[1,2-*a*]pyridin-6-yl]thien-2-yl}methanol;
• [4-(3-Methyl-2-thien-2-ylimidazo[1,2-*a*]pyridin-6-yl)thien-2-yl]methanol;
• {4-[2-(3-Chlorophenyl)-3-methylimidazo[1,2-*a*]pyridin-6-yl]thien-2-yl}methanol;
• {4-[2-(2,4-Difluorophenyl)-3-methylimidazo[1,2-*a*]pyridin-6-yl]thien-2-yl}methanol;
• {4-[2-(4-Fluorophenyl)imidazo[1,2-*a*]pyridin-6-yl]thien-2-yl}methanol;
• {4-[2-(3,4-Difluorophenyl)imidazo[1,2-*a*]pyridin-6-yl]thien-2-yl}methanol;
• 3-[6-(5-Hydroxymethylthien-3-yl)imidazo[1,2-*a*]pyridin-2-yl]benzonitrile;
• {4-[2-(2-Fluorophenyl)imidazo[1,2-*a*]pyridin-6-yl]thien-2-yl}methanol;
• {4-[2-(3-Bromophenyl)imidazo[1,2-*a*]pyridin-6-yl]thien-2-yl}methanol;
• [4-(2-Benzofuran-2-ylimidazo[1,2-*a*]pyridin-6-yl)thien-2-yl]methanol;
• [4-(2-Thien-2-ylimidazo[1,2-*a*]pyridin-6-yl)thien-2-yl]methanol;
• [4-(2-Benzofuran-3-ylimidazo[1,2-*a*]pyridin-6-yl)thien-2-yl]methanol;
• {4-[2-(1-Methyl-1*H*-benzimidazol-2-yl)imidazo[1,2-*a*]pyridin-6-yl]thien-2-yl}methanol;
• {4-[2-(2,3-Dihydrobenzofuran-5-yl)imidazo[1,2-*a*]pyridin-6-yl]thien-2-yl}methanol;
• [4-(2-Furan-2-ylimidazo[1,2-*a*]pyridin-6-yl)thien-2-yl]methanol;
• [4-(2-Benzofuran-5-ylimidazo[1,2-*a*]pyridin-6-yl)thien-2-yl]methanol;
• [4-(2-Thiazol-2-ylimidazo[1,2-*a*]pyridin-6-yl)thien-2-yl]methanol;
• {4-[2-(4-Chlorophenyl)-3-methylimidazo[1,2-*a*]pyridin-6-yl]thiazol-2-yl}methanol;
• {4-[2-(3-Chlorophenyl)-3-methylimidazo[1,2-*a*]pyridin-6-yl]thiazol-2-yl}methanol;
• {4-[2-(4-Trifluoromethylphenyl)imidazo[1,2-*a*]pyridin-6-yl]thiazol-2-yl}methanol;
• {4-[2-(4-Fluorophenyl)imidazo[1,2-*a*]pyridin-6-yl]thiazol-2-yl}methanol;
• {4-[2-(3,4-Difluorophenyl)imidazo[1,2-*a*]pyridin-6-yl]thiazol-2-yl}methanol;
• [4-(2-Thien-3-ylimidazo[1,2-*a*]pyridin-6-yl)thiazol-2-yl]methanol;
• [4-(2-Pyridin-2-ylimidazo[1,2-*a*]pyridin-6-yl)thiazol-2-yl]methanol;
• {4-[2-(5-Chlorothien-2-yl)imidazo[1,2-*a*]pyridin-6-yl]thiazol-2-yl}methanol;
• {4-[2-(3-Bromophenyl)imidazo[1,2-*a*]pyridin-6-yl]thiazol-2-yl}methanol;
• {4-[2-(4-Chloro-3-methylphenyl)imidazo[1,2-*a*]pyridin-6-yl]thiazol-2-yl}methanol;
• [4-(2-Thien-2-ylimidazo[1,2-*a*]pyridin-6-yl)thiazol-2-yl]methanol;
• [4-(2-Benzofuran-3-ylimidazo[1,2-*a*]pyridin-6-yl)thiazol-2-yl]methanol;
• {4-[2-(2,3-Dihydrobenzofuran-5-yl)imidazo[1,2-*a*]pyridin-6-yl]thiazol-2-yl}methanol;
• [4-(2-Benzofuran-5-ylimidazo[1,2-*a*]pyridin-6-yl)thiazol-2-yl]methanol;
• [4-(2-Thiazol-2-ylimidazo[1,2-*a*]pyridin-6-yl)thiazol-2-yl]methanol;
• {5-[2-(3-Hydroxymethylphenyl)imidazo[1,2-*a*]pyridin-6-yl]furan-2-yl}methanol;
• *N*-{3-[6-(5-Hydroxymethylfuran-2-yl)imidazo[1,2-*a*]pyridin-2-yl]phenyl}isobutyramide;
• 2-Fluoro-4-[6-(5-hydroxymethylfuran-2-yl)imidazo[1,2-*a*]pyridin-2-yl]-*N,N-*dimethylbenzamide;
• 2-{4-[2-(1H-Indol-6-yl)imidazo[1,2-*a*]pyridin-6-yl]thien-2-ylpropan-2-ol;
• *N*-{3-[6-(2-Hydroxymethylthiazol-4-yl)imidazo[1,2-*a*]pyridin-2-yl]phenyl}acetamide;
• {4-[2-(4-Hydroxymethylphenyl)imidazo[1,2-*a*]pyridin-6-yl]thiazol-2-yl}methanol;
• {4-[2-(3-Hydroxymethylphenyl)imidazo[1,2-*a*]pyridin-6-yl]thiazol-2-yl}methanol;
• {4-[2-(1H-Indol-5-yl)imidazo[1,2-*a*]pyridin-6-yl]thiazol-2-yl}methanol;
• {4-[2-(4-Methylthien-2-yl)imidazo[1,2-*a*]pyridin-6-yl]thiazol-2-yl}methanol;
• {4-[2-(1H-Indol-4-yl)imidazo[1,2-*a*]pyridin-6-yl]thiazol-2-yl}methanol;
• {4-[2-(2-Fluoropyridin-3-yl)imidazo[1,2-*a*]pyridin-6-yl]thiazol-2-yl}methanol;
• 3-[6-(2-Hydroxymethylthiazol-4-yl)imidazo[1,2-*a*]pyridin-2-yl]-N,N-dimethylbenzamide;
• {4-[2-(1H-Indol-6-yl)imidazo[1,2-*a*]pyridin-6-yl]thiazol-2-yl}methanol;
• {4-[2-(2-Methoxypyridin-3-yl)imidazo[1,2-*a*]pyridin-6-yl]thiazol-2-yl}methanol;
• 4-[6-(2-Hydroxymethylthiazol-4-yl)imidazo[1,2-*a*]pyridin-2-yl]-N-methylbenzamide;
• {4-[2-(4-Methylthien-3-yl)imidazo[1,2-*a*]pyridin-6-yl]thiazol-2-yl}methanol;
• {4-[2-(1-Methyl-*1H*-indol-5-yl)imidazo[1,2-*a*]pyridin-6-yl]thiazol-2-yl}methanol;
• [4-(2-Quinolin-5-ylimidazo[1,2-*a*]pyridin-6-yl)-thiazol-2-yl]methanol;
• *N*-{4-[6-(2-Hydroxymethylthiazol-4-yl)imidazo[1,2-*a*]pyridin-2-yl]phenyl}acetamide;
• {4-[2-(4-Morpholin-4-ylphenyl)imidazo[1,2-*a*]pyridin-6-yl]thiazol-2-yl}methanol;
• [4-(2-Isoquinolin-5-ylimidazo[1,2-*a*]pyridin-6-yl)thiazol-2-yl]methanol;
• *N*-{3-[6-(2-Hydroxymethyl-thiazol-4-yl)imidazo[1,2-*a*]pyridin-2-yl]phenyl}isobutyramide;
• 3-[6-(2-Hydroxymethylthiazol-4-yl)imidazo[1,2-*a*]pyridin-2-yl]-N,N-dimethylbenzenesulphonamide;
• {4-[2-(2,6-Difluoropyridin-4-yl)imidazo[1,2-*a*]pyridin-6-yl]thiazol-2-yl}methanol;
• (4-{2-[1-(3-Methylbutyl)-*1H-*pyrazol-4-yl]imidazo[1,2-*a*]pyridin-6-yl}thiazol-2-yl)methanol;
• 2-[{4-[2-(1*H*-Indol-6-yl)imidazo[1,2-*a*]pyridin-6-yl]-1*H*-pyrazol-3-yl}propan-2-ol;
• 2-{5-Fluoro-2-[2-(1*H*-indol-6-yl)imidazo[1,2-*a*]pyridin-6-yl]pyridin-4-yl}propan-2-ol;
• 2-{2-[2-(*1H*-Indol-6-yl)imidazo[1,2-*a*]pyridin-6-yl]pyridin-4-yl}propan-2-ol;
• 2-{2-[2-(1*H*-Indazol-3-yl)imidazo[1,2-*a*]pyridin-6-yl]pyridin-4-yl}propan-2-ol;
• 2-{2-[2-(5-Methylisoxazol-3-yl)imidazo[1,2-*a*]pyridin-6-yl]pyridin-4-yl}propan-2-ol;
• {5-[2-(*1H*-Indol-6-yl)imidazo[1,2-*a*]pyridin-6-yl]-1-methyl-*1H*-imidazol-2-yl}methanol;
• 2-{5-[2-(4-Chlorophenyl)imidazo[1,2-*a*]pyridin-6-yl]furan-2-yl}propan-2-ol.

6. Medicament, **characterized in that** it comprises a compound of formula (I) according to any one of Claims 1 to 5, or an addition salt of this compound with a pharmaceutically acceptable acid.

7. Pharmaceutical composition, **characterized in that** it comprises a compound of formula (I) according to any one of Claims 1 to 5, or a pharmaceutically acceptable salt of this compound, and also at least one pharmaceutically acceptable excipient.

8. Use of a compound of formula (I) according to any one of Claims 1 to 5, for the preparation of a medicament for use in the treatment and prevention of neurodegenerative diseases.

9. Use of a compound of formula (I) according to any one of Claims 1 to 5, for the preparation of a medicament for use in the treatment and prevention of cerebral traumas and of epilepsy.

10. Use of a compound of formula (I) according to any one of Claims 1 to 5, for the preparation of a medicament for use in the treatment and prevention of psychiatric diseases.

11. Use of a compound of formula (I) according to any one of Claims 1 to 5, for the preparation of a medicament for use in the treatment and prevention of inflammatory diseases.

12. Use of a compound of formula (I) according to any one of Claims 1 to 5, for the preparation of a medicament for use in the treatment and prevention of osteoporosis and cancers.

13. Use of a compound of formula (I) according to any one of Claims 1 to 5, for the preparation of a medicament for use in the treatment and prevention of Parkinson's disease, Alzheimer's disease, tauopathies and multiple sclerosis.

14. Use of a compound of formula (I) according to any one of Claims 1 to 5, for the preparation of a medicament for use in the treatment and prevention of schizophrenia, depression, substance dependence and attention deficit hyperactivity disorders.

15. Process for synthesizing the compounds of formula (I) by reacting compounds of formula (VII) 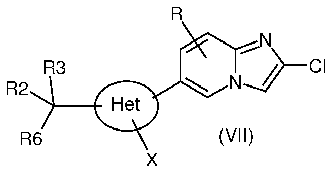 in which R6 represents an OPG group, R2, R3, Het, X and R are as defined in Claim 1, and GP is a group protecting the hydroxyl function, with R1-Z, with R1 being as defined in Claim 1 and Z being a boron or tin derivative, and then deprotecting the product obtained.

16. Process for synthesizing the compounds of formula (VII) by reacting compounds of formula (V) 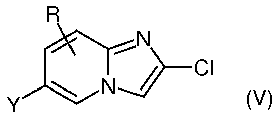 in which Y is a boron derivative, and R is as defined in Claim 1, with a compound of formula (VI) 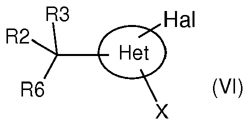 in which R6 represents an OPG group, R2, R3, Het and X are as defined in Claim 1, and GP is a group protecting the hydroxyl function.

17. Process for synthesizing the compounds of formula (I) by reacting compounds of formula (II) 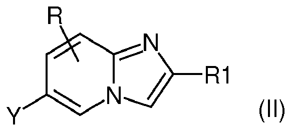 in which R and R1 are as defined in Claim 1, and Y is a boron derivative, with a compound of formula (III) 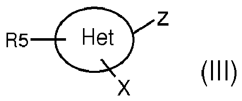 in which R5 represents a group 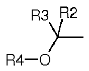 and R2, R3, R4, Het and X are as defined in Claim 1 and Z is a halogen.

18. Process for synthesizing the compounds of general formula (I) according to Claim 1, **characterized in that** an aminopyridine of general formula (XI), 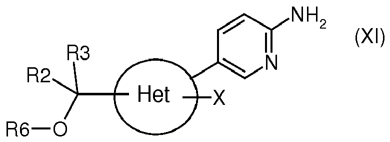 in which R2, R3 and X are defined according to Claim 1, and R6 represents R4, is reacted with a bromoketone of general formula (XII), 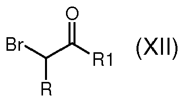 in which R1 is defined according to Claim 1 and R represents a hydrogen atom or a (C₁-C₁₀)alkyl group.

19. Process for synthesizing the compounds of general formula (I) according to Claim 1, **characterized in that**:
a) an aminopyridine of general formula (XI), 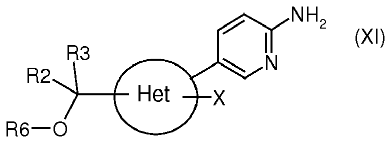 in which R2, R3 and X are defined according to Claim 1, and R6 represents R4, is reacted with a bromoketone of general formula (XII), 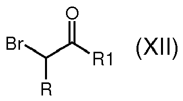 in which R1 is defined according to Claim 1 and R represents a hydrogen atom or a (C₁-C₁₀)alkyl group, so as to obtain a compound of general formula (XIII) 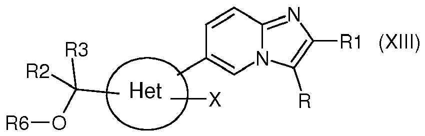 in which R6 represents a group PG protecting the hydroxyl function;
b) the compound of general formula (XIII) obtained in step b) is subjected to a deprotection reaction.

20. Intermediate compounds

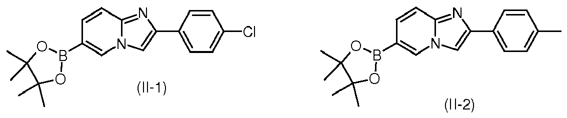

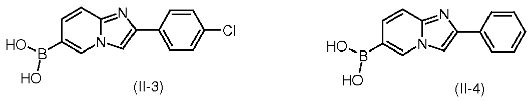

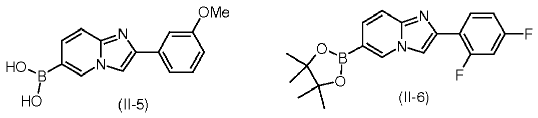

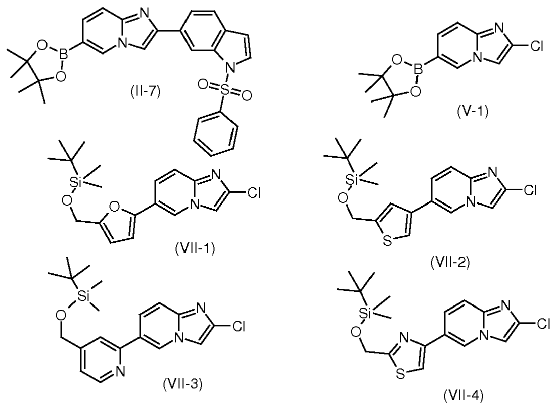

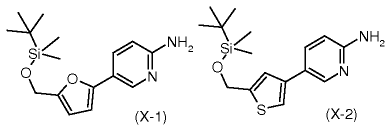

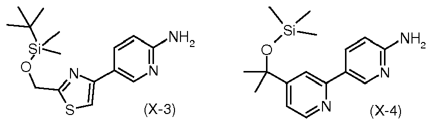

21. Synthesis process according to any one of Claims 15 to 19, wherein the compounds of general formulae (II), (V), (VII) and (X) are the compounds of general formulae (II-1), (II-2), (II-3), (II-4), (II-5), (II-6), (II-7), (V-1), (VII-1), (VII-2), (VII-3), (VII-4), (X-1), (X-2), (X-3) and (X-4).
